# EUROPÄISCHE PATENTANMELDUNG

(11) **EP 2 253 617 A1**
(43) Veröffentlichungstag der Anmeldung: **24.11.2010**
(21) Anmeldenummer: 09160715.0
(22) Anmeldetag: 20.05.2009
(51) Int. Cl.: C07D 213/56, C07D 231/14, C07D 239/36, C07D 277/56, C07D 401/12, C07D 417/12, A01N 43/40, A01N 43/56, A01N 43/78, A01N 43/54, A61P 33/00, A61K 31/381

(54) **Halogen-substituierte Verbindungen als Pestizide**

(71) Anmelder: Bayer CropScience AG, 40789 Monheim (DE)
(72) Erfinder: Die Erfindernennung liegt noch nicht vor

(57) **Zusammenfassung**

Die Erfindung betrifft Verbindungen der allgemeinen Formel (I), 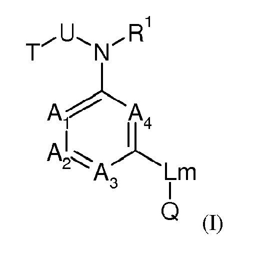 in welcher die Reste A₁, A₂, A₃, A₄, Lm, Q, R¹, T und U die in der Beschreibung angegebene Bedeutung besitzen sowie die Verwendung der Verbindungen zur Bekämpfung von tierischen Schädlingen. Ferner betrifft die Erfindung Verfahren und Intermediate zur Herstellung der Verbindungen gemäß Formel (I).

## Beschreibung

Die vorliegende Anmeldung betrifft neue Halogen-substituierte Verbindungen, Verfahren zu ihrer Herstellung und ihre Verwendung zur Bekämpfung von tierischen Schädlingen, vor allem von Arthropoden und insbesondere von Insekten, Spinnentieren und Nematoden.

Bestimmte Halogen-substituierte Verbindungen mit insektizider Wirkung sind bereits beschrieben (W02008/031534; WO2008/074427; W02008/000438; WO2007/128410; WO2007/051560; WO2008/012027; W02006/137376).

Ferner ist bekannt, dass bestimmte Halogen-substituierte Verbindungen herbizid wirksam (vgl. J. Org. Chem. 1997, 62(17), 5908-5919, J. Heterocycl. Chem. 1998, 35(6), 1493-1499, WO 2004/035545, WO 2004/106324, US 2006/069132, WO 2008/029084) oder fungizid wirksam sind und zur Bekämpfung von Pflanzenkrankheiten eingesetzt werden können (vgl. WO 2009/028280).

Darüber hinaus ist bekannt, dass bestimmte Halogen-substituierte Verbindungen Cytokininhibitorische Aktivitäten aufweisen (WO2006/067445; WO2006/067446; WO2000/007980).

Über die Verwendung der in dieser Anmeldung beanspruchten Halogen-substituierten Verbindungen zur Bekämpfung von tierischen Schädlingen, insbesondere als Pflanzenschutzmittel, ist jedoch nichts bekannt.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Aus all diesen Gründen kann die Suche nach neuen Pflanzenschutzmitteln nie als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Es wurde nun überraschenderweise gefunden, dass bestimmte Halogen-substituierte Verbindungen, sowie deren N-Oxide und Salze biologische Eigenschaften aufweisen und sich insbesondere zur Bekämpfung von tierischen Schädlingen eignen, und deshalb besonders gut im agrochemischen Bereich und im Bereich der Tiergesundheit einsetzbar sind.

Die erfindungsgemäßen Halogen-substituierten Verbindungen sind durch die Formel (I) 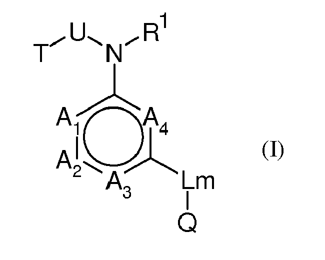 definiert, in der
- R¹: für Wasserstoff, gegebenenfalls substituiertes C₁-C₆-Alkyl, C₁-C₆-Halogenalkyl, C₂-C₆- Alkylen, C₂-C₆-Alkinyl, C₃-C₇-Cycloalkyl, (C₁-C₆-Alkyl)carbonyl, (C₁-C₆- Alkoxy)carbonyl, (Cₗ₁-C₂-Alkoxy)-(C₁-C₂-alkyl), Cyan-(C₁-C₂-alkyl), Aryl-(C₁-C₃-alkyl), Heteroaryl-(C₁-C₃-alkyl) steht,
die chemischen Gruppierungen
- A₁: für CR², einen gegebenenfalls mit G substituierten Stickstoff, Sauerstoff, Schwefel oder eine Bindung,
- A₂: für CR³ oder einen gegebenenfalls mit G substituierten Stickstoff, Sauerstoff oder Schwefel,
- A₃: für CR⁴ oder einen gegebenenfalls mit G substituierten Stickstoff, Sauerstoff oder Schwefel und
- A₄: für CR⁵ oder einen gegebenenfalls mit G substituierten Stickstoff, Sauerstoff oder Schwefel stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, *N*-(C₁-C₆-Alkyl)aminocarbonyl, *N*-(C₃-C₇-Cycloalkyl)aminocarbonyl oder (C₁-C₄-Alkoxy)carbonyl, stehen; wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält und gegebenenfalls substituiert ist, oder
wenn keine der Gruppierungen A₁ und A₂ für Stickstoff steht, können R² und R³ gemeinsam mit dem Kohlenstoff an das sie gebunden sind einen 6-gliedrigen Ring bilden, der 0, 1 oder 2 Stickstoffatome enthält und gegebenenfalls substituiert ist;
- G: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
- U: für eine Gruppierung C(=W), SO oder SO₂ steht,
wobei
- W: für Sauerstoff oder Schwefel steht;
- L: für eine bivalente chemische Gruppierung steht, die ausgewählt ist aus den Gruppierungen, -CH₂NHC(=W)-, -CH₂NR⁶C(=W)-, -C(=W)NH-, -C(=W)NR⁶, -C(=W)NHCH₂-, -C(=W)NR⁶CH2-, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=NH)-, -SO(=N-CN)- und -S(=N-CN)-, -C(=W)NHSO₂-, bei der die rechte Seite der Gruppierung direkt an den Rest Q anknüpft und wobei,
- p: die Werte 0, 1 oder 2 annehmen kann;
- R⁶: für Wasserstoff, gegebenenfalls substituiertes C₁-C₆-Alkyl, C₁-C₆-Halogenalkyl, Aryl-(C₁- C₃-alkyl), Heteroaryl-(C₁-C₃-alkyl), C₂-C₆-Alkenyl, C₂-C₆-Alkinyl, C₃-C₆-Cycloalkyl, (C₁- C₂-Alkyl)-(C₃-C₅-cycloalkyl), (C₃-C₅-Cycloalkyl)-(C₁-C₂-alkyl), (C₁-C₆-Alkyl)carbonyl und (C₁-C₆-Alkoxy)carbonyl steht;
- m: die Werte 0 oder 1 annehmen kann;
- Q: für einen gegebenenfalls mehrfach mit V substituierten gesättigten oder ungesättigten 5- oder 6-gliedrigen Ring steht, oder für einen gegebenenfalls mehrfach substituierten 5- oder 6-gliedrigen, heterozyklischen Ring steht, wobei
- V: für Halogen, Cyano, Nitro, gegebenenfalls substituiertes C₁-C₆-Alkyl, C₁-C₄-Alkenyl, C₁- C₄-Alkinyl, C₁-C₆-Halogenalkyl, C₃-C₆-Cycloalkyl, C₃-C₆-Halogencycloalkyl, C₁-C₆- Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino steht
die chemische Gruppierung T für einen gegebenenfalls. mehrfach mit Z substituierten gesättigten oder ungesättigten 5- oder 6-gliedrigen Ring steht, oder für einen gegebenenfalls mehrfach mit Z substituierten 5- oder 6-gliedrigen, heterozyklischen Ring steht;
- Z: für Wasserstoff, Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆-Halogenalkyl- sulfonyl, *N*,*N*-Di-(C₁-C₆-alkyl)amino, -C(=W)NR⁷R⁶, -C(=W)OR⁸, -S(O)₂NR⁹R¹⁰, -S(O)ₚR¹¹, -S(O)(=NR¹²)R¹³, (C₃-C₆-Cycloalkyl)carbonyl, (C₃-C₆-Cycloalkyl)-(C₁-C₃- alkyl), Aryl-(C₁-C₃-alkyl), Heteroaryl-(C₁-C₃-alkyl) und gegebenenfalls mit R¹⁴ substituiertes Phenyl und Pyridinyl steht;
- R⁷: ausgewählt ist aus Wasserstoff oder den gegebenenfalls substituierten Gruppierungen C₁- C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
- R⁸: ausgewählt ist aus Wasserstoff oder den gegebenenfalls mit R⁶ substituierten Gruppierungen C₁-C₆-Alkyl, C₁-C₆-Halogenalkyl, C₂-C₆-Alkenyl, C₂-C₆-Halogenalkenyl, C₂-C₆-Alkinyl, C₃-C₆-Cycloalkyl, C₃-C₆-Halogencycloalkyl, (C₁-C₂-Alkyl)-(C₃-C₆- cycloalkyl) und (C₃-C₆-Cycloalkyl)-(C₁-C₂-Alkyl);
- R⁹: ausgewählt ist aus Wasserstoff oder den gegebenenfalls substituierten Gruppierungen C₁- C₆-Alkyl, C₁-C₆-Halogenalkyl, C₂-C₆-Alkenyl, C₂-C₆-Alkinyl, C₃-C₆-Cycloalkyl, (C₁-C₂- Alkyl)-(C₃-C₆-cycloalkyl), (C₃-C₆-Cycloalkyl)-(C₁-C₂-Alkyl), (C₁-C₆-Alkyl)carbonyl und (C₁-C₆-Alkoxy)carbonyl;
- R¹⁰: ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-Alkyl);
- R¹¹: ausgewählt ist aus den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃C₆-Cycloalkyl)-(C₁-C₂alkyl), C₁-C₄-Haloalkyl;
- R¹²: ausgewählt ist aus Wasserstoff, C₁-C₆-Alkyl, C₁-C₆-Halogenalkyl, C₂-C₆-Alkenyl, C₂-C₆- Alkinyl, C₃-C₆-Cycloalkyl, (C₁-C₂-Alkyl)-(C₃-C₆-cycloalkyl), (C₃-C₆-Cycloalkyl)-(C₁-C₂- alkyl), (C₁-C₆-Alkyl)carbonyl und (C₁-C₆-Alkoxy)carbonyl;
- R¹³: ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-alkyl);
- R¹⁴: ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₁-C₆-Alkylanüno, *N*,*N*-Di-(C₁-C₆)alkylamino, (C₁-C₆- Alkyl)carbonyl, (C₁-C₆-Alkoxy)carbonyl, *N*-(C₁-C₆-Alkyl)aminocarbonyl und *N*,*N*-Di-(C₁- C₆-alkyl)aminocarbonyl;
- R¹⁵: ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆- Alkylsulfonyl, -CN, -NO₂ sowie gegebenenfalls mit R¹⁶ substituiertem Phenyl oder Pyridyl;
- R¹⁶: ausgewählt ist aus Halogen, -CN, -NO₂, C₁-C₆-Alkyl, C₁-C₆-Alkoxy, C₁-C₆-Alkylthio, C₁- C₆-Alkylsulfinyl, C₁-C₆-Alkylsulfonyl, (C₁-C₆-Alkyl)carbonyl, (C₁-C₆-Alkoxy)carbonyl, *N*-(C₁-C₆-Alkyl)aminocarbonyl, oder gegebenenfalls mit R¹⁸ substituiertem Phenyl oder Pyridyl;
- R¹⁷: ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆- Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*-C₁-C₆- Alkylamino, *N*,*N*-Di-(C₁-C₆)alkylamino, (C₁-C₃-Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
- R¹⁸: ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₁-C₆-Alkylamino, *N*,*N*-Di-(C₁-C₆)alkylamino, (C₁-C₃- Alkyl)carbonyl, (C₁-C₃-Alkoxy)carbonyl, *N*-(C₁-C₆-Alkyl)aminocarbonyl, *N*,*N*-Di-(C₁-C₆- alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Bevorzugt sind Verbindungen der Formel (I) 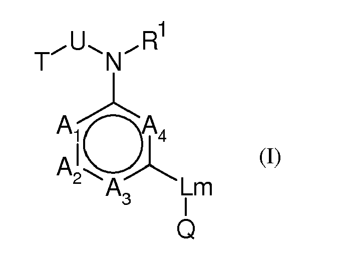 definiert, in denen
- R¹: für Wasserstoff, gegebenenfalls substituiertes C₁-C₆-Alkyl, C₂-C₆-Alkylen, C₂-C₆-Alkinyl, C₃-C₇-Cycloalkyl, (C₁-C₆-Alkyl)carbonyl, (C₁-C₆-Alkoxy)carbonyl, (C₁-C₂-Alkoxy)-(C₁- C₂-alkyl), Cyan-(C₁-C₂-alkyl), Aryl(C₁-C₃-alkyl), Heteroaryl(C₁-C₃-alkyl) steht,
die chemischen Gruppierungen
- A₁: für CR², einen gegebenenfalls mit G substituierten Stickstoff, Sauerstoff, Schwefel oder eine Bindung,
- A₂: für CR³ oder einen gegebenenfalls mit G substituierten Stickstoff, Sauerstoff oder Schwefel,
- A₃: für CR⁴ oder einen gegebenenfalls mit G substituierten Stickstoff, Sauerstoff oder Schwefel und
- A₄: für CR⁵ oder einen gegebenenfalls mit G substituierten Stickstoff, Sauerstoff oder Schwefel stehen, wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, *N*-(C₁-C₆-Alkyl)aminocarbonyl, *N*-(C₃-C₆-Cycloalkyl)aminocarbonyl oder (C₁-C₄-Alkoxy)carbonyl, stehen;
wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoff an das sie gebunden sind einen 6-gliedrigen Ring bilden, der 0, 1 oder 2 Stickstoffatome enthält;
- G: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
- U: für eine Gruppierung C(=W), SO oder SO₂ steht,
wobei
- W: für Sauerstoff oder Schwefel steht;
- L: für eine bivalente chemische Gruppierung steht, die ausgewählt ist aus den Gruppierungen -CH₂NHC(=W)-, -CH₂NR⁶C(=W)-, -C(=W)NH-, -C(=W)NR⁶, -C(=W)NHCH₂-, -C(=W)NR⁶CH₂-, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=N-CN)- und -S(=N-CN)-, -C(=W)NHSO₂-, bei der die rechte Seite der Gruppierung direkt an den Rest Q anknüpft und wobei,
- p: die Werte 0, 1 oder 2 annehmen kann;
- R⁶: für Wasserstoff, gegebenenfalls substituiertes C₁-C₆-Alkyl, C₁-C₆-Halogenalkyl, Aryl-(C₁- C₃-alkyl), Heteroaryl-(C₁-C₃-alkyl), C₂-C₆-Alkenyl, C₂-C₆-Alkinyl, C₃-C₆-Cycloalkyl, (C₁- C₂-Alkyl)-(C₃-C₆-cycloalkyl), (C₃-C₆-Cycloalkyl)-(C₁-C₂-alkyl), (C₁-C₆-Alkyl)carbonyl und (C₁-C₆-Alkoxy)carbonyl steht;
- m: den Wert 1 annimmt;
- Q: für ein gegebenenfalls mehrfach mit V substituiertes Phenyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
- V: für Halogen, Cyano, Nitro, gegebenenfalls substituiertes C₁-C₆-Alkyl, C₁-C₄-Alkenyl, C₁- C₄-Alkinyl, C₁-C₆-Halogenalkyl, C₃-C₆-Cycloalkyl, C₃-C₆-Halogencycloalkyl, C₁-C₆- Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino steht;
die chemische Gruppierung T für ein gegebenenfalls mehrfach mit Z substituiertes Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxdiazol oder Thiadiazol stehen kann,
- Z: für Wasserstoff, Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino, -C(=W)NR⁷R⁶, -C(=W)OR⁸, -S(O)₂NR⁹R¹⁰, -S(O)ₚR¹¹, -S(O)(=NR¹²)R¹³, (C₃-C₆-Cycloalkyl)carbonyl, (C₃-C₆- Cycloalkyl)-(C₁-C₃-alkyl), Aryl-(C₁-C₃-alkyl), Heteroaryl-(C₁-C₃-alkyl) und gegebenenfalls mit R¹⁴ substituiertes Phenyl und Pyridinyl steht;
- R⁷: ausgewählt ist aus Wasserstoff oder den gegebenenfalls substituierten Gruppierungen C₁- C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
- R⁸: ausgewählt ist aus Wasserstoff oder den gegebenenfalls mit R⁶ substituierten Gruppierungen C₁-C₆-Alkyl, C₁-C₆-Halogenalkyl, C₂-C₆-Alkenyl, C₂-C₆-Halogenalkenyl,
- R⁹: C₂-C₆-Alkinyl, C₃-C₆-Cycloalkyl, C₃-C₆-Halogencycloalkyl, (C₁-C₂-Alkyl)-(C₃-C₆- cycloalkyl) und (C₃-C₆-Cycloalkyl)-(C₁-C₂-Alkyl); ausgewählt ist aus Wasserstoff oder den gegebenenfalls substituierten Gruppierungen C₁- C₆-Alkyl, C₁-C₆-Halogenalkyl, C₂-C₆-Alkenyl, C₂-C₆-Alkinyl, C₃-C₆-Cycloalkyl, (C₁-C₂- Alkyl)-(C₃-C₆-cycloalkyl), (C₃-C₆-Cycloalkyl)-(C₁-C₂-Alkyl), (C₁-C₆-Alkyl)carbonyl und (C₁-C₆-Alkoxy)carbonyl;
- R¹⁰: ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-Alkyl);
- R¹¹: ausgewählt ist aus den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃-C₆-Cycloalkyl)-(C₁-C₂alkyl), C₁-C₄-Haloalkyl;
- R¹²: ausgewählt ist aus Wasserstoff, C₁-C₆-Alkyl, C₁-C₆-Halogenalkyl, C₂-C₆-Alkenyl, C₂-C₆- Alkinyl, C₃-C₆-Cycloalkyl, (C₁-C₂-Alkyl)-(C₃-C₆-cycloalkyl), (C₃-C₆-Cycloalkyl)-(C₁-C₂- alkyl), (C₁-C₆-Alkyl)carbonyl und (C₁-C₆-Alkoxy)carbonyl;
- R¹³: ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-alkyl);
- R¹⁴: ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₁-C₆-Alkylamino, *N*,*N*-Di-(C₁-C₆-alkyl)amino, (C₁-C₆- Alkyl)carbonyl, (C₁-C₆-Alkoxy)carbonyl, *N*-(C₁-C₆-Alkyl)aminocarbonyl und *N*,*N*-Di-(C₁- C₆-alkyl)aminocarbonyl;
- R¹⁵: ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆- Alkylsulfonyl, -CN, -NO₂ sowie gegebenenfalls mit R¹⁶ substituiertem Phenyl oder Pyridyl;
- R¹⁶: ausgewählt ist aus Halogen, -CN, -NO₂, C₁-C₆-Alkyl, C₁-C₆-Alkoxy, C₁-C₆-Alkylthio, C₁- C₆-Alkylsulfinyl, C₁-C₆-Alkylsulfonyl, (C₁-C₆-Alkyl)carbonyl, (C₁-C₆-Alkoxy)carbonyl, *N*-(C₁-C₆-Alkyl)aminocarbonyl, oder gegebenenfalls mit R¹⁸ substituiertem Phenyl oder Pyridyl;
- R¹⁷: ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆- Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*-C₁-C₆- Alkylamino, *N*,*N*-Di-(C₁-C₆-alkyl)amino, (C₁-C₃-Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
- R¹⁸: ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, (C₁-C₃- Alkyl)carbonyl, (C₁-C₃-Alkoxy)carbonyl, *N*-(C₁-C₆-Alkyl)aminocarbonyl, *N*,*N*-Di-(C₁-C₆- alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Besonders bevorzugt sind Verbindungen der Formel (I) 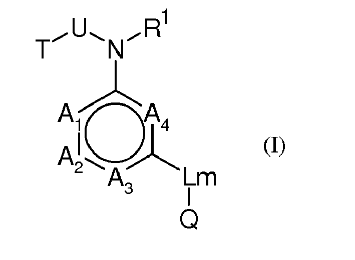 definiert, in denen
- R¹: für Wasserstoff, gegebenenfalls substituiertes C₁-C₆-Alkyl, C₂-C₆-Alkylen, C₂-C₆-Alkinyl, C₃-C₇-Cycloalkyl, (C₁-C₆-Alkyl)carbonyl, (C₁-C₆-Alkoxy)carbonyl, (C₁-C₂-Alkoxy)-(C₁- C₂-alkyl), Cyan-(C₁-C₂-alkyl), Aryl(C₁-C₃-alkyl), Heteroaryl(C₁-C₃-alkyl) steht,
die chemischen Gruppierungen
- A₁: für CR², einen gegebenenfalls mit G substituierten Stickstoff, Sauerstoff, Schwefel oder eine Bindung,
- A₂: für CR³ oder einen gegebenenfalls mit G substituierten Stickstoff, Sauerstoff oder Schwefel,
- A₃: für CR⁴ oder einen gegebenenfalls mit G substituierten Stickstoff, Sauerstoff oder Schwefel und
- A₄: für CR⁵ oder einen gegebenenfalls mit G substituierten Stickstoff, Sauerstoff oder Schwefel stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, *N*-(C₁-C₆-Alkyl)aminocarbonyl, *N*-(C₃-C₆-Cycloalkyl)aminocarbonyl oder (C₁-C₄-Alkoxy)carbonyl, stehen;
wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoff an das sie gebunden sind einen 6-gliedrigen Ring bilden, der 0, 1 oder 2 Stickstoffatome enthält;
- G: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
- U: für eine Gruppierung C(=W), SO oder SO₂ steht,
wobei
- W: für Sauerstoff oder Schwefel steht;
- L: für eine bivalente chemische Gruppierung steht, die ausgewählt ist aus den Gruppierungen -CH₂NHC(=W)-, -CH₂NR⁶C(=W)-, -C(=W)NH-, -C(=W)NR⁶, -C(=W)NHCH₂-, -C(=W)NR⁶CH₂-, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=N-CN)- und -S(=N-CN)-, -C(=W)NHSO₂-, bei der die rechte Seite der Gruppierung direkt an den Rest Q anknüpft und wobei,
p die Werte 0, 1 oder 2 annehmen kann;
- R⁶: für Wasserstoff, gegebenenfalls substituiertes C₁-C₆-Alkyl, Aryl-(C₁-C₃-alkyl), Heteroaryl- (C₁-C₃-alkyl), C₂-C₆-Alkenyl, C₂-C₆-Alkinyl, C₃-C₆-Cycloalkyl, (C₁-C₂-Alkyl)-(C₃-C₆- cycloalkyl), (C₃-C₆-Cycloalkyl)-(C₁-C₂-alkyl), (C₁-C₆-Alkyl)carbonyl und (C₁-C₆- Alkoxy)carbonyl steht;
- m: den Wert 1 annimmt;
- Q: für ein gegebenenfalls mehrfach mit V substituiertes Phenyl, Pyridin, Oxadiazol, Oxazol, Isoxazol, Pyrazol, Thiazol, Thiadiazol, Triazol steht, wobei
- V: für Halogen, Cyano, Nitro, gegebenenfalls substituiertes C₁-C₆-Alkyl, C₁-C₄-Alkenyl, C₁- C₄-Alkinyl, C₁-C₆-Halogenalkyl, C₃-C₆-Cycloalkyl, C₃-C₆-Halogencycloalkyl, C₁-C₆- Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino steht,
- T: für einen der unten dargestellten Reste (T-1) - (T-56) stehen kann, 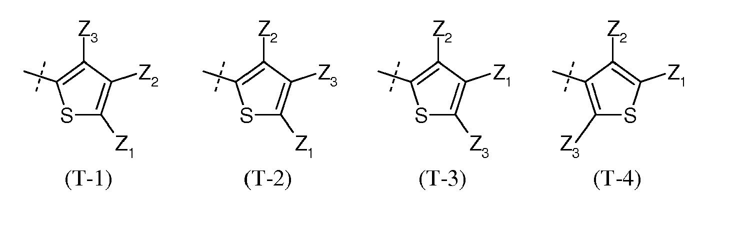 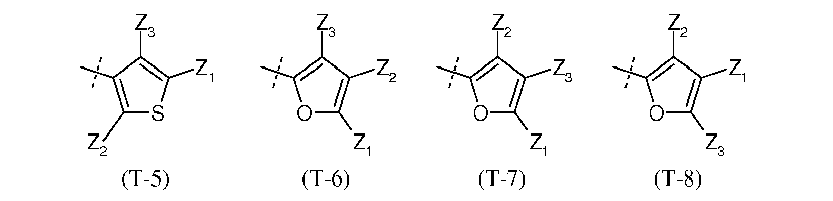 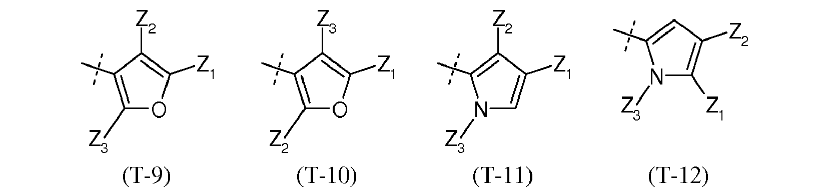 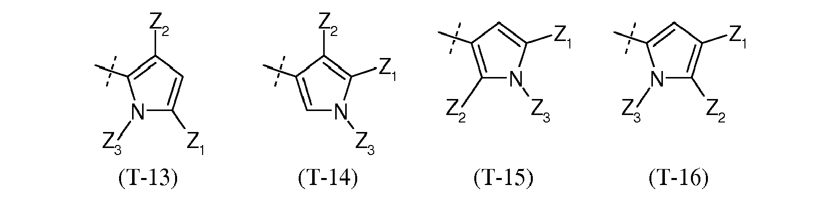 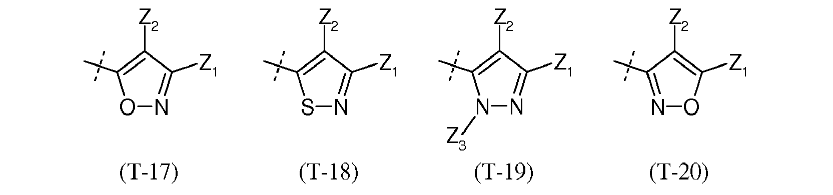 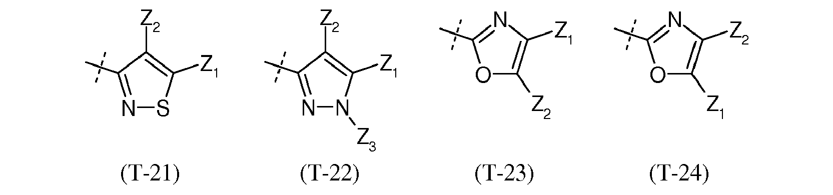 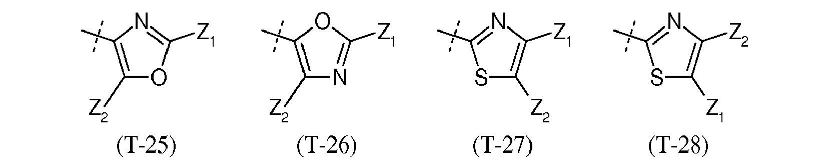 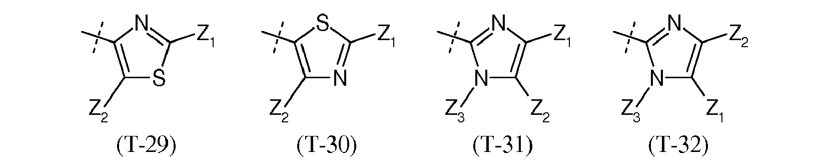 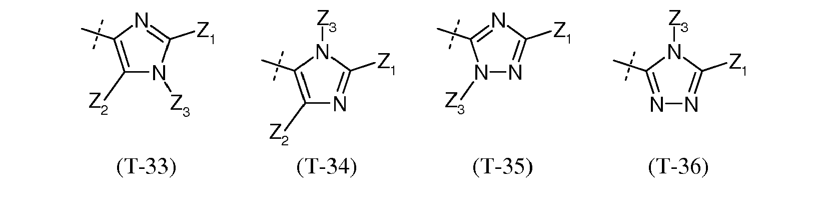 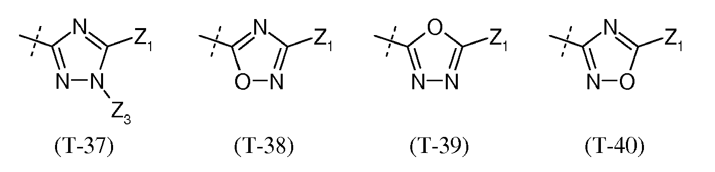 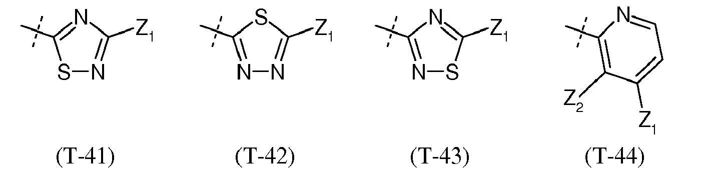 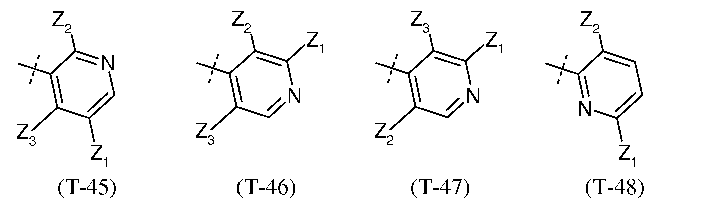 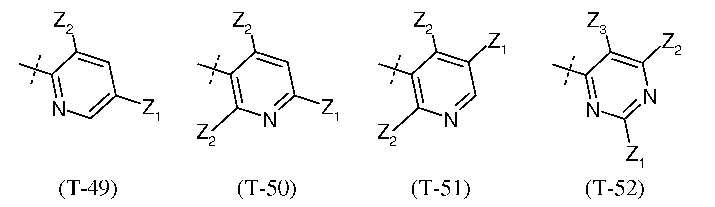 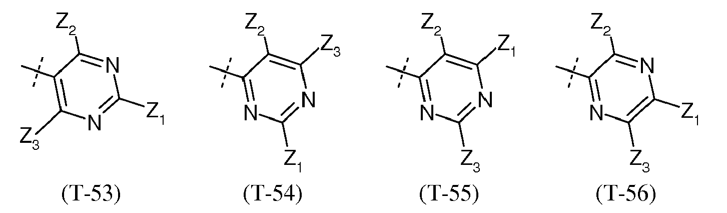 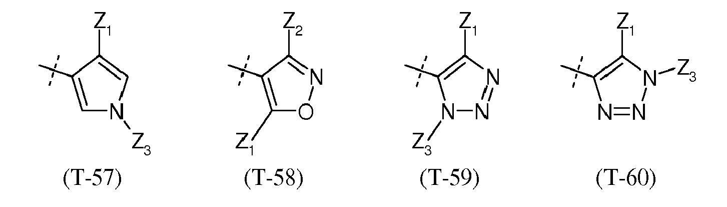
wobei
- Z₁: für ein gegebenenfalls substituiertes C₁-C₆-Halogenalkyl, C₃-C₆-Halogencycloalkyl, C₁-C₆- Halogenalkoxy, C₁-C₆-Halogenalkylthio, C₁-C₆-Halogenalkylsulfinyl, C₁-C₆- Halogenalkylsulfonyl, (C₃-C₆-Cycloalkyl)carbonyl, (C₃-C₆-Cycloalkyl)-(C₁-C₃-alkyl), Aryl-(C₁-C₃-alkyl), Heteroaryl-(C₁-C₃-alkyl) oder gegebenenfalls mit R¹⁴ substituiertes Phenyl und Pyridinyl steht,
- Z₂: für Wasserstoff, Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆-Halogen- alkylsulfonyl, *N*,*N*-Di-(C₁-C₆-alkyl)amino, -C(=W)NR⁷R⁶, -C(=W)OR⁸, -S(O)₂NR⁹R¹⁰, -S(O)ₚR¹¹, -S(O)(=NR¹²)R¹³ und gegebenenfalls mit R¹⁴ substituiertes Phenyl und Pyridinyl steht;
- Z₃: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆- Halogenalkoxy, C₁-C₆-Alkylthio, C₁-C₆-Halogenalkylthio, und gegebenenfalls mit R¹⁴ substituiertes Phenyl und Pyridinyl steht;
- R⁷: ausgewählt ist aus Wasserstoff oder den gegebenenfalls substituierten Gruppierungen C₁- C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
- R⁸: ausgewählt ist aus Wasserstoff oder den gegebenenfalls mit R⁶ substituierten Gruppierung C₁-C₆-Alkyl, C₁-C₆-Halogenlkyl, C₂-C₆-Alkenyl, C₂-C₆-Halogenalkenyl, C₂-C₆-Alkinyl, C₃-C₆-Cycloalkyl, C₃-C₆-Halogencycloalkyl, (C₁-C₂-Alkyl)-(C₃-C₆-cycloalkyl) und (C₃-C₆- Cycloalkyl)-(C₁-C₂-Alkyl);
- R⁹: ausgewählt ist aus Wasserstoff oder den gegebenenfalls substituierten Gruppierungen C₁- C₆-Alkyl, C₁-C₆-Halogenalkyl, C₂-C₆-Alkenyl, C₂-C₆-Alkinyl, C₃-C₆-Cycloalkyl, (C₁-C₂- Alkyl)-(C₃-C₆-cycloalkyl), (C₃-C₆-Cycloalkyl)-(C₁-C₂-Alkyl), (C₁-C₆-Alkyl)carbonyl und (C₁-C₆-Alkoxy)carbonyl;
- R¹⁰: ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-Alkyl);
- R¹¹: ausgewählt ist aus den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl), (C₃- C₆-Cycloalkyl)-(C₁-C₂-alkyl) und C₁-C₄-Haloalkyl;
- R¹²: ausgewählt ist aus Wasserstoff, C₁-C₆-Alkyl, C₁-C₆-Halogenalkyl, C₂-C₆-Alkenyl, C₂-C₆- Alkinyl, C₃-C₆-Cycloalkyl, (C₁-C₂-Alkyl)-(C₃-C₆-cycloalkyl), (C₃-C₆-Cycloalkyl)-(C₁-C₂- alkyl), (C₁-C₆-Alkyl)carbonyl und (C₁-C₆-Alkoxy)carbonyl;
- R¹³: ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-alkyl);
- R¹⁴: ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₁-C₆-Alkylamio, *N*,*N*-Di-(C₁-C₆-alkyl)amino, (C₁-C₆- Alkyl)carbonyl, (C₁-C₆-Alkoxy)carbonyl, *N*-(C₁-C₆-Alkyl)amiocarbonyl und *N*,*N*-Di-(C₁- C₆-alkyl)aminocarbonyl;
- R¹⁵: ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆- Alkylsulfonyl, -CN, -NO₂ sowie gegebenenfalls mit R¹⁶ substituiertem Phenyl oder Pyridyl;
- R¹⁶: ausgewählt ist aus Halogen, -CN, -NO₂, C₁-C₆-Alkyl, C₁-C₆-Alkoxy, C₁-C₆-Alkylthio, C₁- C₆-Alkylsulfinyl, C₁-C₆-Alkylsulfonyl, (C₁-C₆-Alkyl)carbonyl, (C₁-C₆-Alkoxy)carbonyl, *N*-(C₁-C₆-Alkyl)aminocarbonyl, oder gegebenenfalls mit R¹⁸ substituiertem Phenyl oder Pyridyl;
- R¹⁷: ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆- Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*-C₁-C₆- Alkylamino, *N*,*N*-Di-(C₁-C₆-alkyl)amino, (C₁-C₃-Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
- R¹⁸: ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, (C₁-C₃- Alkyl)carbonyl, (C₁-C₃-Alkoxy)carbonyl, *N*-(C₁-C₆-Alkyl)aminocarbonyl, *N*,*N*-Di-(C₁-C₆- alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Ganz besonders bevorzugt sind Verbindungen der Formel (I) 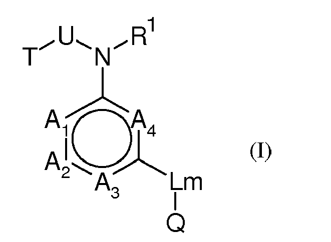 in denen
- R¹: für Wasserstoff, gegebenenfalls substituiertes C₁-C₆-Alkyl, C₂-C₆-Alkylen, C₂-C₆-Alkinyl, C₃-C₇-Cycloalkyl, (C₁-C₆-Alkyl)carbonyl, (C₁-C₆-Alkoxy)carbonyl, Cyan-(C₁-C₂-alkyl), (C₁-C₂-Alkoxy)-(C₁-C₂-alkyl), Aryl(C₁-C₃-alkyl), Heteroaryl(C₁-C₃-alkyl) steht,
die chemischen Gruppierungen
- A₁: für CR², einen gegebenenfalls mit G substituierten Stickstoff, Sauerstoff, Schwefel oder eine Bindung"
- A₂: für CR³ oder einen gegebenenfalls mit G substituierten Stickstoff, Sauerstoff oder Schwefel,
- A₃: für CR⁴ oder einen gegebenenfalls mit G substituierten Stickstoff, Sauerstoff oder Schwefel und
- A₄: für CR⁵ oder einen gegebenenfalls mit G substituierten Stickstoff, Sauerstoff oder Schwefel stehen,
wobei aber nicht mehr als drei der chemischen Gruppierungen A₁ bis A₄ gleichzeitig für Stickstoff stehen;
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, *N*-(C₁-C₆-Alkyl)aminocarbonyl, *N*-(C₃-C₆-Cycloalkyl)aminocarbonyl oder (C₁-C₄-Alkoxy)carbonyl, stehen; wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoff an das sie gebunden sind einen 6-gliedrigen Ring bilden, der 0, 1 oder 2 Stickstoffatome enthält;
- G: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
- U: für eine Gruppierung C(=W), SO oder SO₂ steht,
wobei
- W: für Sauerstoff oder Schwefel steht;
- L: für eine bivalente chemische Gruppierung steht, die ausgewählt ist aus den Gruppierungen -C(=O)NH-, -C(=O)NR⁶, -C(=O)NHSO₂-, bei der die rechte Seite der Gruppierung direkt an den Rest Q anknüpft und wobei,
- R⁶: für Wasserstoff, gegebenenfalls substituiertes C₁-C₆-Alkyl, Aryl-(C₁-C₃-alkyl), Heteroaryl- (C₁-C₃-alkyl), C₂-C₆-Alkenyl, C₂-C₆-Alkinyl, C₃-C₆-Cycloalkyl, (C₁-C₂-Alkyl)-(C₃-C₆- cycloalkyl), (C₃C₆-Cycloalkyl)-(C₁-C₂alkyl), (C₁-C₆-Alkyl)carbonyl und (C₁-C₆- Alkoxy)carbonyl steht;
- m: den Wert 1 annimmt;
- Q: für einen der unten dargestellten Reste (Q-1) - (Q-8) steht, 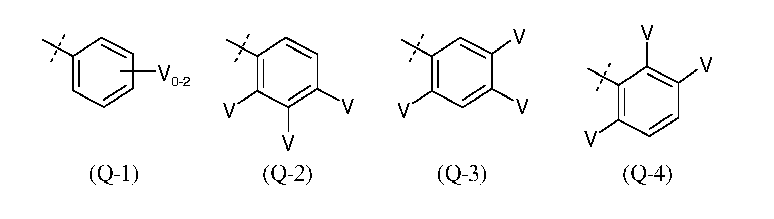 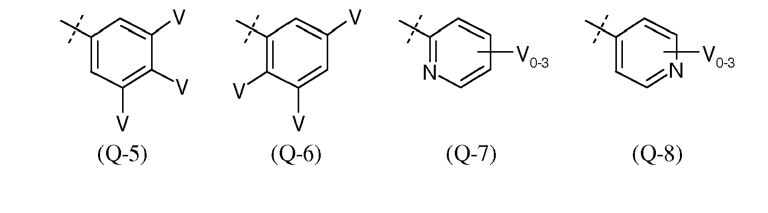
wobei
- V: für Halogen, Cyano, Nitro, gegebenenfalls substituiertes C₁-C₆-Alkyl, C₁-C₄-Alkenyl, C₁- C₄-Alkinyl, C₁-C₆-Halogenalkyl, C₃-C₆-Cycloalkyl, C₃-C₆-Halogencycloalkyl, C₁-C₆- Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino steht;
- T: für (T-1), (T-2), (T-3), (T-4), (T-5), (T-8), (T-9), (T-11), (T-12), (T-13), (T-14), (T-17), (T- 18), (T-19), (T-20), (T-21), (T-22), (T-23), (T-24), (T-25), (T-26), (T-27), (T-28), (T-29), (T-30), (T-31), (T-33), (T-34), (T-35), (T-44) (T-45), (T-46), (T-47), (T-48), (T-49), (T- 50), (T-51), (T-53), (T-55), (T-57),(T-58),(T-59),(T-60),
wobei
- Z₁: für ein gegebenenfalls substituiertes C₁-C₆-Halogenalkyl, C₃-C₆-Halogencycloalkyl,, C₁- C₆-Halogenalkoxy, C₁-C₆-Halogenalkylthio, C₁-C₆-Halogenalkylsulfinyl, C₁-C₆- Halogenalkylsulfonyl, (C₃-C₆-Cycloalkyl)carbonyl, (C₃-C₆-Cycloalkyl)-(C₁-C₃-alkyl), Aryl-(C₁-C₃-alkyl), Heteroaryl-(C₁-C₃-alkyl) oder gegebenenfalls mit R¹⁴ substituiertes Phenyl und Pyridinyl steht,
- Z₂: für Wasserstoff, Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆- Alkylsulfinyl, C₁-C₆-Halogenalkylsulfinyl, C₁-C₆-Alkylsulfonyl, C₁-C₆- Halogenalkylsulfonyl, *N*,*N*-Di-(C₁-C₆-alkyl)amino, -C(=W)NR⁷R⁶, -C(=W)OR⁸, -S(O)₂NR⁹R¹⁰, -S(O)pR¹¹, -S(O)(=NR¹²)R¹³ und gegebenenfalls mit R¹⁴ substituiertes Phenyl und Pyridinyl steht;
- Z₃: für Wasserstoff, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
- R⁷: ausgewählt ist aus Wasserstoff oder den gegebenenfalls substituierten Gruppierungen C₁- C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
- R⁸: ausgewählt ist aus Wasserstoff oder den gegebenenfalls mit R⁶ substituierten Gruppierung C₁-C₆-Alkyl, C₁-C₆-Halogenlkyl, C₂-C₆-Alkenyl, C₂-C₆-Halogenalkenyl, C₂-C₆-Alkinyl, C₃-C₆-Cycloalkyl, C₃-C₆-Halogencycloalkyl, (C₁-C₂-Alkyl)-(C₃-C₆-cycloalkyl) und (C₃-C₆- Cycloalkyl)-(C₁-C₂-Alkyl);
- R⁹: ausgewählt ist aus Wasserstoff oder den gegebenenfalls substituierten Gruppierungen C₁- C₆-Alkyl, C₁-C₆-Halogenalkyl, C₂-C₆-Alkenyl, C₂-C₆-Alkinyl, C₃-C₆-Cycloalkyl, (C₁-C₂- Alkyl)-(C₃-C₆-cycloalkyl), (C₃-C₆-Cycloalkyl)-(C₁-C₂-Alkyl), (C₁-C₆-Alkyl)carbonyl und (C₁-C₆-Alkoxy)carbonyl;
- R¹⁰: ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-Alkyl);
- R¹¹: ausgewählt ist aus den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl), (C₃- C₆-Cycloalkyl)-(C₁-C₂-alkyl) und C₁-C₄-Haloalkyl;
- R¹²: ausgewählt ist aus Wasserstoff, C₁-C₆-Alkyl, C₁-C₆-Halogenalkyl, C₂-C₆-Alkenyl, C₂-C₆- Alkinyl, C₃-C₆-Cycloalkyl, (C₁-C₂-Alkyl)-(C₃-C₆-cycloalkyl), (C₃-C₆-Cycloalkyl)-(C₁-C₂- alkyl), (C₁-C₆-Alkyl)carbonyl und (C₁-C₆-Alkoxy)carbonyl;
- R¹³: ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂- Alkyl)-(C₃-C₆-cycloalkyl) und (C₃-C₆-Cycloalkyl)-(C₁-C₂-alkyl);
- R¹⁴: ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-C₁-C₆-Alkylamino, *N*,*N*-Di-(C₁-C₆-alkyl)amino, (C₁-C₆- Alkyl)carbonyl, (C₁-C₆-Alkoxy)carbonyl, *N*-(C₁-C₆-Alkyl)aminocarbonyl und *N*,*N*-Di-(C₁- C₆-alkyl)aminocarbonyl;
- R¹⁵: ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆-
- R¹⁶: Alkylsulfonyl, -CN, -NO₂ sowie gegebenenfalls mit R¹⁶ substituiertem Phenyl oder Pyridyl; ausgewählt ist aus Halogen, -CN, -NO₂, C₁-C₆-Alkyl, C₁-C₆-Alkoxy, C₁-C₆-Alkylthio, C₁- C₆-Alkylsulfinyl, C₁-C₆-Alkylsulfonyl, (C₁-C₆-Alkyl)carbonyl, (C₁-C₆-Alkoxy)carbonyl, *N*-(C₁-C₆-Alkyl)aminocarbonyl, oder gegebenenfalls mit R¹⁸ substituiertem Phenyl oder Pyridyl;
- R¹⁷: ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆- Halogenalkoxy, C₁-C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*-C₁-C₆- Alkylamino, *N*,*N*-Di-(C₁-C₆-alkyl)amino, (C₁-C₃-Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
- R¹⁸: ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, (C₁-C₃- Alkyl)carbonyl, (C₁-C₃-Alkoxy)carbonyl, *N*-(C₁-C₆-Alkyl)aminocarbonyl, *N*,*N*-Di-(C₁-C₆- alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.
Ganz besonders bevorzugt sind auch Verbindungen der Formel (I) 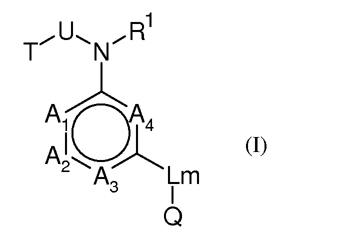 in denen
- R¹: für Wasserstoff, Methyl, Ethyl, n-Propyl, Isopropyl, n-Butyl, Isobutyl, sec-Butyl, tert- Butyl, Methoxymethyl, Ethoxymethyl, Propoxymethyl, Methylcarbonyl, Ethylcarbonyl, n- Propylcarbonyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl steht;
die chemischen Gruppierungen
- A₁: für CR², einen gegebenenfalls mit G substituierten Stickstoff, Sauerstoff, Schwefel oder eine Bindung,
- A₂: für CR³ oder einen gegebenenfalls mit G substituierten Stickstoff, Sauerstoff oder Schwefel,
- A₃: für CR⁴ oder einen gegebenenfalls mit G substituierten Stickstoff, Sauerstoff oder Schwefel und
- A₄: für CR⁵ oder einen gegebenenfalls mit G substituierten Stickstoff, Sauerstoff oder Schwefel stehen,
wobei aber nicht mehr als drei der chemischen Gruppierungen A₁ bis A₄ gleichzeitig für Stickstoff stehen;
- R² und R⁵: unabhängig voneinander für Wasserstoff, Methyl, Fluor und Chlor stehen und
- R³ und R⁴: unabhängig voneinander für Wasserstoff, Fluor, Chlor, Brom, Cyano, Nitro, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N*-Cyclopropylaminocarbonyl stehen;
- G: für Wasserstoff, Methyl, Ethyl, n-Propyl, Isopropyl, n-Butyl, Isobutyl, sec-Butyl, tert- Butyl" Ethenyl, 1-Propenyl, 2-Propenyl, Fluormethyl, Difluormethyl, Trifluormethyl, Chlormethyl, Pentafluorethyl, 2,2,2-Trifluorethyl, Cyclopropyl, Cyclobutyl, Methoxymethyl, Ethoxymethyl, Propoxymethyl, 2-Methoxyethoxymethyl, Methylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2- Cyanoethyl, Phenyl, 4-Fluorphenyl, 4-Chlorphenyl, 4-Trifluormethylphenyl, 2,4- Dichlorphenyl, 2-Chlor-4-trifluormethyl-phenyl steht;
wobei
- U: für C(=W) und,
- W: für Sauerstoff steht;
- L: für eine bivalente chemische Gruppierung steht, die ausgewählt ist aus den Gruppierungen -C(=O)NH-, -C(=O)NR⁶, -C(=O)NHSO₂-, bei der die rechte Seite der Gruppierung direkt an den Rest Q anknüpft und wobei,
- R⁶: für Wasserstoff, Methyl, Ethyl, n-Propyl, Isopropyl, n-Butyl, Isobutyl, sec-Butyl, tert- Butyl, Methoxymethyl, Ethoxymethyl, Propoxymethyl, Methylcarbonyl, Ethylcarbonyl, n- Propylcarbonyl, Isopropylcarbonyl, sec-Butylcarbonyl, tert-Butylcarbonyl, Methoxycarbonyl, Ethoxycarbonyl, n-Propoxycarbonyl, Isopropoxycarbonyl, sec- Butoxycarbonyl, tert-Butoxycarbonyl, Cyanomethyl, 2-Cyanoethyl, Pyrid-2-yl-methyl, Pyrid-3-yl-methyl und Pyrid-4-yl-methyl steht;
- m: den Wert 1 annimmt;
- Q: für einen der unten dargestellten Reste (Q-1) - (Q-8) steht, 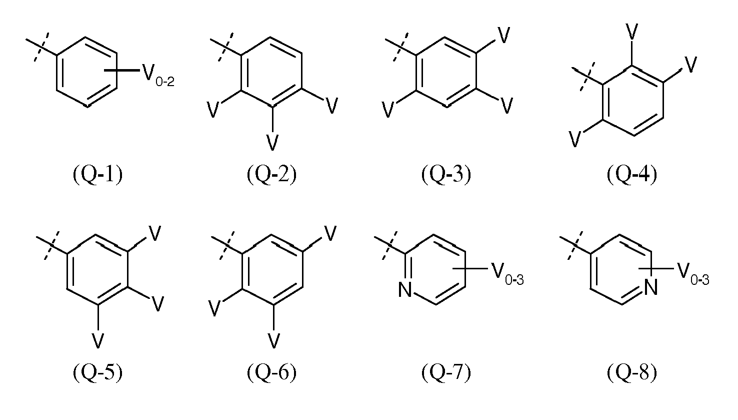
- V: für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1- Fluorethyl, 2-Fluorethyl, 2,2-Difluorethyl, 2,2,2-Trifluorethyl, 1,2,2,2-Tetrafluorethyl, 1- Chlor-1,2,2,2-tetrafluorethyl, 2,2,2-Trichlorethyl, 2-Chlor-2,2-difluorethyl, 1,1- Dilfluorethyl, Pentafluorethyl Pentafluor-tert-butyl, Heptafluor-n-propyl, Heptafluor- isopropyl, Nonafluor-n-butyl, Cyclopropyl, Cyclobutyl, Methoxy, Ethoxy, n-Propoxy, 1- Methylethoxy, Fluormethoxy, Difluormethoxy, Chlor-difluormethoxy, Dichlor- fluormethoxy, Trifluormethoxy, 2,2,2-Trifluorethoxy, 2-Chlor-2,2-difluorethoxy, Pentafluorethoxy, Methylthio, Methylsulfonyl, Methylsulfinyl, Trifluormethylsulfonyl, Trifluormethylsulfinyl steht;
- T: für (T-8), (T-9), (T-11), (T-17), (T-18), (T-19), (T-21), (T-29), (T-35), (T-44), (T-49), (T- 50), (T-53), (T-55), (T-57),
wobei
- Z₁: für Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2- Fluorethyl, 2,2-Difluorethyl, 2,2,2-Trifluorethyl, 1,2,2,2-Tetrafluorethyl, 1-Chlor-1,2,2,2- tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Trifluormethoxy-1,1,2,2-tetrafluorethoxy-difluormethyl, Trifluor- methylthio, Trifluormethylsulfinyl, Trifluormethylsulfonyl, Trifluormethoxy, Difluor- methoxy, Cyclopropyl, Cyclobutyl, Cyclopropyl-hydroxymethyl, Cyclopropyl- fluormethyl, Cyclopropylcarbonyl, 2,2,2-Trifluorethoxy, 1-Trifluormethylethoxy, 3,3,3,2,2-Pentafluorpropoxy, Phenyl, 2-Chlorphenyl, 3-Chlorphenyl, 4-Chlorphenyl, 4- Fluorphenyl, 2-Methylphenyl, 3-Methylphenyl, 4-Methylphenyl, 3-Trifluormethylphenyl, 4-Trifluormethylphenyl, 4-Nitrophenyl, 4-(tert-Butyl)phenyl, 4-Methoxyphenyl, 4- Phenoxyphenyl, 2,4-Dichlorphenyl, 3,4-Dichlorphenyl, 3,5-Dichlorphenyl, 2,5- Dichlorphenyl, 2,6-Difluorphenyl, 4-Trifluormethoxyphenyl, 4-Cyanophenyl, 2- Trifluormethyl-pyrid-5-yl, 3-Chlor-5-trifluormethyl-pyrid-2-yl, (3,5-Bis(trifluormethyl)- 1H-pyrazol-1-yl)-methyl, (3,5-Bis(pentafluorethyl)-1H-pyrazol-1-yl)-methyl, (3,5- Bis(trifluormethyl)-1H-triazol-1-yl)-methyl, (2-Methyl-2-H-tetrazol-5-yl)-methyl, (5- Trifluormethyl-2H-tetrazol-2-yl)-methyl, (3,5-Dichlorphenyl)-methyl, (3,5-Bis(trifluor- methyl)phenyl)-methyl, (2,4-Dichlorphenyl)-methyl, (2,6-Dichlorphenyl)-methyl, (4- Chlorphenyl)-methyl, (4-Trifluormethylphenyl)-methyl steht,
- Z₂: für Wasserstoff, Halogen, Cyano, Nitro, Methyl, Ethyl, n-Propyl, Isopropyl, n-Butyl, Isobutyl, sec-Butyl, tert-Butyl, Ethenyl, 1-Propenyl, 2-Propenyl, Ethinyl, 1-Propinyl, 1- Butinyl, Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2- Fluorethyl, 2,2-Difluorethyl, 2,2,2-Trifluorethyl, 1,2,2,2-Tetrafluorethyl, 1-Chlor-1,2,2,2- tetrafluorethyl, 2,2,2-Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n-butyl, Cyclopropyl, Cyclobutyl, Methoxy, Ethoxy, n-Propoxy, 1- Methylethoxy, Fluormethoxy, Difluormethoxy, Chlor-difluormethoxy, Dichlor- fluormethoxy, Trifluormethoxy, 2,2,2-Trifluorethoxy, 2-Chlor-2,2-difluorethoxy, Pentafluorethoxy, Methylthio, Methylsulfonyl, Methylsulfinyl, Trifluormethylsulfonyl, Trifluormethylsulfinyl, *N*,*N*-Dimethylamino, 4-Fluorphenyl, 4-Chlorphenyl, 4- Trifluormethylphenyl steht;
- Z₃: für Wasserstoff, Methyl, Ethyl, n-Propyl, Isopropyl, n-Butyl, Isobutyl, sec-Butyl, tert- Butyl" Ethenyl, 1-Propenyl, 2-Propenyl, Fluormethyl, Difluormethyl, Trifluormethyl, Chlormethyl, Pentafluorethyl, 2,2,2-Trifluorethyl, Cyclopropyl, Cyclobutyl, Methoxymethyl, Ethoxymethyl, Propoxymethyl, 2-Methoxyethoxymethyl, Methyl- carbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2-Cyanoethyl, Phenyl, 4- Fluorphenyl, 4-Chlorphenyl, 4-Trifluormethylphenyl, 2,4-Dichlorphenyl, 2-Chlor-4- trifluormethyl-phenyl steht;
ferner können
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

Insbesondere bevorzugt sind Verbindungen der allgemeinen Formel (Iaa), 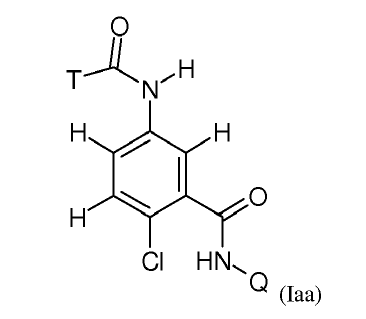 wobei der Rest T ausgewählt sein kann aus:
Methyl-3-(pentafluorethyl)-4-(trifluormethyl)-1H-pyrazol-5-yl, 2-Methyl-6-(trifluormethyl)pyridin-3-yl, 2-(Trifluormethyl)-1,3-thiazol-4-yl, 4-Brom-1-methyl-3-(trifluormethyl)-1H-pyrazol-5-yl, 4-(Difluormethyl)-2-(pentafluorethyl)pyrimidin-5-yl, 4-Cyclopropyl-2-(pentafluorethyl)pyrimidin-5-yl, 2,4-Bis(pentafluorethyl)pyrimidin-5-yl, 3-{[3,5-Bis(pentafluorethyl)-1H-pyrazol-1-yl]methyl}-1-methyl-4-(trifluormethyl)-1H-pyrazol-5-yl,und
der Rest Q ausgewählt sein kann aus: Pyridin-4-yl, Pyridin-3-yl, 4-Fluorphenyl, 4-Chlorpyridin-3-yl, 2-Chlorpyridin-4-yl, 6-Chlorpyridin-3-yl, Pyridin-2-yl, 3-Chlorphenyl, 4-Methoxyphenyl, 2-Chlorpyridin-3-yl, Phenyl, 3-Methoxyphenyl, 6-Chlorpyridin-2-yl, 2,4-Difluorphenyl, 4-Fluor-2-methylphenyl, 4-Chlorpyridin-2-yl, 4-Fluor-3-methylphenyl, 3-Chlor-4-fluor-2-methylphenyl, 5-Chlorpyridin-2-yl, 4-Fluor-3-(trifluormethyl)phenyl, 3,4-Dichlorphenyl, 3,4,5-Trichlorphenyl.

Erfindungsgemäß steht "Alkyl" - in Alleinstellung oder als Bestandteil einer chemischen Gruppe - für geradkettige oder verzweigte Kohlenstoffwasserstoffe, vorzugsweise mit 1 bis 6 Kohlenstoffatomen, wie beispielsweise Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylpropyl, 1,3-Dimethylbutyl, 1,4-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl. Ferner bevorzugt für Alkyle mit 1 bis 4 Kohlenstoffatomen, wie unter anderem Methyl, Ethyl, Ethyl, n-Propyl, Isopropyl, n-Butyl, Isobutyl, sec-Butyl oder tert-Butyl. Die erfindungsgemäßen Alkyle können mit einem oder mehreren, gleichen oder verschiedenen Resten substituiert sein.

Erfindungsgemäß steht "Alkenyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Doppelbindung, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-dimethyl-3-butenyl, 1,2-Dimethyl-2-butenyl, 1,2-Dimethyl-3-butenyl, 1,3-Dimethyl-2-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl. Ferner bevorzugt für Alkenyle mit 2 bis 4 Kohlenstoffatomen, wie unter anderem 2-Propenyl, 2-Butenyl oder 1-Methyl-2-propenyl. Die erfindungsgemäßen Alkenyle können mit einem oder mehreren, gleichen oder verschiedenen Resten substituiert sein.

Erfindungsgemäß steht "Alkinyl" - in Alleinstellung oder als Bestandteil einer chemischen Gruppe - für geradkettige oder verzweigte Kohlenstoffwasserstoffe, vorzugsweise mit 2 bis 6 Kohlenstoffatomen und mindestens einer Dreifachbindung wie beispielsweise 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-3-butinyl, 1,2-Dimethyl-3-butinyl, 2,2-Dimethyl-3-butinyl, 1-Ethyl-3-butinyl, 2-Ethyl-3-butinyl, 1-Ethyl-1-methyl-2-propinyl und 2,5-Hexadiynyl. Ferner bevorzugt für Alkinyle mit 2 bis 4 Kohlenstoffatomen wie unter anderem Ethinyl, 2-Propinyl oder 2-Butinyl-2-propenyl. Die erfindungsgemäßen Alkinyle können mit einem oder mehreren, gleichen oder verschiedenen Resten substituiert sein.

Erfindungsgemäß steht "Cycloalkyl" - in Alleinstellung oder als Bestandteil einer chemischen Gruppe - für mono-, bi- oder tricyclische Kohlenwasserstoffe, vorzugsweise mit 3 bis 10 Kohlenstoffen wie beispielsweise Cyclopropyl, Cyclobutyl, Cyclopentyl, Cyclohexyl, Cycloheptyl, Cyclooctyl, Bicyclo[2.2.1]heptyl, Bicyclo[2.2.2]octyl oder Adamantyl. Ferner bevorzugt für Cycloalkyle mit 3, 4, 5, 6 oder 7 Kohlenstoffatomen, wie unter anderem Cyclopropyl oder Cyclobutyl. Die erfindungsgemäßen Cycloalkyle können mit einem oder mehreren, gleichen oder verschiedenen Resten substituiert sein.

Erfindungsgemäß steht "Alkylcycloalkyl" für mono-, bi- oder tricyclisches Alkylcycloalkyl, vorzugsweise mit 4 bis 10 oder 4 bis 7 Kohlenstoffatomen, wie beispielsweise Ethylcyclopropyl, Isopropylcyclobutyl, 3-Methylcyclopentyl und 4-Methyl-cyclohexyl. Ferner bevorzugt für Alkylcycloalkyle mit 4, 5 oder 7 Kohlenstoffatomen wie unter anderen Ethylcyclopropyl oder 4-Methyl-cyclohexyl. Die erfindungsgemäßen Alkylcycloalkyle können mit einem oder mehreren, gleichen oder verschiedenen Resten substituiert sein.

Erfindungsgemäß steht "Cycloalkylalkyl" für mono, bi- oder tricyclisches Cycloalkylalkyl, vorzugsweise mit 4 bis 10 oder 4 bis 7 Kohlenstoffatomen, wie beispielsweise Cyclopropylmethyl, Cyclobutylmethyl, Cyclopentylmethyl, Cyclohexylmethyl und Cyclopentylethyl. Ferner bevorzugt für Cycloalkylalkyle mit 4, 5 oder 7 Kohlenstoffatomen wie unter anderen Cyclopropylmethyl oder Cyclobutylmethyl. Die erfindungsgemäßen Cycloalkylalkyle können mit einem oder mehreren, gleichen oder verschiedenen Resten substituiert sein.

Erfindungsgemäß steht "Halogen" für Fluor, Chlor, Brom oder Iod, insbesondere für Fluor, Chlor oder Brom.

Die erfindungsgemäßen mit Halogen substituierten chemischen Gruppen, wie beispielsweise Halogenalkyl, Halogencycloalkyl, Halogenalkyloxy, Halogenalkylthio, Halogenalkylsunfinyl oder Halogenalkylsulfonyl sind einfach oder mehrfach bis zur maximal möglichen Substituentenzahl mit Halogen substituiert. Bei mehrfacher Substitution mit Halogen, können die Halogenatome gleich oder verschieden sein und können alle an eines oder an mehrere Kohlenstoffatome gebunden sein. Dabei steht Halogen insbesondere für Fluor, Chlor, Brom oder Iod, vorzugsweise für Fluor, Chlor oder Brom und besonders bevorzugt für Fluor.

Erfindungsgemäß steht "Halogencycloalkyl" für mono-, bi- oder tricyclisches Halogencycloalkyl, vorzugsweise mit 3 bis 10 Kohlenstoffatomen, wie unter anderen 1-Fluor-cyclopropyl, 2-Fluor-cyclopropyl oder 1-Fluor-cyclobutyl. Ferner bevorzugt für Halogencycloalkyl mit 3, 5 oder 7 Kohlenstoffatomen. Die erfindungsgemäßen Halogencycloalkyle können mit einem oder mehreren, gleichen oder verschiedenen Resten substituiert sein.

Erfindungsgemäß steht "Halogenalkyl" "Halogenalkenyl" oder "Halogenalkinyl" für mit Halogen substituierte Alkyle, Alkenyle oder Alkinyle mit vorzugsweise 1 bis 9 gleichen oder verschiedenen Halogenatomen, wie beispielsweise Monohaloalkyl (= Monohalogenalkyl) wie CH₂CH₂Cl, CH₂CH₂F, CHClCH₃, CHFCH₃, CH₂Cl, CH₂F; Perhaloalkyl wie CCl₃ oder CF₃ oder CF₂CF₃; Polyhaloalkyl wie CHF₂, CH₂F, CH₂CHFCl, CHCl₂, CF₂CF₂H, CH₂CF₃. Entsprechendes gilt für Haloalkenyl und andere durch Halogen substituierten Reste. Haloalkoxy ist z.B. OCF₃, OCHF₂, OCH₂F, OCF₂CF₃, OCH₂CF₃ und OCH₂CH₂Cl;

Weitere Beispiele für Halogenalkyle sind Trichlormethyl, Chlordifluormethyl, Dichlorfluormethyl, Chlormethyl, Brommethyl, 1-Fluorethyl, 2-Fluorethyl, 2,2-Difluorethyl, 2,2,2-Trifluorethyl, 2,2,2-Trichlorethyl, 2-Chlor-2,2-difluorethyl, Pentafluorethyl und Pentafluor-tert-butyl. Bevorzugt sind Halogenalkyle mit 1 bis 4 Kohlenstoffatomen und 1 bis 9, vorzugsweise 1 bis 5 gleichen oder verschiedenen Halogenatomen, die ausgewählt sind unter Fluor, Chlor oder Brom. Besonders bevorzugt sind Halogenalkyle mit 1 oder 2 Kohlenstoffatomen und mit 1 bis 5 gleichen oder verschiedenen Halogenatomen, die ausgewählt sind unter Fluor oder Chlor, wie unter anderen Difluormethyl, Trifluormethyl oder 2,2-Difluorethyl.

Erfindungsgemäß steht "Hydroxyalkyl" für geradkettigen oder verzweigten Alkohol, vorzugsweise mit 1 bis 6 Kohlenstoffatomen, wie beispielsweise Methanol, Ethanol, n-Propanol, Isopropanol, n-Butanol, Isobutanol, sec-Butanol und tert-Butanol. Ferner bevorzugt für Hydroxyalkylgruppen mit 1 bis 4 Kohlenstoffatomen. Die erfindungsgemäßen Hydroxyalkylgruppen können mit einem oder mehreren, gleichen oder verschiedenen Resten substituiert sein

Erfindungsgemäß steht "Alkoxy" für geradkettiges oder verzweigtes O-Alkyl, vorzugsweise mit 1 bis 6 Kohlenstoffatomen, wie beispielsweise Methoxy, Ethoxy, n-Propoxy, Isopropoxy, n-Butoxy, Isobutoxy, sec-Butoxy und tert-Butoxy. Ferner bevorzugt für Alkoxygruppen mit 1 bis 4 Kohlenstoffatomen. Die erfindungsgemäßen Alkoxygruppen können mit einem oder mehreren, gleichen oder verschiedenen Resten substituiert sein.

Erfindungsgemäß steht "Halogenalkoxy" für mit Halogen substituiertes geradkettiges oder verzweigtes O-Alkyl, vorzugsweise mit 1 bis 6 Kohlenstoffatomen, wie unter anderem Difluormethoxy, Trifluormethoxy, 2,2-Difluorethoxy, 1,1,2,2-Tetrafluorethoxy, 2,2,2-Trifluorethoxy und 2-Chlor-1,1,2-trifluorethoxy. Ferner bevorzugt für Halogenalkoxygruppen mit 1 bis 4 Kohlenstoffatomen. Die erfindungsgemäßen Halogenalkoxygruppen können mit einem oder mehreren, gleichen oder verschiedenen Resten substituiert sein.

Erfindungsgemäß steht "Alkylthio" für geradkettiges oder verzweigtes S-Alkyl, vorzugsweise mit 1 bis 6 Kohlenstoffatomen, wie beispielsweise Methylthio, Ethylthio, n-Propylthio, Isopropylthio, n-Butylthio, Isobutylthio, sec-Butylthio und tert-Butylthio. Ferner bevorzugt für Alkylthiogruppen mit 1 bis 4 Kohlenstoffatomen. Die erfindungsgemäßen Alkylthiogruppen können mit einem oder mehreren, gleichen oder verschiedenen Resten substituiert sein.

Beispiele für Halogenalkylthioalkyle, d.h. mit Halogen substituierte Alkylthiogruppen, sind unter anderem Difluormethylthio, Trifluormethylthio, Trichlormethylthio, Chlordifluormethylthio, 1-Fluorethylthio, 2-Fluorethylthio, 2,2-Difluorethylthio, 1,1,2,2-Tetrafluorethylthio, 2,2,2-Trifluorethylthio oder 2-Chlor-1,1,2-trifluorethylthio.

Erfindungsgemäß steht "Alkylsulfinyl" für geradkettiges oder verzweigtes Alkylsulfinyl, vorzugsweise mit 1 bis 6 Kohlenstoffatomen wie beispielsweise Methylsulfinyl, Ethylsulfinyl, n-Propylsulfinyl, Isopropylsulfinyl, n-Butylsulfinyl, Isobutylsulfinyl, sec-Butylsulfinyl und tert-Butylsulfinyl. Ferner bevorzugt für Alkylsulfinylgruppen mit 1 bis 4 Kohlenstoffatomen. Die erfindungsgemäßen Alkylsulfinylgruppen können mit einem oder mehreren, gleichen oder verschiedenen Resten substituiert sein.

Beispiele für Halogenalkylsulfinylgrupen, d.h. mit Halogen substituierte Alkylsulfinylgruppen, sind unter anderem Difluormethylsulfinyl, Trifluormethylsulfinyl, Trichlormethylsulfinyl, Chlordifluormethylsulfinyl, 1-Fluorethylsulfinyl, 2-Fluorethylsulfinyl, 2,2-Difluorethylsulfinyl, 1,1,2,2-Tetrafluorethylsulfinyl, 2,2,2-Trifluorethylsulfinyl und 2-Chlor-1,1,2-trifluorethylsulfinyl.

Erfindungsgemäß steht "Alkylsulfonyl" für geradkettiges oder verzweigtes Alkylsulfonyl, vorzugsweise mit 1 bis 6 Kohlenstoffatomen wie beispielsweise Methylsulfonyl, Ethylsulfonyl, n-Propylsulfonyl, Isopropylsulfonyl, n-Butylsulfonyl, Isobutylsulfonyl, sec-Butylsulfonyl und tert-Butylsulfonyl. Ferner bevorzugt für Alkylsulfonylgruppen mit 1 bis 4 Kohlenstoffatomen. Die erfindungsgemäßen Alkylsulfonylgruppen können mit einem oder mehreren, gleichen oder verschiedenen Resten substituiert sein.

Beispiele für Halogenalkylsulfonylgrupen, d.h. mit Halogen substituierte Alkylsulfonylgruppen sind unter anderem Difluormethylsulfonyl, Trifluormethylsulfonyl, Trichlormethylsulfonyl, Chlordifluormethylsulfonyl, 1-Fluorethylsulfonyl, 2-Fluorethylsulfonyl, 2,2-Difluorethylsulfonyl, 1,1,2,2-Tetrafluorethylsulfonyl, 2,2,2-Trifluorethylsulfonyl und 2-Chlor-1,1,2-trifluorethylsulfonyl.

Erfindungsgemäß steht "Alkylcarbonyl" für geradkettiges oder verzweigtes Alkyl-C(=O), vorzugsweise mit 2 bis 7 Kohlenstoffatomen, wie Methylcarbonyl, Ethylcarbonyl, n-Propylcarbonyl, Isopropylcarbonyl, sec-Butylcarbonyl und tert-Butylcarbonyl. Ferner bevorzugt für Alkylcarbonyle mit 1 bis 4 Kohlenstoffatomen. Die erfindungsgemäßen Alkylcarbonyle können mit einem oder mehreren, gleichen oder verschiedenen Resten substituiert sein.

Erfindungsgemäß steht "Cycloalkylcarbonyl" für geradkettiges oder verzweigtes Cycloalkylcarbonyl, vorzugsweise mit 3 bis 10 Kohlenstoffatomen im Cycloalkylteil, wie beispielsweise Cyclopropylcarbonyl, Cyclobutylcarbonyl, Cyclopentylcarbonyl, Cyclohexylcarbonyl, Cycloheptylcarbonyl, Cyclooctylcarbonyl, Bicyclo[2.2.1]heptyl, Bycyclo[2.2.2]octylcarbonyl und Adamantylcarbonyl. Ferner bevorzugt für Cycloalkylcarbonyl mit 3, 5 oder 7 Kohlenstoffatomen im Cycloalkylteil. Die erfindungsgemäßen Cycloalkylcarbonylgruppen können mit einem oder mehreren, gleichen oder verschiedenen Resten substituiert sein.

Erfindungsgemäß steht "Alkoxycarbonyl" - in Alleinstellung oder als Bestandteil einer chemischen Gruppe - für geradkettiges oder verzweigtes Alkoxycarbonyl, vorzugsweise mit 1 bis 6 Kohlenstoffatomen oder 1 bis 4 Kohlenstoffatomen im Alkoxyteil, wie beispielsweise Methoxycarbonyl, Ethoxycarbonyl, n-Propoxycarbonyl, Isopropoxycarbonyl, sec-Butoxycarbonyl und tert-Butoxycarbonyl. Die erfindungsgemäßen Alkoxycarbonylgruppen können mit einem oder mehreren, gleichen oder verschiedenen Resten substituiert sein.

Erfindungsgemäß steht "Alkylaminocarbonyl" für geradkettiges oder verzweigtes Alkylaminocarbonyl mit vorzugsweise 1 bis 6 Kohlenstoffatomen oder 1 bis 4 Kohlenstoffatomen im Alkylteil, wie beispielsweise Methylaminocarbonyl, Ethylaminocarbonyl, n-Proylaminocarbonyl, Isopropylaminocarbonyl, sec-Butylaminocarbonyl und tert-Butylaminocarbonyl. Die erfindungsgemäßen Alkylaminocarbonylgruppen können mit einem oder mehreren, gleichen oder verschiedenen Resten substituiert sein.

Erfindungsgemäß steht "*N*,*N*-Dialkylamino-carbonyl" für geradkettiges oder verzweigtes *N*,*N-*Dialkylaminocarbonyl mit vorzugsweise 1 bis 6 Kohlenstoffatomen oder 1 bis 4 Kohlenstoffatomen im Alkylteil, wie beispielsweise *N*,*N*-Dimethylamino-carbonyl, N.N-Diethylamino-carbonyl, *N*,*N*-Di(n-propylamino)-carbonyl, *N*,*N*-Di-(isopropylamino)-carbonyl und *N*,*N*-Di-(sec-butylamino)-carbonyl. Die erfindungsgemäßen *N*,*N*-Dialkylamino-carbonylgruppen können mit einem oder mehreren, gleichen oder verschiedenen Resten substituiert sein.

Erfindungsgemäß steht "Aryl" für ein mono-, bi- oder polycyclisches aromatisches System mit vorzugsweise 6 bis 14, insbesondere 6 bis 10 Ring-Kohlenstoffatomen, wie beispielsweise Phenyl, Naphthyl, Anthryl, Phenanthrenyl, vorzugsweise Phenyl. Ferner steht Aryl auch für mehrcyclische Systeme, wie Tetrahydronaphtyl, Indenyl, Indanyl, Fluorenyl, Biphenyl, wobei die Bindungsstelle am aromatischen System ist. Die erfindungsgemäßen Arylgruppen können mit einem oder mehreren, gleichen oder verschiedenen Resten substituiert sein.

Beispiele substitutierter Aryle stellen die Arylalkyle dar, die gleichfalls mit einem oder mehreren, gleichen oder verschiedenen Resten im Alkyl- und/oder Arylteil substituiert sein können. Beispiele solcher Arylalkyle sind unter anderem Benzyl und 1-Phenylethyl.

Erfindungsgemäß steht "Heterocyclus", "heterocyclischer Ring" oder "heterocyclisches Ringsystem" für ein carbocyclisches Ringsystem mit mindestens einem Ring, in dem mindestens ein Kohlenstoffatom durch ein Heteroatom ersetzt ist, vorzugsweise durch ein Heteroatom aus der Gruppe N, O, S, P, B, Si, Se und der gesättigt, ungesättigt oder heteroaromatisch ist und dabei unsubstituiert oder mit einem Substituenten Z substituiert sein kann, wobei die Bindungsstelle an einem Ringatom lokalisiert ist. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen. Die heterocyclischen Ringe enthalten gewöhnlicherweise nicht mehr als 4 Stickstoffatome, und/oder nicht mehr als 2 Sauerstoffatome und/oder nicht mehr als 2 Schwefelatome. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden erfindungsgemäß auch mehrcyclische Systeme umfaßt, wie beispielsweise 8-Aza-bicyclo[3.2.1]octanyl oder 1-Aza-bicyclo[2.2.1]heptyl. Im Falle von gegebenenfalls substituiertem Heterocyclyl werden erfindungsgemäß auch spirocyclische Systeme umfasst, wie beispielsweise 1-Oxa-5-aza-spiro[2.3]hexyl.

Erfindungsgemäße Heterocyclylgruppen sind beispielsweise Piperidinyl, Piperazinyl, Morpholinyl, Thiomorpholinyl, Dihydropyranyl, Tetrahydropyranyl, Dioxanyl, Pyrrolinyl, Pyrrolidinyl, Imidazolinyl, Imidazolidinyl, Thiazolidinyl, Oxazolidinyl, Dioxolanyl, Dioxolyl, Pyrazolidinyl, Tetrahydrofuranyl, Dihydrofuranyl, Oxetanyl, Oxiranyl, Azetidinyl, Aziridinyl, Oxazetidinyl, Oxaziridinyl, Oxazepanyl, Oxazinanyl, Azepanyl, Oxopyrrolidinyl, Dioxopyrrolidinyl, Oxomorpholinyl, Oxopiperazinyl und Oxepanyl.

Eine besondere Bedeutung kommt Heteroarylen, also heteroaromatischen Systemen zu. Erfindungsgemäß steht der Ausdruck Heteroaryl für heteroaromatische Verbindungen, das heißt vollständig ungesättigte aromatische heterocyclische Verbindungen, die unter die vorstehende Definiton von Heterocyclen fallen. Vorzugsweise für 5- bis 7-gliedrige Ringe mit 1 bis 3, vorzugsweise 1 oder 2 gleichen oder verschiedenen Heteroatomen aus der oben genannten Gruppe. Erfindungsgemäße Heteroaryle sind beispielsweise Furyl, Thienyl, Pyrazolyl, Imidazolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Pyrrolyl, Pyridyl, Pyridazinyl, Pyrimidinyl, Pyrazinyl, 1,3,5-, 1,2,4- und 1,2,3-Triazinyl, 1,2,4-, 1,3,2-, 1,3,6- und 1,2,6-Oxazinyl, Oxepinyl, Thiepinyl, 1,2,4-Triazolonyl und 1,2,4-Diazepinyl. Die erfindungsgemäßen Heteroarylgruppen können ferner mit einem oder mehreren, gleichen oder verschiedenen Resten substituiert sein.

Substituierte Gruppen, wie ein substituierter Alkyl-, Alkenyl-, Alkinyl-, Cycloalkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy oder eine der Carboxygruppe äquivalente Gruppe, Cyano, Isocyano, Azido, Alkoxycarbonyl, Alkylcarbonyl, Formyl, Carbamoyl, Mono- und *N*,*N*-Dialkylamino-carbonyl, substituiertes Amino, wie Acylamino, Mono- und *N*,*N*-Dialkylamino, Trialkylsilyl und gegebenenfalls substituiertes Cycloalkyl, gegebenenfalls substituiertes Aryl, gegebenenfalls substituiertes Heterocyclyl, wobei jeder der letztgenannten cyclischen Gruppen auch über Heteroatome oder divalente funktionelle Gruppen wie bei den genannten Alkylresten gebunden sein kann, und Alkylsulfinyl, wobei beide Enantiomere der Alkylsulfonylgruppe umfasst sind, Alkylsulfonyl, Alkylphosphinyl, Alkylphosphonyl und, im Falle cyclischer Reste (= "cyclischer Grundkörper"), auch Alkyl, Haloalkyl, Alkylthio-alkyl, Alkoxy-alkyl, gegebenfalls substituiertes Mono- und *N*,*N*-Dialkyl-aminoalkyl und Hydroxyalkyl bedeutet.

Im Begriff "substituierte Gruppen" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Alkenylthio, Alkinylthio, Alkenyloxycarbonyl, Alkinyloxycarbonyl, Alkenylcarbonyl, Alkinylcarbonyl, Mono- und *N*,*N*-Dialkenylamino-carbonyl, Mono- und Dialkinylaminocarbonyl, Mono- und *N*,*N*-Dialkenylamino, Mono- und *N*,*N*-Dialkinylamino, Trialkenylsilyl, Trialkinylsilyl, gegebenenfalls substituiertes Cycloalkenyl, gegebenenfalls substituiertes Cycloalkinyl, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden oder einer Oxogruppe, Iminogruppe sowie einer substituierten Iminogruppe.

Wenn zwei oder mehrere Reste einen oder mehrere Ringe bilden, so können diese carbocyclisch, heterocyclisch, gesättigt, teilgesättigt, ungesättigt, beispielsweise auch aromatisch und weiter substituiert sein.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bevorzugte Substituenten für die Substituentenebenen sind beispielsweise Amino, Hydroxy, Halogen, Nitro, Cyano, Isocyano, Mercapto, Isothiocyanato, Carboxy, Carbonamid, SF₅, Aminosulfonyl, Alkyl, Cycloalkyl, Alkenyl, Cycloalkenyl, Alkinyl, *N*-Monoalkyl-amino, *N*,*N*-Dialkylamino, *N*-Alkanoylamino, Alkoxy, Alkenyloxy, Alkinyloxy, Cycloalkoxy, Cycloalkenyloxy, Alkoxycarbonyl, Alkenyloxycarbonyl, Alkinyloxycarbonyl, Aryloxycarbonyl, Alkanoyl, Alkenylcarbonyl, Alkinylcarbonyl, Arylcarbonyl, Alkylthio, Cycloalkylthio, Alkenylthio, Cycloalkenylthio, Alkinylthio, Alkylsulfenyl und Alkylsulfinyl, wobei beide Enantiomere der Alkylsulfinylgruppe umfasst sind, Alkylsulfonyl, *N*-Mono-alkylaminosulfonyl, *N*,*N*-Dialkyl-aminosulfonyl, Alkylphosphinyl, Alkylphosphonyl, wobei für Alkylphosphinyl bzw. Alkylphosphonyl beide Enantiomere umfasst sind, *N*-Alkyl-aminocarbonyl, *N*,*N*-Dialkyl-amino-carbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino, Benzylamino, Heterocyclyl und Trialkylsilyl.

Substituenten, die aus mehreren Substituentenebenen zusammengesetzt sind, sind bevorzugt Alkoxyalkyl, Alkylthioalkyl, Alkylthioalkoxy, Alkoxyalkoxy, Phenethyl, Benzyloxy, Halogenalkyl, Halogencycloalkyl, Halogenalkoxy, Halogenalkylthio, Halogenalkylsulfinyl, Halogenalkylsulfonyl, Halogenalkanoyl, Halogenalkylcarbonyl, Halogenalkoxycarbonyl, Halogenalkoxyalkoxy, Halogenalkoxyalkylthio, Halogenalkoxyalkanoyl, Halogenalkoxyalkyl.

Bei Resten mit C-Atomen sind solche mit 1 bis 6 C-Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Hydroxy, Amino, Alkoxy, Acyl und Aryl *N*-substituiert sind; vorzugsweise *N*-Mono- und *N*,*N*-Dialkylamino, (z.B. Methylamino, Ethylamino, *N*,*N-*Dimethylamino, *N*,*N*-Diethylamino, *N*,*N*-Di-n-propylamino, *N*,*N*-Diisopropylamino oder *N,N-*Dibutylamino), *N*-Mono- oder *N*,*N*-Dialkoxyalkylaminogruppen (z.B. *N*-Methoxymethylamino, *N-*Methoxyethylamino, *N*,*N*-Di-(methoxymethyl)-amino oder *N*,*N*-Di-(methoxyethyl)-amino), *N-*Mono- und *N*,*N*-Diarylamino, wie gegebenenfalls substituierte Aniline, Acylamino, *N,N-*diacylamino, *N*-Alkyl-*N*-arylamino, *N*-Alkyl-*N*-acylamino sowie gesättigte *N*-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Erfindungsgemäß umfasst der Begriff "cyclische Aminogruppen" heteroaromatische oder aliphatische Ringsysteme mit einem oder mehreren Stickstoffatomen. Die Heterocyclen sind gesättigt oder ungesättigt, bestehen aus einem oder mehreren, gegebenenfalls kondensierten Ringsystemen und beinhalten gegebenenfalls weitere Heteroatome, wie beispielsweise ein oder zwei Stickstoff-, Sauerstoff- und/oder Schwefelatome. Ferner umfasst der Begriff auch solche Gruppen, die einen Spiroring oder verbrücktes Ringsystem aufweisen. Die Anzahl der Atome, die die cyclische Aminogruppe bilden, ist beliebig und kann z.B. im Falle eines Einringsystems aus 3 bis 8 Ringatomen und im Falle eines Zweiringsystems aus 7 bis 11 Atomen.

Beispielhaft für cyclische Aminogruppen mit gesättigten und ungesättigten monocyclischen Gruppen mit einem Stickstoffatom als Heteroatom seien 1-Azetidinyl, Pyrrolidino, 2-Pyrrolidin-1-yl, 1-Pyrrolyl, Piperidino, 1,4-Dihydropyrazin-1-yl, 1,2,5,6-Tetrahydropyrazin-1-yl, 1,4-Dihydropyridin-1-yl, 1,2,5,6-Tetrahydropyridin-1-yl, Homopiperidinyl genannt; beispielhaft für cyclische Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-piperazin-1-yl, 1,2-Dihydro-pyrimidin-1-yl, Perhydropyrimidin-1-yl, 1,4-Diazacycloheptan-1-yl, genannt; beispielhaft für cyclische Aminogruppen mit gesättigten und ungesättigten monocyclischen Gruppen mit einem oder zwei Sauerstoffatomen und einem bis drei Stickstoffatomen als Heteroatome, wie beispielsweise Oxazolidin-3-yl, 2,3-Dihydroisoxazol-2-yl, Isoxazol-2-yl, 1,2,3-Oxadiazin-2-yl, Morpholino, beispielhaft für cyclische Aminogruppen mir gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino, oder Dioxothiomorpholino genannt; beispielhaft für cyclische Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclische Aminogruppen mit spirocyclischen Gruppen sei das 2-Azaspiro[4,5]decan-2-yl genannt; beispielhaft für cyclische Aminogruppen mit verbrückten heterocyclischen Gruppen sei das 2-Azabicyclo[2,2,1]heptan-7-yl genannt.

Substituiertes Amino schließt auch quartäre Ammoniumverbindungen (Salze) mit vier organischen Substituenten am Stickstoffatom ein.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, (C₁-C₄)Alkylthio, (C₁-C₄)Halogenalkylthio, Cyano, Isocyano und Nitro substituiert ist, z.B. o-, m- und p-Tolyl, Dimethylphenyle, 2-, 3- und 4-Chlorphenyl, 2-, 3- und 4-Fluorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Gegebenenfalls substituiertes Cycloalkyl ist vorzugsweise Cycloalkyl, das unsubstituiert oder ein-oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy , (C₁-C₄)Alkoxy-(C₁-C₄)alkyl, (C₁-C₄)Halogenalkyl und (C₁-C₄)Halogenalkoxy substituiert ist, insbesondere durch einen oder zwei (C₁-C₄)Alkylreste substituiert ist,

Gegebenenfalls substituiertes Heterocyclyl ist vorzugsweise Heterocyclyl, das unsubstituiert oder ein- oder mehrfach, vorzugsweise bis zu dreifach durch gleiche oder verschiedene Reste aus der Gruppe Halogen, Cyano, (C₁-C₄)Alkyl, (C₁-C₄)Alkoxy, (C₁-C₄)Alkoxy-(C₁-C₄)alkoxy, (C₁-C₄)Alkoxy-(C₁-C₄)alkyl, (C₁-C₄)Halogenalkyl, (C₁-C₄)Halogenalkoxy, Nitro und Oxo substituiert ist, insbesondere ein- oder mehrfach durch Reste aus der Gruppe Halogen, (C₁-C₄)Alkyl, (C₁-C₄)Alkoxy, (C₁-C₄)Halogenalkyl und Oxo, ganz besonders durch einen oder zwei (C₁-C₄)Alkylreste substituiert ist.

Beispiele für Alkyl substituierte Heteroaryle sind Furylmethyl, Thienylmethyl, Pyrazolylmethyl, Imidazolylmethyl, 1,2,3- und 1,2,4-Triazolylmethyl, Isoxazolylmethyl, Thiazolylmethyl, Isothiazolylmethyl, 1,2,3-, 1,3,4-, 1,2,4- und 1,2,5-Oxadiazolylmethyl, Azepinylmethyl, Pyrrolylmethyl, Pyridylmethyl" Pyridazinylmethyl, Pyrimidinylmethyl, Pyrazinylmethyl, 1,3,5-, 1,2,4- und 1,2,3-Triazinylmethyl, 1,2,4-, 1,3,2-, 1,3,6- und 1,2,6-Oxazinylmethyl, Oxepinylmethyl, Thiepinylmethyl und 1,2,4-Diazepinylmethyl.

Erfindungsgemäß geeignete Salze der erfindungsgemäßen Verbindungen, beispielsweise Salze mit Basen oder Säureadditionssalze, sind alle üblichen nicht toxischen Salze, vorzugsweise landwirtschaftlich und/oder physiologisch annehmbare Salze. Beispielsweise Salze mit Basen oder Säureadditionssalze. Bevorzugt werden Salze mit anorganischen Basen, wie beispielsweise Alkalimetallsalze (z.B. Natrium-, Kalium- oder Cäsiumsalze), Erdalkalimetallsalze (z.B. Calzium- oder Magnesiumsalze), Ammoniumsalze oder Salze mit organischen Basen, insbesondere mit organischen Aminen, wie beispielsweise Triethylammonium-, Dicyclohexylammonium-, *N,N'-*Dibenzylethylendiammonium-, Pyridinium-, Picolinium- oder Ethanolammoniumsalze, Salze mit anorganischen Säuren (z.B. Hydrochloride, Hydrobromide, Dihydrosulfate, Trihydrosulfate, oder Phosphate), Salze mit organischen Carbonsäuren oder organischen Sulfosäuren (z.b. Formiate, Acetate, Trifluoracetate, Maleate, Tartrate, Methansulfonate, Benzolsulfonate oder 4-Toluolsulfonate). Bekannterweise können tert- Amine, wie beispielsweise manche der erfindungsgemäßen Verbindungen, *N*-Oxide bilden, welche ebenfalls erfindungsgemäße Salze darstellen.

Ferner bevorzugte Ausführungsformen der Erfindung stellen die Verbindungen der allgemeinen Formeln (Ia) bis (Ij) dar.

In den nachfolgenden allgemeinen Formeln **(I**a) bis **(Ij)** haben die Gruppierungen und Substituenten U, L, m, Q, T, **R¹, R**² **R**³, **R**⁴ und **R**⁵ die oben angegebenen Bedeutungen.

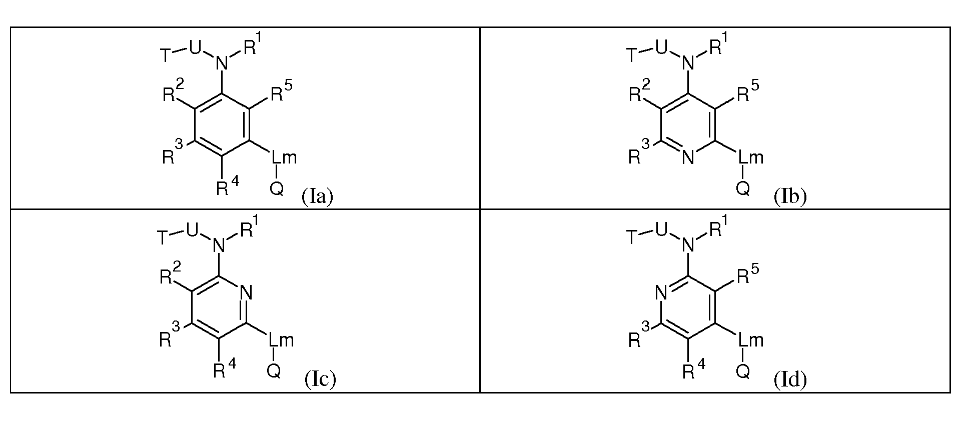

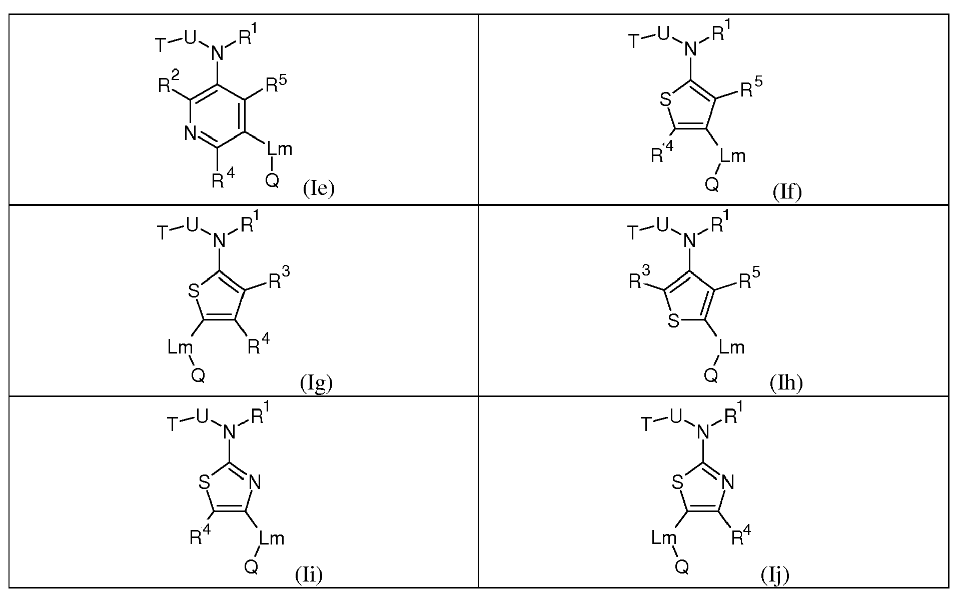

Insbesondere bevorzugt sind Verbindungen der allgemeinen Formel (I-a-a) 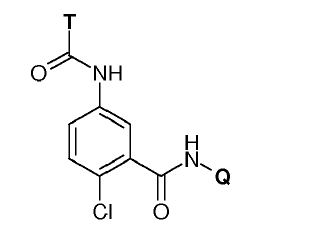 in denen
- T: für einen Rest ausgewählt aus Methyl-3-(pentafluorethyl)-4-(trifluormethyl)-1H-pyrazol-5- yl, 2-Methyl-6-(trifluormethyl)pyridin-3-yl, 2-(Trifluormethyl)-1,3-thiazol-4-yl, 4-Brom-1- methyl-3-(trifluormethyl)-1H-pyrazol-5-yl, 4-(Difluormethyl)-2-(pentafluorethyl)pyri- midin-5-yl, 4-Cyclopropyl-2-(pentafluorethyl)pyrimidin-5-yl, 2,4-Bis(pentafluor- ethyl)pyrimidin-5-yl, 3-{[3,5-Bis(pentailuorethyl)-1H-pyrazol-l-yl]methyl}-l-methyl-4- (trifluormethyl)-1H-pyrazol-5-yl steht,
- Q: für einen Rest ausgewählt aus Pyridin-4-yl, Pyridin-3-yl, 4-Fluorphenyl, 4-Chlorpyridin-3- yl, 2-Chlorpyridin-4-yl, 6-Chlorpyridin-3-yl, Pyridin-2-yl, 3-Chlorphenyl, 4- Methoxyphenyl, 2-Chlorpyridin-3-yl, Phenyl, 3-Methoxyphenyl, 6-Chlorpyridin-2-yl, 2,4- Difluorphenyl, 4-Fluor-2-methylphenyl, 4-Chlorpyridin-2-yl, 4-Fluor-3-methylphenyl, 3- Chlor-4-fluor-2-methylphenyl, 5-Chlorpyridin-2-yl, 4-Fluor-3-(trifluormethyl)phenyl, 3,4- Dichlorphenyl, 3,4,5-Trichlorphenyl Bezüglich der Definitionen im Text

Die erfindungsgemäßen Verbindungen können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die erfindungsgemäßen Verbindungen können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Schließlich wurde gefunden, dass die neuen Verbindungen der Formel (I) sich bei guter Pflanzenverträglichkeit, günstiger Warmblütertoxizität und guter Umweltverträglichkeit vor allem zur Bekämpfung von tierischen Schädlingen, insbesondere von Arthropoden, Insekten, Spinnentieren, Helminthen, Nematoden und Mollusken, die in der Landwirtschaft, in den Forsten, im Vorrats- und Materialschutz sowie auf dem Hygienesektor oder im Bereich der Tiergesundheit vorkommen, eignen. Gleichfalls können die erfindungsgemäßen Verbindungen im Bereich der Tiergesundheit verwendet werden, beispielsweise zur Bekämpfung von Endo- und/oder Ectoparasiten.

Die erfindungsgemäßen Verbindungen können als Mittel zur Bekämpfung tierischer Schädlinge, vorzugsweise als Pflanzenschutzmittel eingesetzt werden. Sie sind gegen normal sensible und resistente Arten sowie gegen alle oder einzelne Entwicklungsstadien wirksam.

Die erfindungsgemäßen Verbindungen können in allgemein bekannte Formulierungen überführt werden. Solche Formulierungen enthalten im Allgemeinen von 0,01 bis 98 Gew.-% Wirkstoff, vorzugsweise von 0,5 bis 90 Gew.-%.

Die erfindungsgemäßen Verbindungen können in ihren handelsüblichen Formulierungen sowie in den aus diesen Formulierungen bereiteten Anwendungsformen in Mischung mit anderen Wirkstoffen oder Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise von 0,00001 bis 1 Gew.-% liegen.

### Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Coccidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Außerdem wurde gefunden, daß die erfindungsgemäßen Verbindungen eine hohe insektizide Wirkung gegen Insekten zeigen, die technische Materialien zerstören. Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam.

Unter Pflanzen werden alle Pflanzenarten, Pflanzensorten und Pflanzenpopulationen, wie erwünschte und unerwünschte Wildpflanzen oder Kulturpflanzen verstanden. Erfindungsgemäß zu behandelnde Kulturpflanzen sind Pflanzen, die natürlich vorkommen, oder solche, die durch konventionelle Züchtungs- und Optimierungsmethoden oder durch biotechnologische und gentechnologische Methoden oder durch Kombinationen der vorgenannten Methoden erhalten wurden. Der Begriff Kulturpflanze umfasst selbstverständlich auch transgene Pflanzen.

Unter Pflanzensorten versteht man Pflanzen mit neuen Eigenschaften, sogenannten Traits, die sowohl durch konventionelle Züchtung, durch Mutagenese oder durch rekombinante DNA-Techniken oder einer Kombination hieraus gezüchtet worden sind. Dies können Sorten, Rassen, Bio- und Genotypen sein.

Unter Pflanzenteilen werden alle oberirdischen und unterirdischen Teile und Organe der Pflanzen, wie Spross, Blatt, Blüte und Wurzel verstanden, insbesondere Blätter, Nadeln, Stängel, Stämme, Blüten, Fruchtkörper, Früchte, Samen, Wurzeln, Knollen und Rhizome. Der Begriff Pflanzenteilen umfasst weiterhin Erntegut sowie vegetatives und generatives Vermehrungsmaterial, wie z.B. Stecklinge, Knollen, Rhizome, Ableger und Samen bzw. Saatgut.

In einer erfindungsgemäßen Ausführungsform werden natürlich vorkommende oder durch konventionelle Züchtungs- und Optimierungsmethoden (z.B. Kreuzung oder Protoplastenfusion) erhaltene Pflanzenarten und Pflanzensorten sowie deren Pflanzenteile behandelt.

In einer weiteren erfindungsgemäßen Ausführungsform werden transgene Pflanzen, die durch gentechnologische Methoden gegebenenfalls in Kombination mit konventionellen Methoden erhalten wurden und deren Teile behandelt.

Das erfindungsgemäße Behandlungsverfahren wird vorzugsweise auf genetisch modifizierten Organismen, wie beispielsweise Pflanzen oder Pflanzenteile, verwendet.

Genetisch modifizierte Pflanzen, sogenannte transgene Pflanzen, sind Pflanzen, bei denen ein heterologes Gen stabil in das Genom integriert worden ist.

Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im allgemeinen von 1 bis 10 Tagen, vorzugsweise 1 bis 7 Tagen, nach der Behandlung der Pflanzen mit den Wirkstoffen.

Pflanzen, die weiterhin vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Neben den vorgenannten Pflanzen und Pflanzensorten, können auch solche erfindungsgemäß behandelt werden, die gegen einen oder mehrere abiotische Streßfaktoren widerstandsfähig sind.

Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen. Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_CrickmoreBt/ beschrieben sind, zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insecticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotid-adenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produziere;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfojrc.it/gmp_browse.aspx und http://www.agbios.com/dbase.php).

Die erfindungsgemäße Behandlung der Pflanzen und Pflanzenteile mit den Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Insbesondere eignen sich die erfindungsgemäßen Mischungen zur Behandlung von Saatgut. Bevorzugt sind dabei die vorstehend als bevorzugt oder besonders bevorzugt genannten erfindungsgemäßen Kombinationen zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt wird, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ein weiterer Vorteil besteht in der synergistischen Erhöhung der insektiziden Wirksamkeit der erfindungsgemäßen Mittel gegenüber dem insektiziden Einzelwirkstoff, die über die zu erwartende Wirksamkeit der beiden einzeln angewendeten Wirkstoffe hinausgeht. Vorteilhaft ist auch die synergistische Erhöhung der fungiziden Wirksamkeit der erfindungsgemäßen Mittel gegenüber dem fungiziden Einzelwirkstoff, die über die zu erwartende Wirksamkeit des einzeln angewendeten Wirkstoffs hinausgeht. Damit wird eine Optimierung der Menge der eingesetzten Wirkstoffe ermöglicht.

Ebenso ist es als vorteilhaft anzusehen, dass die erfindungsgemäßen Mischungen insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Mittel eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie *Bacillus, Rhizobium, Pseudomonas, Serratia, Trichoderma, Clavibacter, Glomus* oder *Gliocladium* stammen. Die vorliegende Erfindung eignet sich besonders für die Behandlung von transgenem Saatgut, das zumindest ein heterologes Gen enthält, das aus *Bacillus sp.* stammt und dessen Genprodukt Wirksamkeit gegen Maiszünsler und/oder Maiswurzel-Bohrer zeigt. Besonders bevorzugt handelt es sich dabei um ein heterologes Gen, das aus *Bacillus thuringiensis* stammt.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Darüber hinaus können die erfindungsgemäßen Verbindungen zur Bekämpfung einer Vielzahl verschiedener Schädlinge einschließlich beispielsweise schädlicher saugender Insekten, beißender Insekten und anderen an Pflanzen parasitierenden Schädlingen, Vorratsschädlingen, Schädlingen, die industrielle Materialien zerstören und Hygieneschädlingen einschließlich Parasiten im Bereich Tiergesundheit verwendet und zu ihrer Bekämpfung wie zum Beispiel ihrer Auslöschung und Ausmerzung eingesetzt werden. Die vorliegende Erfindung schließt somit auch ein Verfahren zur Bekämpfung von Schädlingen ein.

Im Bereich Tiergesundheit, d.h. auf dem veterinärmedizinischen Gebiet, wirken die Wirkstoffe gemäß der vorliegenden Erfindung gegen tierische Parasiten, insbesondere Ektoparasiten oder Endoparasiten. Der Begriff Endoparasiten schließt insbesondere Helminthen wie Cestoden, Nematoden oder Trematoden, und Protozoen wie Kozzidien ein. Ektoparasiten sind typischerweise und vorzugsweise Arthropoden, insbesondere Insekten wie Fliegen (stechend und leckend), parasitische Fliegenlarven, Läuse, Haarlinge, Federlinge, Flöhe und dergleichen; oder Akariden wie Zecken, zum Beispiel Schildzecken oder Lederzecken, oder Milben wie Räudemilben, Laufmilben, Federmilben und dergleichen.

Zu diesen Parasiten gehören:
Aus der Ordnung der Anoplurida z.B. Haematopinus spp., Linognathus spp., Pediculus spp., Phtirus spp., Solenopotes spp.; spezielle Beispiele sind: Linognathus setosus, Linognathus vituli, Linognathus ovillus, Linognathus oviformis, Linognathus pedalis, Linognathus stenopsis, Haematopinus asini macrocephalus, Haematopinus eurysternus, Haematopinus suis, Pediculus humanus capitis, Pediculus humanus corporis, Phylloera vastatrix, Phthirus pubis, Solenopotes capillatus;
Aus der Ordnung der Mallophagida und den Unterordnungen Amblycerina und Ischnocerina z.B. Trimenopon spp., Menopon spp., Trinoton spp., Bovicola spp., Werneckiella spp., Lepikentron spp., Damalina spp., Trichodectes spp., Felicola spp.; spezielle Beispiele sind: Bovicola bovis, Bovicola ovis, Bovicola limbata, Damalina bovis, Trichodectes canis, Felicola subrostratus, Bovicola caprae, Lepikentron ovis, Werneckiella equi;
Aus der Ordnung der Diptera und den Unterordnungen Nematocerina und Brachycerina z.B. Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; spezielle Beispiele sind: Aedes aegypti, Aedes albopictus, Aedes taeniorhynchus, Anopheles gambiae, Anopheles maculipennis, Calliphora erythrocephala, Chrysozona pluvialis, Culex quinquefasciatus, Culex pipiens, Culex tarsalis, Fannia canicularis, Sarcophaga carnaria, Stomoxys calcitrans, Tipula paludosa, Lucilia cuprina, Lucilia sericata, Simulium reptans, Phlebotomus papatasi, Phlebotomus longipalpis, Odagmia ornata, Wilhelmia equina, Boophthora erythrocephala, Tabanus bromius, Tabanus spodopterus, Tabanus atratus, Tabanus sudeticus, Hybomitra ciurea, Chrysops caecutiens, Chrysops relictus, Haematopota pluvialis, Haematopota italica, Musca autumnalis, Musca domestica, Haematobia irritans irritans, Haematobia irritans exigua, Haematobia stimulans, Hydrotaea irritans, Hydrotaea albipuncta, Chrysomya chloropyga, Chrysomya bezziana, Oestrus ovis, Hypoderma bovis, Hypoderma lineatum, Przhevalskiana silenus, Dermatobia hominis, Melophagus ovinus, Lipoptena capreoli, Lipoptena cervi, Hippobosca variegata, Hippobosca equina, Gasterophilus intestinalis, Gasterophilus haemorroidalis, Gasterophilus inermis, Gasterophilus nasalis, Gasterophilus nigricornis, Gasterophilus pecorum, Braula coeca;
Aus der Ordnung der Siphonapterida z.B. Pulex spp., Ctenocephalides spp., Tunga spp., Xenopsylla spp., Ceratophyllus spp.; spezielle Beispiele sind: Ctenocephalides canis, Ctenocephalides felis, Pulex irritans, Tunga penetrans, Xenopsylla cheopis;
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp. (z.B. Suppella longipalpa);
Aus der Unterklasse der Acari (Acarina) und den Ordnungen der Meta- und Mesostigmata z.B. Argas spp., Ornithodorus spp., Otobius spp., Ixodes spp., Amblyomma spp., Rhipicephalus (Boophilus) spp., Dermacentor spp., Haemophysalis spp., Hyalomma spp., Dermanyssus spp., Rhipicephalus spp. (der ursprünglichen Gattung der Mehrwirtszecken), Ornithonyssus spp., Pneumonyssus spp., Raillietia spp., Pneumonyssus spp., Sternostoma spp., Varroa spp., Acarapis spp.; spezielle Beispiele sind: Argas persicus, Argas reflexus, Ornithodorus moubata, Otobius megnini, Rhipicephalus (Boophilus) microplus, Rhipicephalus (Boophilus) decoloratus, Rhipicephalus (Boophilus) annulatus, Rhipicephalus (Boophilus) calceratus, Hyalomma anatolicum, Hyalomma aegypticum, Hyalomma marginatum, Hyalomma transiens, Rhipicephalus evertsi, Ixodes ricinus, Ixodes hexagonus, Ixodes canisuga, Ixodes pilosus, Ixodes rubicundus, Ixodes scapularis, Ixodes holocyclus, Haemaphysalis concinna, Haemaphysalis punctata, Haemaphysalis cinnabarina, Haemaphysalis otophila, Haemaphysalis leachi, Haemaphysalis longicorni, Dermacentor marginatus, Dermacentor reticulatus, Dermacentor pictus, Dermacentor albipictus, Dermacentor andersoni, Dermacentor variabilis, Hyalomma mauritanicum, Rhipicephalus sanguineus, Rhipicephalus bursa, Rhipicephalus appendiculatus, Rhipicephalus capensis, Rhipicephalus turanicus, Rhipicephalus zambeziensis, Amblyomma americanum, Amblyomma variegatum, Amblyomma maculatum, Amblyomma hebraeum, Amblyomma cajennense, Dermanyssus gallinae, Ornithonyssus bursa, Ornithonyssus sylviarum, Varroa jacobsoni;
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.; spezielle Beispiele sind: Cheyletiella yasguri, Cheyletiella blakei, Demodex canis, Demodex bovis, Demodex ovis, Demodex caprae, Demodex equi, Demodex caballi, Demodex suis, Neotrombicula autumnalis, Neotrombicula desaleri, Neoschöngastia xerothermobia, Trombicula akamushi, Otodectes cynotis, Notoedres cati, Sarcoptis canis, Sarcoptes bovis, Sarcoptes ovis, Sarcoptes rupicaprae (=S. caprae), Sarcoptes equi, Sarcoptes suis, Psoroptes ovis, Psoroptes cuniculi, Psoroptes equi, Chorioptes bovis, Psoergates ovis, Pneumonyssoidic mange, Pneumonyssoides caninum, Acarapis woodi.

Die erfindungsgemäßen Wirkstoffe eignen sich auch zur Bekämpfung von Arthropoden, Helminthen und Protozoen, die Tiere befallen. Zu den Tieren zählen landwirtschaftliche Nutztiere wie z.B. Rinder, Schafe, Ziegen, Pferde, Schweine, Esel, Kamele, Büffel, Kaninchen, Hühner, Puten, Enten, Gänse, Zuchtfische, Honigbienen. Zu den Tieren zählen außerdem Haustiere - die auch als Heimtiere bezeichnet werden - wie z.B. Hunde, Katzen, Stubenvögel, Aquarienfische sowie sogenannte Versuchstiere wie z.B. Hamster, Meerschweinchen, Ratten und Mäuse.

Durch die Bekämpfung dieser Arthropoden, Helminthen und/oder Protozoen sollen Todesfälle vermindert und die Leistung (bei Fleisch, Milch, Wolle, Häuten, Eiern, Honig usw.) und die Gesundheit des Wirtstieres verbessert werden, so dass durch den Einsatz der erfindungsgemäßen Wirkstoffe eine wirtschaftlichere und einfachere Tierhaltung möglich ist.

So ist es beispielsweise wünschenswert, die Aufnahme von Blut des Wirts durch die Parasiten (falls zutreffend) zu verhindern oder zu unterbrechen. Eine Bekämpfung der Parasiten kann außerdem dazu beitragen, die Übertragung infektiöser Substanzen zu verhindern.

Der Begriff "Bekämpfung", so wie er hier bezogen auf den Bereich Tiergesundheit verwendet wird, bedeutet, dass die Wirkstoffe wirken, indem sie das Vorkommen des betreffenden Parasiten in einem mit solchen Parasiten befallenen Tier auf unschädliche Niveaus reduzieren. Genauer gesagt bedeutet "Bekämpfung", wie hier verwendet, dass der Wirkstoff den betreffenden Parasiten tötet, sein Wachstum hemmt oder seine Proliferation inhibiert.

Im allgemeinen können die erfindungsgemäßen Wirkstoffe, wenn sie für die Behandlung von Tieren eingesetzt werden, direkt angewendet werden. Vorzugsweise werden sie als pharmazeutische Zusammensetzungen angewendet, die im Stand der Technik bekannte pharmazeutisch unbedenkliche Exzipienten und/oder Hilfsstoffe enthalten können.

Die Anwendung (= Verabreichung) der Wirkstoffe im Bereich Tiergesundheit und in der Tierhaltung erfolgt in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subkutan, intravenös, intraperitoneal u.a.), Implantate, durch nasale Applikation, durch dermale Applikation in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw. Die Wirkstoffe können als Shampoo oder als geeignete, in Aerosolen oder drucklosen Sprays, z.B. Pumpsprays und Zerstäubersprays, anwendbare, Formulierungen formuliert werden.

Bei der Anwendung für Nutztiere, Geflügel, Haustiere etc. kann man die erfindungsgemäßen Wirkstoffe als Formulierungen (beispielsweise Pulver, Spritzpulver [wettable powders, "WP"], Emulsionen, Emulsionskonzentrate [emulsifiable concentrates ,"EC"], fließfähige Mittel, homogene Lösungen und Suspensionskonzentrate [suspension concentrates, "SC"]), die die Wirkstoffe in einer Menge von 1 bis 80 Gew.-% enthalten, direkt oder nach Verdünnung (z.B. 100-bis 10 000facher Verdünnung) anwenden oder sie als chemisches Bad verwenden.

Beim Einsatz im Bereich Tiergesundheit können die erfindungsgemäßen Wirkstoffe in Kombination mit geeigneten Synergisten oder anderen Wirkstoffen wie beispielsweise Akariziden, Insektiziden, Anthelmintika, Mittel gegen Protozoen, verwendet werden.

Die erfindungsgemäßen Verbindungen können nach üblichen, dem Fachmann bekannten Methoden hergestellt werden.

Die erfindungsgemäßen Verbindungen können nach üblichen, dem Fachmann bekannten Methoden hergestellt werden.

Im Reaktionsschema 1 ist das allgemeine Darstellungsverfahren A für die erfindungsgemäßen Verbindungen der allgemeinen Formel **(I-1)** abgebildet.

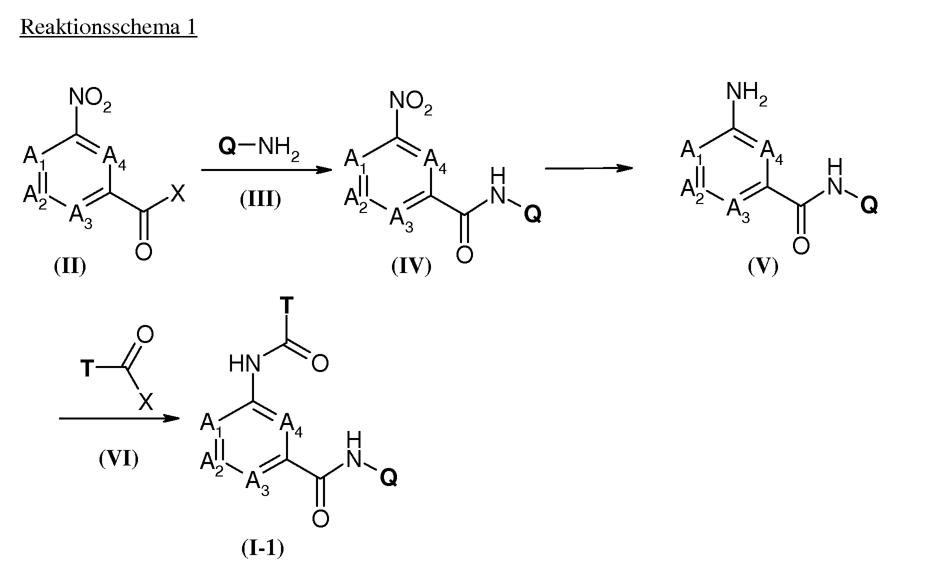

In den allgemeinen Formeln der Verbindungen **(I-1), (II), (III), (IV), (V)** und **(VI)** im Reaktionsschema 1 haben die chemischen Gruppierungen A₁, A₂, A₃ und A₄ sowie die Reste Q und T die weiter oben beschriebenen Bedeutungen. In den allgemeinen Formeln **(II)** und **(VI)** steht X für eine geeignete Abgangsgruppe.

Erfindungsgemäße Verbindungen des Typs **(I-1)** können durch die Umsetzung von (hetero)aromatischen Aminoverbindungen der allgemeinen Formel (**V**) mit aktivierten Carbonsäurederivaten der allgemeinen Formel **(VI)** dargestellt werden. Die Reaktion kann gegebenenfalls in Gegenwart eines Verdünnungsmittels durchgeführt werden. In diesem Reaktionsschritt kann gegebenenfalls ein geeignetes basisches Reaktionshilfsmittel zum Einsatz kommen.

Mischungungen der Verdünnungs- bzw. Lösungsmittel sind ebenfalls einsetzbar. Die Verdünnungs- bzw. Lösungsmittel werden vorteilhafterweise in einer solchen Menge eingesetzt, dass das Reaktionsgemisch während des ganzen Verfahrens gut rührbar ist.

Als Verdünnungs- bzw. Lösungsmittel zur Durchführung der erfindungsgemäßen Verfahren kommen grundsätzlich alle unter den spezifischen Reaktionsbedingungen inerten organischen Lösungsmittel in Frage. Als Beispiele sind zu nennen: Halogenkohlenwasserstoffe (z.B. Chlorkohlenwasserstoffe, wie Tetraethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol), Alkohole (z.B. Methanol, Ethanol, Isopropanol, Butanol), Ether (z.B. Ethylpropylether, Methyl-tert-butylether, n-Butylether, Anisol, Phenetol, Cyclohexylmethylether, Dimethylether, Diethylether, Diproplether, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids), Amine (z.B. Trimethyl-, Triethyl-, Triprcpyl-, Tributylamin, N-Methylmorpholin, Pyridin und Tetramethylendiamin), Nitrokohlenwasserstoffe (z.B. Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlorbenzonitril), Tetrahydrothiophendioxid, Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Sulfone (z.B. Dimethyl-, Diethyl-, Dipropyl-, Dibutyl-, Diphenyl-, Dihexyl-, Methylethyl-, Ethylpropyl-, Ethylisobutyl- und Pentamethylensulfon), aliphatische, cycloaliphatische oder aromatische Kohlenwasserstoffe (z.B. Pentan, Hexan, Heptan, Oktan, Nonan und technische Kohlenwasserstoffe), ferner sogenannte "White Spirits" mit Komponenten mit Siedepunkten im Bereich von beispielsweise 40°C bis 250°C, Cymol, Benzinfraktionen innerhalb eines Siedeinterwalles von 70°C bis 190°C, Cyclohexan, Methylcyclohexan, Petrolether, Ligroin, Octan, Benzol, Toluol, Chlorbenzol, Brombenzol, Nitrobenzol, Xylol, Ester (z.B. Methyl-, Ethyl-, Butyl-, Isobutylacetat, Dimethyl-, Dibutyl-, Ethylencarbonat); Amide (z.B. Hexamethylenphosphorsäuretriamid, Formamid, N-Methyl-formamid, N,N-Dimethyl-formamid, N,N-Dipropyl-formamid, N,N-Dibutyl-formamid, N-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformyl-piperazin) und Ketone (z.B. Aceton, Acetophenon, Methylethylketon, Methylbutylketon).

Als bevorzugte Verdünnungsmittel kann jedes Lösungsmittel verwendet werden, das die Reaktion nicht beeinträchtigt, wie zum Beispiel Wasser. In Frage kommen aromatische Kohlenwasserstoffe wie Benzol oder Toluol; halogenierte Kohlenwasserstoffe wie Dichlormethan, Chloroform oder Tetrachlorkohlenwasserstoff, offenkettige oder zyklische Ether wie Diethylether, Dioxan, Tetrahydrofuran oder 1,2-Dimethoxyethan; Ester wie Ethylacetat und Butylacetat; Ketone wie zum Beispiel Aceton, Methyl-isobutylketon und Cyclohexanon; Amide wie Dimethylformamid und Dimethylacetamid; Nitrile wie Acetonitril; und andere inerte Lösungsmittel wie 1,3-Dimethyl-2-imidazolidinon; die Lösungsmittel können allein oder in Kombination von 2 oder mehr eingesetzt werden.

Als basische Reaktionshilfsmittel zur Durchführung des erfindungsgemäßen Verfahrens können alle geeigneten Säurebindemittel eingesetzt werden. Als Beispiele sind zu nennen Erdalkali- oder Alkalimetallverbindungen (z.B. Hydroxide, Hydride, Oxide und Carbonate des Lithiums, Natriums, Kaliums, Magnesiums, Calciums und Bariums), Amidinbasen oder Guanidinbasen (z.B. 7-Methyl-1,5,7-triaza-bicyclo(4.4.0)dec-5-en (MTBD); Diazabicyclo(4.3.0)nonen (DBN), Diazabicyclo(2.2.2)octan (DABCO), 1,8-Diazabicyclo(5.4.0)undecen (DBU), Cyclohexyltetrabutyl-guanidin (CyTBG), Cyclohexyltetramethylguanidin (CyTMG), N,N,N,N-Tetramethyl-1,8-naphthalindiamin, Pentamethylpiperidin) und Amine, insbesondere tertiäre Amine, (z.B. Triethylamin, Trimethylamin, Tribenzylamin, Triisopropylamin, Tributylamin, Tricyclohexylamin, Triamylamin, Trihexylamin, N,N-Dimethylanilin, N,N-Dimethyl-toluidin, N,N-Dimethyl-p-aminopyridin, N-Methyl-pyrrolidin, N-Methyl-piperidin, N-Methyl-imidazol, N-Methyl-pyrazol, N-Methyl-morpholin, N-Methyl-hexamethylendiamin, Pyridin, 4-Pyrrolidinopyridin, 4-Dimethylamino-pyridin, Chinolin, α-Picolin, β-Picolin, Isochinolin, Pyrimidin, Acridin, N,N,N',N'-Tetramethylendiamin, N,N,N',N'-Tetraethylendiamin, Chinoxalin, N-Propyl-diisopropylamin, N-Ethyl-diisopropylamin, N,N'-Dimethyl-cyclohexylamin, 2,6-Lutidin, 2,4-Lutidin oder Triethyldiamin).

Als bevorzugtes basisches Reaktionshilfsmittel kann eine organische Base wie Triethylamin, Ethyl-diisopropylamin, Tri-n-butylamin, Pyridin und 4-Dimethylamino-pyridin verwendet werden; Des Weiteren können beispielsweise folgende Basen eingesetzt werden: Alkalimetallhydroxide wie z.B Natriumhydroxid und Kaliumhydroxid; Carbonate wie Natriumhydrogencarbonat und Kaliumcarbonat; Phosphate wie Dikalium-hydrogenphosphat und Tri-natriumphosphat; Alkalimetallhydride wie Natriumhydrid; Alkalimetallalkoholate wie Natriummethanolat und Natriumethanolat. Diese basischen Reaktionshilfmittel können in Verhältnissen von 0.01 bis 5.0 Moläquivalenten in Bezug auf die Verbindungen der Formel **(V)** und **(VI)** eingesetzt werden.

Die geeignete Reaktionstemperatur liegt im Bereich von -20°C bis zum Siedepunkt des jeweiligen Lösungsmittels und die Reaktionsdauer liegt je nach Wahl der Reaktanden, Lösungsmittel und Reaktionstemperatur zwischen wenigen Minuten bis 96 Stunden.

Carbonsäurehalogenide (X = Halogen), wie sie durch die allgemeine Formel **(VI)** repräsentiert werden, können in üblicher Art und Weise durch die Umsetzung einer Carbonsäure mit geeigneten Halogenierungsreagenzien erhalten werden. Beispielsweise können anorganische Säurehalogenide wie Thionylchlorid, Thionylbromid, Oxalylchlorid, Phosphortri- oder Phosphorpentahalogenide für die Halogenierungsreaktion verwendet werden, wobei die Chloride bevorzugt sind [Houben-Weyl, Methoden der organischen Chemie, Bd. VIII, 4. Auflage, 1952, G. Thieme Verlag Stuttgart-New York, S. 359, 463 ff.].

Die Darstellung von Carboxamiden repräsentiert durch die Formel **(I-1)** kann aber auch unter der Verwendung von Kupplungsreagenzien durchgeführt werden. Als Kupplungsagenzien finden alle, die zur Herstellung einer Ester- oder Amidbindung geeignet sind (vgl. z. B. Houben-Weyl, Methoden der Organischen Chemie, Band 15/2; Bodansky et al., Peptide Synthesis 2nd ed. (Wiley & Sons, New York 1976) oder Gross, Meienhofer, The Peptides: Analysis, Synthesis, Biology (Academic Press, New York 1979), Verwendung. Vorzugsweise werden folgende Methoden herangezogen: Aktivestermethode mit Pentachlor (Pcp) und Pentafluorphenol (Pfp), N-Hydroxysuccinimid (HOSu), N-Hydroxy5-norbornen-2,3-dicarboxamid (HONB), 1-Hydroxybenzotriazol (HOBt) oder 3-Hydroxy-4-oxo-3,4-dihydro-1,2,3-benzotriazin als Alkoholkomponente, Kupplung mit Carbodiimiden wie N-(3-Dimethylaminopropyl)-N'-ethylcarbodiimid oder Dicyclohexylcarbodiimid (DCCI), nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutylchlorformiat) (IIDQ) oder Kupplung mit Phosphoniumreagenzien, wie Benzotriazol-1-yl-oxy-tris(dimethylaminophosphonium)hexafluoro-phosphat (BOP), Bis(2-oxo-3-oxazolidinyl)-phosphonium-säurechlorid (BOP-Cl), Benzotriazol-1-yl-trispyrrolidinophosphonium-hexafluorophosphat (PyBOP^{®}) Bromo-tris-pyrrolidino-phosphoniumhexafluorophosphat (PyBroP^{®}) oder mit Phosphonsäurereagenzien, wie Cyanphosphon-säurediethylester (DEPC) und Diphenylphosphorylazid (DPPA), Uronium-reagenzien, wie 2-(1H-Benzotriazol-1-yl)-1,1,3,3-tetramethyluronium-tetrafluoroborat (TNTU), 2-(2-Oxo-1(2H)-pyridyl)-1,1,3,3-bis-pentamethylen-tetramethyluronium-tetrafluoroborat (TOPPipU), O-(N-Succinimidyl-1,1,3,3-tetramethyluronium-tetrafluoroborat (TSTU) oder wie 2-(1H-Benzo-triazol-1-yl)-1,1,3,3-tetramethyluronium-hexafluorophosphat (HBTU) oder Reagenzien vom Onium Typ, wie beispielsweise 1-Ethyl-2-fluoropyridiniumtetrafluoroborat (FEP) oder Reagenzien vom Hetarylcarbonyl Typ wie beispielsweise 1,1'-Carbonyl-1H-imidazol.

Verbindungen der allgemeinen Formel (V) können durch eine Reduktionsreaktion aus Verbindungen der allgemeinen Formel (IV) hergestellt werden. Geeignete Verfahren für solche Reduktionen sind Hydrierungen und Metall-vermittelte Reaktionen wie z.B. durch Zinn(II)chlorid, Eisenpulver, Zinkpulver und diesen ähnliche Verbindungen.

Hydrierungen können in einem geeigneten Lösungsmittel in Anwesenheit eines Katalysators unter Wasserstoffatmosphäre (Normaldruck oder Hochdruck). Als geeignete Katalysatoren zur Durchführung der katalytischen Hydrierung kommen alle üblichen Hydrierkatalysatoren, wie beispielsweise Platin-Katalysatoren (Platin-Platte, Platin-Schwamm, Platin-Schwarz, kolloidales Platin, Platinoxid, Platindraht usw.), Palladium-Katalysatoren (beispielsweise Palladium-Schwamm, Palladium-Schwarz, Palladiumoxid, Palladium-Kohle, kolloidales Palladium, Palladium-Bariumsulfat, Palladium-Bariumcarbonat, Palladium-Hydroxid usw.), NickelKatalysatoren beispielsweise reduziertes Nickel, Nickeloxid, Raney-Nickel usw.), Ruthenium-Katalysatoren, Cobalt-Katalysatoren (beispielsweise reduziertes Cobalt, Raney-Cobalt usw.), Kupfer-Katalysatoren (beispielsweise reduziertes Kupfer, Raney-Kupfer, Ullmann-Kupfer usw.) in Frage. Vorzugsweise verwendet man jedoch Edelmetallkatalysatoren, wie beispielsweise Platin- und Palladium- oder Ruthenium-Katalysatoren gegebenenfalls auf einem geeigneten Träger wie beispielsweise auf Kohlenstoff oder Silizium, Rhodium-Katalysatoren, wie beispielsweise Tris(triphenylphosphin)rhodium(I)-chlorid in Gegenwart von Triphenylphosphin.

Selbstverständlich kann durch Verwendung eines "chiralen Hydrierkatalysators" z. B. mit chiralen Diphosphinliganden, beispielsweise *(2S,3S)-(-)-*2,3-Bis(diphenylphosphino)-butan [(S,S)-Chiraphos] (N. K. Roberts in "Catalytic Aspects of Metal Phosphine Complexes", ACS Washington, S. 337 (1982)) oder (*R*)-(+)-2,2'- bzw. (*S*)-(-)-2,2'-Bis(diphenylphosphino)-1,1'-binaphthalin [*R(*+*)*-BINAP bzw. *S(-)*-BINAP] (vgl. A. Miyashita et al. Tetrahedron 40, 1245 (1984)) auch der Anteil eines Isomers im Isomerengemisch deutlich erhöht werden bzw. lässt sich das Entstehen eines weiteren Isomers sogar vollständig unterdrücken.

Bevorzugt verwendet man Palladiumkatalysatoren wie z.B. Palladium auf Kohle, Nickelkatalysatoren wie Raney-Nickel, Kobaltkatalysatoren, Rutheniumkatalysatoren, Rhodiumkatalysatoren, Platinkatalysatoren und diesen ähnliche Verbindungen.

Geeignete Lösungsmittel sind Wasser, Alkohole wie Methanol und Ethanol, aromatische Kohlenwasserstoffe wie Benzol und Toluol, offenkettige oder zyklische Ether wie Diethylether, Dioxan und Tetrahydrofuran sowie Ester wie Essigsäureethylester. Die Reduktionen können in einem Druckbereich von 1 bar bis 100 bar durchgeführt werden, wobei die Temperatur zwischen - 20°C und dem Siedepunkt des eingesetzten Lösungsmittels variieren kann. Je nach Reaktionsbedingungen liegen die Reaktionszeiten zwischen wenigen Minuten und 96 Stunden.

Die Metall-vermittelten Reduktionen wie z.B. mit Zinn(II)chlorid können nach einem in Organic Syntheses Coll. Vol. (**III**), 453 beschriebenen Verfahren durchgeführt werden.

Verbindungen der allgemeinen Formel **(IV)** können durch die Umsetzung eines Amins der allgemeinen Formel **(III)** mit aktivierten Carbonsäurederivaten der allgemeinen Formel **(II)** dargestellt werden. Dabei gelten dieselben Bedingungen für die Wahl des Lösungsmittels, der Reaktionsbedingungen, der Reaktionsdauer und der Reagenzien wie bei der oben beschriebenen Darstellung von Verbindungen der allgemeinen Formel **(I-1).**

Das Reaktionsschema 2 zeigt das allgemeine Darstellungsverfahren B für die Synthese der erfindungsgemäßen Verbindungen der allgemeinen Formel **(I-1).**

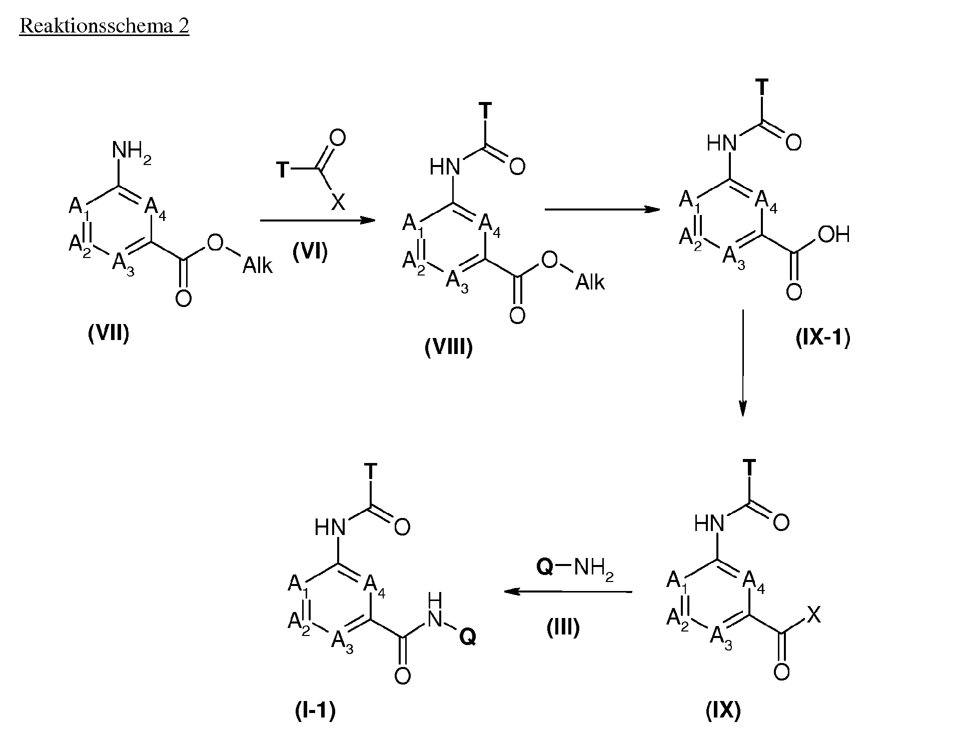

In den allgemeinen Formeln der Verbindungen **(I-1), (III), (VI), (VII), (VIII), (IX-1)** und **(IX)** im Reaktionsschema 2 haben die chemischen Gruppierungen A₁, A₂, A₃ und A₄ sowie die Reste Q und T die weiter oben beschriebenen Bedeutungen. In den allgemeinen Formeln **(VI)** und **(IX)** steht X für eine geeignete Abgangsgruppe und "Alk" für einen geeigneten Alkylrest, wie z.B. Methyl oder Ethyl.

Erfindungsgemäße Verbindungen der allgemeinen Formel **(I-1)** können durch die Umsetzung einer (hetero)aromatischen Aminoverbindungen der allgemeinen Formel **(VII)** mit aktivierten Carbonsäurederivaten der allgemeinen Formel **(IX)** dargestellt werden. Dabei gelten dieselben Bedingungen für die Wahl des Lösungsmittels, der Reaktionsbedingungen, der Reaktionsdauer und der Reagenzien wie bei der im Darstellungsverfahren A beschriebenen Umsetzung von Verbindungen der allgemeinen Formeln **(V)** und **(VI)** zu **(I-1).**

Die Darstellung von aktivierten Carbonsäurederivaten der allgemeinen Formel **(IX)** kann durch eine zweistufige Synthese aus den entsprechenden Carbonsäureestern der allgemeinen Formel **(VIII)** erfolgen. Im ersten Reaktionsschritt wird die in Form eines Carbonsäureesters geschützte Carbonsäurefunktion (O-Alk) der Verbindung **(VIII)** in Abhängigkeit des verwendeten Alkylesters mit einem geeigneten Reagenz entschützt [Greene's protective groups in organic synthesis, 4. Edition, P. G. M. Wuts, T. W. Greene, John Wiley & Sons, Inc., Hoboken, New Jersey] und die daraus resultierende freie Hydroxygruppe der Säurefunktion von **(IX-1)** in eine geeignete Abgangsgruppe X überführt. Dabei können dieselben Verfahren eingesetzt werden, die bereits in der Darstellung von **(VI)** beschrieben wurden. Verbindungen der allgemeinen Formel **(VIII)** können durch die Umsetzung von (hetero)aromatischen Aminoverbindungen der allgemeinen Formel **(VII)** mit aktivierten Carbonsäurederivaten der allgemeinen Formel **(VI)** dargestellt werden. Dabei gelten dieselben Bedingungen für die Wahl des Lösungsmittels, der Reaktionsbedingungen, der Reaktionsdauer, der Reagenzien wie bei der im Darstellungsverfahren A beschriebenen Synthese von Verbindungen der allgemeinen Formel **(I-1).**

Wenn es sich bei den erfindungsgemäßen Verbindungen der allgemeinen Formel **(I)** um Verbindungen der allgemeinen Formeln **(I-2)** und **(I-3)** handelt, kann die Synthese über das Darstellungsverfahren C erfolgen (vgl. Reaktionsschema 3).

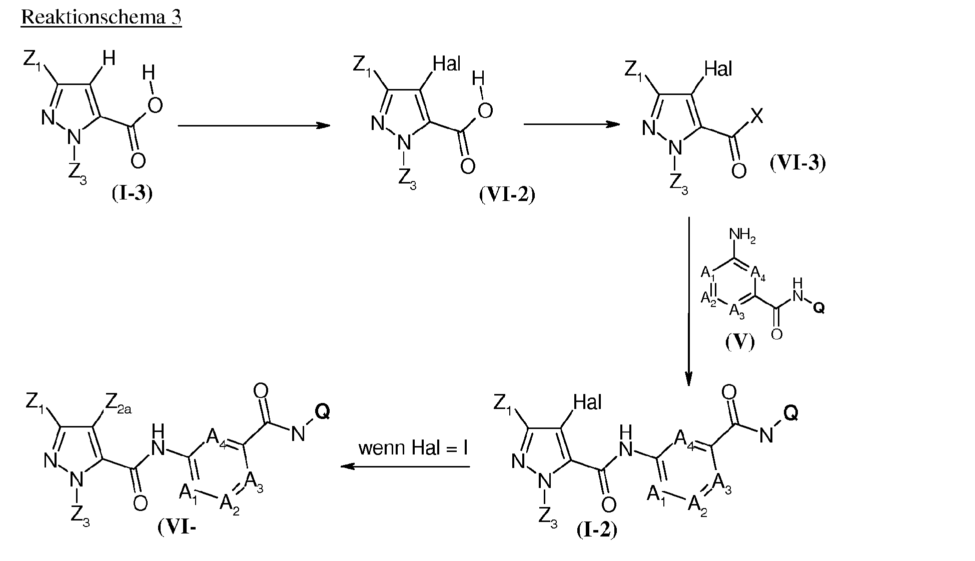

In den allgemeinen Formeln der Verbindungen **(I-2), (I-3), (V), (VI-1), (VI-2)** und **(VI-3)** im Reaktionsschema 3 haben die chemischen Gruppierungen A₁, A₂, A₃ und A₄ sowie die Reste Z₁, Z₃, und Q die weiter oben beschriebenen Bedeutungen. In den Verbindungen mit der allgemeinen Formel **(I-3)** steht der Rest Z₂ₐ für einen gegebenenfalls substituierten Aryl- oder Heteroarylrest, für einen gegebenenfalls substituierten Alkyl-, Alkenyl- oder Akinylrest, bevorzugt für einen Perfluoralkylrest. In den Verbindungen der allgemeinen Formel **(VI-3)** steht X für eine geeignete Abgangsgruppe. In den Verbindungen der allgemeinen Formel **(I-2), (VI-2)** und **(VI-3)** steht Hal steht für ein Halogen aus der Reihe Chlor, Brom oder Iod.

Erfindungsgemäße Verbindungen der allgemeinen Formel **(I-3)** können aus Verbindungen der allgemeinen Formel **(I-2),** worin Hal für Iod steht, dargestellt werden. In den Verbindungen der allgemeinen Formel **(I-2)** lässt sich das Iodatom metallvermittelt beispielsweise durch einen Perfluoralkylrest [WO 2005/095351], Aryl [J..Org. Chem. 2007, 72(9), 3589-3591; Synthesis 1997, (5), 563-566; Heterocycles 2006, 68(11), 2247-2252] oder Alkenylrest [WO 2005/060749; Org. Proc. Res. & Dev. 2005, 9(5), 694-696; Chem. & Pharm. Bull. 2005, 53(2), 153-163; J. Org. Chem. 1986, 51(26), 5286-90] ersetzen.

Die erfindungsgemäßen Verbindungen der allgemeinen Formel **(I-2)** können durch Umsetzung von aktivierten Carbonsäurederivaten der allgemeinen Formel **(VI-3)** mit (hetero)aromatischen Aminoverbindungen der allgemeinen Formel **(V)** dargestellt werden. Die Reaktionsbedingungen für diese Umsetzung sind bereits im Darstellungsverfahren A bei der Synthese von Verbindungen der allgemeinen Formel **(I-1)** beschrieben worden.

Die Verbindungen der allgemeinen Formel **(VI-3)** können in einem zweistufigen Reaktionsverfahren ausgehend von Pyrazol-5-carbonsäuren des Typs **(VI-1),** die nach literaturbekannten Methoden hergestellt werden können [J..Org. Chem. 2008, 73(9), 3523-3529; Bioorg. & Med. Chem. Letters 2005, 15(22), 4898-4906; US-Pat. 2006/069270], erhalten werden. Dabei werden im ersten Reaktionsschritt die Pyrazole der allgemeinen Formel **(VI-1)** nach literaturbekannten Methoden in der 4-Position halogeniert [Chlorierung: Bioorg. Med. Chem. Lett. 2007, 17, 5620-5623; Bromierung: Chem. Ber. 1976, 109, 268-273; Iodierung: Tetrahedron Lett. 2001, 42, 863-865]. Die Hydroxyfunktion der Pyrazol-5-carbonsäure von **(VI-2)** wird anschließend nach denen bereits im Darstellungsverfahren A zur Herstellung von **(VI)** beschriebenen Methoden in die Abgangsgruppe X überführt.

Im Reaktionschema 4 ist das allgemeine Darstellungsverfahren D für die Darstellung der erfindungsgemäßen Verbindungen der allgemeinen Formel **(I-4)** abgebildet. Die Synthese von Verbindungen der allgemeinen Formel **(I-4)** kann dabei nach zwei verschiedenen Reaktionswegen ausgehend von Intermediaten der allgemeinen Formel **(VI-9)** erfolgen. Zum Einen können diese Intermediate **(VI-9)** durch Kupplung mit (hetero)aromatischen Aminoverbindungen der allgemeinen Formel **(V)** zu **(I-4)** umgesetzt werden. Zum Anderen können die Intermediate **(VI-9)** auch mit (hetero)aromatischen Aminoverbindungen der allgemeinen Formel **(VII)** gekuppelt werden. Bei beiden Kupplungsreaktionen können die im Darstellungsverfahren A genannten Verfahren zur Darstellung der Verbindungen der allgemeinen Formel **(I-1)** aus **(V)** und **(VI)** verwendet werden. Anschließend wird der jeweilige Carbonsäureester von Verbindungen der allgemeinen Formel **(VIII-1)** unter literaturbekannten Bedingungen gespalten [Greene's protective groups in organic synthesis, 4. Edition, P. G. M. Wuts, T. W. Greene, John Wiley & Sons, Inc., Hoboken, New Jersey]. Die Hydroxyfunktionen der so erhaltenen Carbonsäuren der allgemeinen Formel **(IX-2)** werden anschließend analog zu denen im Darstellungsverfahren A beschriebenen Verfahren zur Herstellung von Verbindungen der allgemeinen Formel **(VI)** beschriebenen Methoden aktiviert und die so erhaltenen aktivierten Carbonsäurederivate der allgemeinen Formel **(IX-3)** mit (hetero)aromatischen Aminoverbindungen der allgemeinen Formel **(III)** zu den erfindungsgemäßen Verbindungen der allgemeinen Formel **(I-5)** gekuppelt. Bei der Kupplungsreaktion können die im Darstellungsverfahren A genannten Verfahren zur Darstellung der Verbindungen der allgemeinen Formel **(I-1)** aus **(V)** und **(VI)** verwendet werden. Die zentralen aktivierten Carbonsäurederivate der allgemeinen Formel **(VI-9)** sind aus den entsprechenenden Carbonsäuren der allgmeinen Formel **(VI-8)** nach denen bei der Synthese von **(VI)** beschrieben Methoden zugänglich. Die Verbindungen der allgemeinen Formel **(VI-8)** sind aus dem entsprechenden Ethylester **(VI-7)** nach literaturbekannten Verfahren durch Esterspaltung zugänglich [Greene's protective groups in organic synthesis, 4. Edition, P. G. M. Wuts, T. W. Greene, John Wiley & Sons, Inc., Hoboken, New Jersey]. Die Verbindungen der allgemeinen Formel **(VI-7)** können aus Verbindungen der allgemeinen Formel **(VI-6)** mit substituierten 5-gliedrigen Heterozyklen der allgemeinen Formel **(X)** gebildet werden. Bevorzugt werden hierbei 5-gliedrige Heterozyklen verwendet, die mindestens einen halogenierten Substituenten, beispielsweise einen perfluorierten Alkylrest enthalten.

Die Verbindungen der allgemeinen Formel **(VI-6)** können durch Sulfonylierung von Verbindungen der allgemeinen Formel **(VI-5)** mit geeigneten Sulfonierungsreagenzien wie z.B. Methansulfonsäure- oder Toluolsulfonsäurechlorid dargestellt werden. Beide Reaktionen können gemäß einem literaturbekannten Verfahren durchgeführt werden [Tetrahedron Assym. 2006, 17, 2149-2153]. Die Hydroxymethylverbindungen der allgemeinen Formel **(VI-5)** können analog zu einen bekannten Literaturverfahren durch selektive Reduktion aus Verbindungen der allgemeinen Formel **(VI-4)** dargestellt werden [J. Med. Chem. 2007, 50, 6291-6294]. Verbindungen der allgemeinen Formel **(VI-4)** lassen nach literaturbekannten Verfahren darstellen [J. Fluorine Chem. 1993, 61, 17-21].

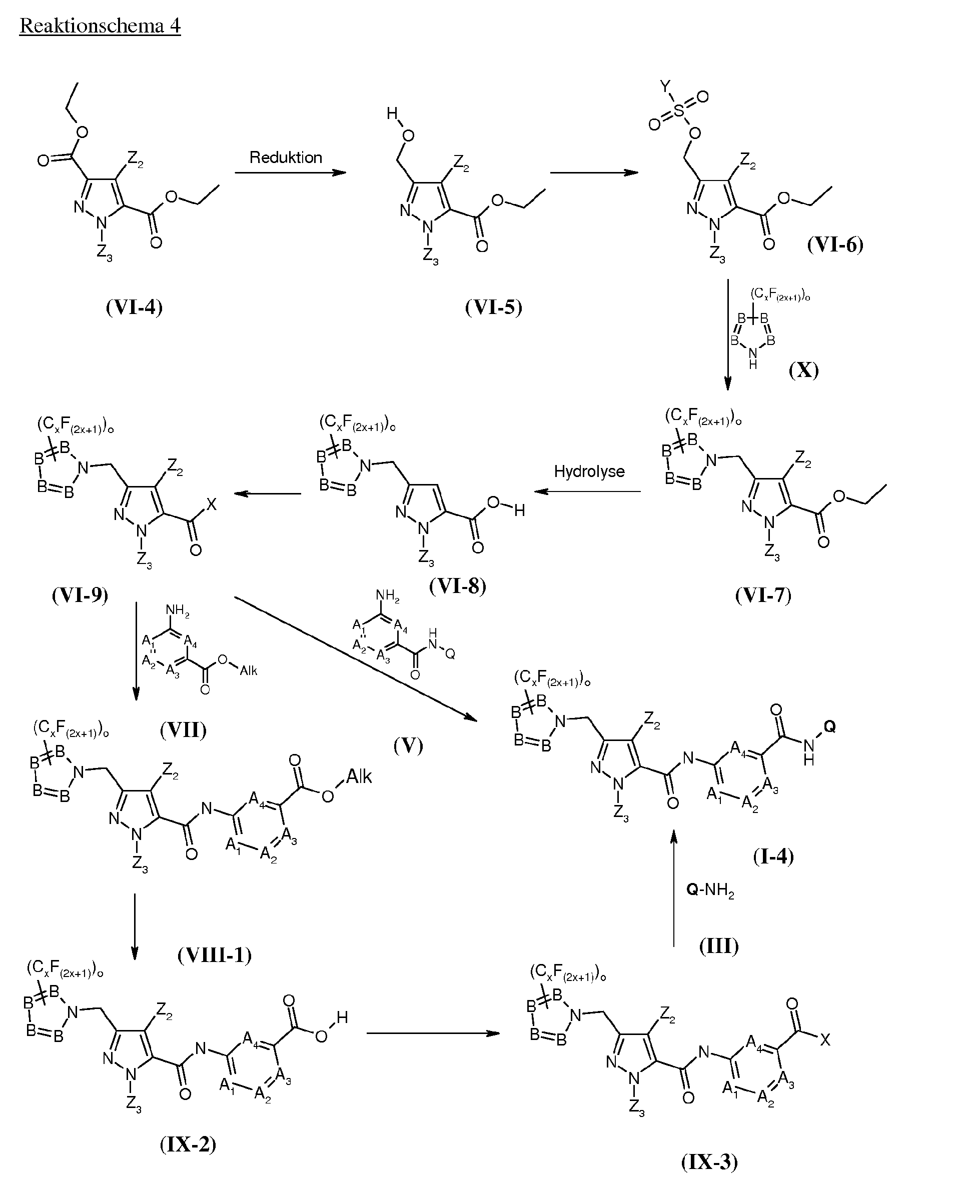

In den allgemeinen Formeln der Verbindungen **(I-4), (III), (V), (VI-4), (VI-5), (VI-6), (VI-7),** (**VI-8), (VI-9), (VII), (VIII-1), (IX-2), (IX-3)** und (X) im Reaktionsschema 4 haben die chemische Gruppierungen A₁, A₂, A₃ und A₄ sowie die Reste Z₂, Z₃, und Q die weiter oben beschriebenen Bedeutungen. In den Verbindungen der Formel **(IX-3)** und **(VI-9)** steht X für eine geeignete Abgangsgruppe und "Alk" für einen Alkylrest, wie z.B. Methyl oder Ethyl. In den Verbindungen der Formel **(VI-6)** steht Y für einen Alkyl- bzw. (Het)Arylrest wie z.B. Methyl oder *para*-Tolyl. In den allgemeinen Formeln der Verbindungen **(I-4), (VI-7), (VI-8), (VI-9), (VIII-1), (IX-2), (IX-3)** und **(X)** steht die chemische Gruppierung B unabhängig voneinander für ein gegebenenfalls substituiertes Kohlenstoffatom oder für ein Stickstoffatom, wobei der *N*-Heterozyklus bis zu vier Stickstoffatome enthalten. Bevorzugte Substituenten für das Kohlenstoffatom sind fluorierte Alkylreste der Formel -[CₓF₍₂ₓ₊₁₎]ₒ, in denen der Wert x für die Zahlen 1-3 und der Wert o für die Zahlen 1-2 stehen kann.

Aktivierte Carbonsäurederivate der allgemeinen Formel **(II)** sind aus (hetero)aromatischen Carbonsäuren der allgemeinen Formel **(II-1)** nach bereits im Darstellungsverfahren A beschriebenen Methoden zur Darstellung von **(VI)** zugänglich. Substanzen der allgemeinen Formel **(II-1)** sind allgemein bekannte Verbindungen der organischen Chemie, die nach ebenfalls bekannten Syntheseverfahren erhalten werden können. Mögliche Synthesewege der (hetero)aromatischen Aminocarbonsäuren der allgemeinen Formel **(II-1)** sind im Reaktionsschema 5 abgebildet.

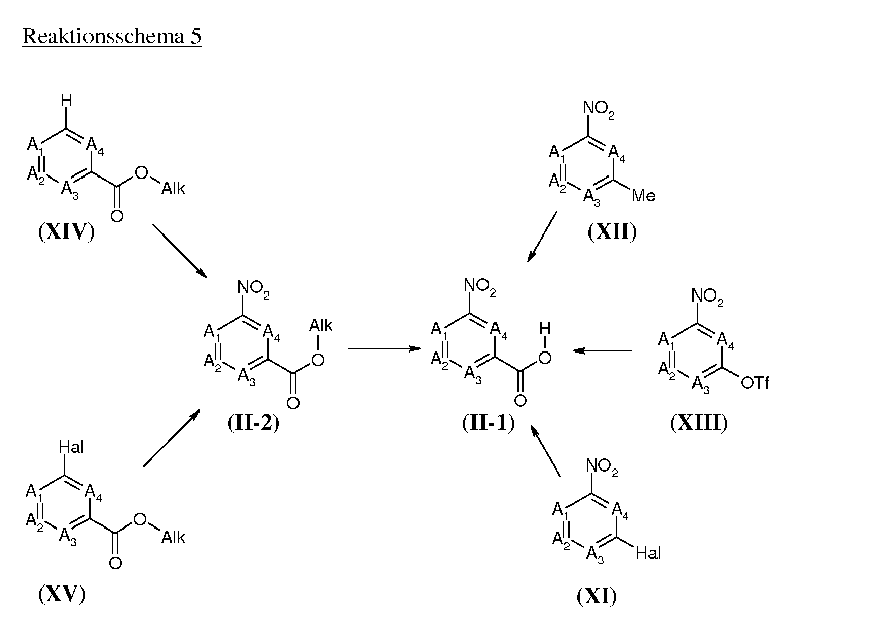

In den allgemeinen Formeln der Verbindungen **(II-1), (II-2), (XI), (XII), (XIII), (XIV)** und **(XV)** im Reaktionsschema 5 haben die chemische Gruppierungen A₁, A₂, A₃ und A₄ die oben beschriebenen Bedeutungen. Beispielsweise steht in den Verbindungen mit den allgemeinen Formeln **(II-2), (XIV)** und **(XV)** "Alk" für einen Alkylrest, wie z. B. Methyl oder Ethyl und in den Verbindungen der allgemeinen Formel **(XI)** "Hal" für ein Halogenatom aus der Reihe Fluor, Chlor, Brom und Iod.

Als Edukte zur Darstellung von Aminocarbonsäuren der allgemeinen Formel **(II-1)** können z.B. halogenierte (hetero)aromatische Nitroverbindungen der Formel **(XI)** eingesetzt werden. In den Verbindungen der Formel **(XI)** kann das Halogenatom durch eine Cyanogruppe ersetzt und diese anschließend unter saueren oder basischen Reaktionsbedingungen hydrolysiert werden. Der Halogen-Cyan-Austausch kann z.B. durch eine nucleophile Substitution am (Hetero)aromaten mit geeigneten Metallcyaniden wie z.B. Natriumcyanid [US 4,766,219] oder auch durch eine Kupfervermittelte Reaktion [J. Antibiotics 1994, 47(12), 1456-65] erfolgen.

Des Weiteren können (hetero)aromatische Aminocarbonsäuren der allgemeinen Formel **(II-1)** auch aus den entsprechenden Methylverbindungen des Typs **(XII)** durch Oxidation mit einem geeigneten Oxidationsmittel dargestellt werden (vgl. z. B. Oxidationsreagenzien in: Comprehensive Organic Transformations; R. C. Larock, Wiley-VCH, 1999; die auf den Seiten 1213-1215 zitierte Literatur; Methods for the Oxidation of Organic Compounds; A. H. Haines, Academic Press 1985; Kapitel 3.4, Seite 117; Houbel-Weyl, Methoden der Organischen Chemie, Bd. VII/2b Ketone Teil II, Georg Thieme Verlag Stuttgart, 1976, Seite 1287).

Als geeignete Oxidationsmittel sind beispielsweise saure Dichromate (vgl. Chromium Oxidations in Organic Chemistry; Cainelli, Cardillo, Springer Verlag: New York, 1984; Reagents for Organic Synthesis; Fieser, Vol. 1, Wiley: New York, 1967, S. 142-147, 1059-1064 und weitere Bände aus dieser Reihe), Chromtrioxid und diesen ähnliche Verbindungen [Tetrahedron Lett. 1995, 36(25), 4369-72; Bioorg. Med. Chem. Lett. 2007, 17(4), 1043-1046] oder Permanganate, wie Kaliumpermanganat, oder Periodate wie beispielsweise Natriumperiodat, bekannt. Die Oxidationsreagenzien können aber auch polymergebunden vorliegen (vgl. Review: McKillop, Young Synthesis 401-422 (1979)). Sowohl Chromsäuren als auch Permanganate wurden auf diese Weise als Oxidationsmittel verwendet. Ebenfalls sind zahlreiche Phasen-Transfer-Reaktionen mit Permanganaten, Chromsäuren (Tetrahedron Lett. 4167 (1977), Landini et al. 134 (1979)) und Rutheniumtetroxid (Morris, Kiely J. Org. Chem. 52, 1149 (1987)) bekannt. Selbst Ultraschallinduzierte Oxidationsreaktionen sind denkbar - so ist die Verwendung von Kaliumpermanganat erwähnt (Yamawaki et al. 379 (1983)). Ebenso können für solche Oxidationen auch enzymatische Verfahren verwendet werden [WO 95/02061].

Die anschließend notwendige Reduktion der Nitrofunktion kann analog zu den weiter oben beschriebenen Verfahren durchgeführt werden.

Des Weiteren können (hetero)aromatische Aminocarbonsäuren der allgemeinen Formel **(II)** aus den entsprechenden (Hetero)aryl-Triflaten des Typs **(XIII)** mit Hilfe eines Palladium-katalysierten Verfahrens hergestellt werden [Synthesis 2006, (4), 594-596].

Eine weitere Methode zur Darstellung von (hetero)aromatischen Aminocarbonsäuren der allgemeinen Formel **(II-1)** ist die literaturbekannte Spaltung von Carbonsäureestern der allgemeinen Formel **(II-2)** [Greene's protective groups in organic synthesis, 4. Edition, P. G. M. Wuts, T. W. Greene, John Wiley & Sons, Inc., Hoboken, New Jersey]. Diese Ester sind über verschiedene literaturbekannte Verfahren zugänglich und können beispielsweise aus den Verbindungen der allgemeinen Formeln **(XIV)** und **(XV)** durch Nitrierung dargestellt werden. Die Nitrierungen können beispielsweise analog zu literaturbekannten Verfahren durchgeführt werden [Justus Liebigs Ann..Chem. 1958, 611, 194-205; Organikum, Wiley-VCH, 22. Auflage, 358ff].

Die Synthese der (hetero)aromatischen Carbonsäurederivate der allgemeinen Formel **(VII)** sind im Reaktionsschema 6 abgebildet. Die Darstellung von Verbindungen der allgemeinen Formel **(VII)** kann durch die Reduktion der Nitrofunktion von Verbindungen der allgemeinen Formel **(II-2)** erfolgen. Die Herstellung von Verbindungen der allgemeinen Formel **(II-2)** ist bereits im weiter oben genannten Reaktionschema 5 beschrieben. Des Weiteren können diese Verbindungen auch durch Veresterung der Carbonsäurefunktion von Verbindungen der allgemeinen Formel **(II-1)** synthetisiert werden. Die Veresterungen können beispielsweise nach literaturbekannten Verfahren durchgeführt werden [Organikum, Wiley-VCH, 22. Auflage, 474ff; Bioorg. Med. Chem. Lett. 2007, 17(22), 6305-6310].

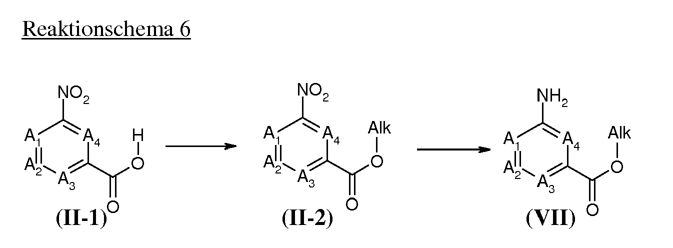

In den allgemeinen Formeln der Verbindungen **(II-1), (II-2)** und **(VII)** im Reaktionsschema 6 haben die chemische Gruppierungen A₁, A₂, A₃ und A₄ die weiter oben beschriebenen Bedeutungen. In den allgemeinen Formeln **(II-2)** und **(VII)** steht "Alk" für einen Alkylrest, wie z.B. Methyl oder Ethyl.

Die Synthese der (hetero)aromatischen Carbonsäurederivate der allgemeinen Formel **(VI)** ist im Reaktionsschema 7 abgebildet.

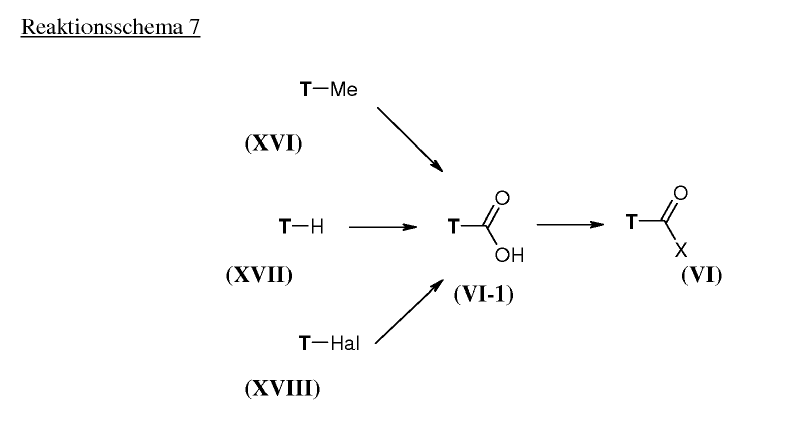

In den Formeln der Verbindungen **(VI-1), (VI), (XVI), (XVII)** und **(XVIII)** im Reaktionsschema 7 hat der Rest T hat die weiter oben beschriebene Bedeutung. In der Formel **(XVIII)** steht Hal für ein Halogenatom aus der Reihe Fluor, Chlor, Brom und Iod. In der Formel **(VI)** steht X steht für eine geeignete Abgangsgruppe.

Die (hetero)aromatischen Carbonsäuren der allgemeinen Formel **(VI-1)** können unter anderem aus Methylderivaten der allgemeinen Formel **(XVI),** beispielsweise durch Oxidation der Methylgruppe dargestellt werden. Dabei können die bereits bei der Oxidation von Methylgruppen der Verbindungen der allgemeinen Formel **(XII)** genannten Verfahren angewendet werden.

Die (hetero)aromatischen Carbonsäuren der allgemeinen Struktur **(VI-1)** können ebenfalls aus geeigneten Verbindungen der allgemeinen Formel **(XVII)** durch Deprotonierung mit einer geeigneten Base und durch Abfangen des entsprechenden Carbanions mit Kohlendioxid dargestellt werden [J. Med. Chem. 2008, 51(4), 937-947; Bioorg..Med. Chem. Lett. 2007, 17(22), 6274-6279]. Als Base eignen sich z.B. Lithiumdiisopropylamid, n-Butyllithium, sec-Butyllithium und diesen ähnliche Verbindungen.

Für das oben beschriebene Verfahren zur Darstellung von (hetero)aromatischen Carbonsäuren der allgemeinen Formel **(VI-1)** eignen sich ebenfalls die entsprechenden halogenierten Heterozyklen der allgemeinen Formel **(XVIII).** Dabei wird das Carbanion allerdings nicht durch Deprotonierung generiert, sondern durch eine Metallierungsreaktion [Angew. Chem., Intern. Ed. 2008, 47(2), 311-315]. Für diese Metallierungsreaktionen eignen sich vorzugsweise n-Butyllithium, tert-Butyllithium und iso-Propylmagnesiumchlorid.

Die (hetero)aromatischen Carbonsäuren der allgemeinen Formel **(VII-1)** können ebenfalls aus halogenierten Verbindungen der allgemeinen Formel **(XVIII)** mit Hilfe von literaturbekannten Palladium-katalysierten Reaktionen in die entsprechenden (hetero)aromatischen Carbonsäureester überführt [Russ. J. .Appl. Chem. 2007, 80(4), 571-575]. Diese (hetero)aromatischen Carbonsäureester können dann nach literaturbekannten Verfahren gespalten werden. [Greene's protective groups in organic synthesis, 4. Edition, P. G. M. Wuts, T. W. Greene, John Wiley & Sons, Inc., Hoboken, New Jersey]

Die (hetero)aromatischen Carbonsäuren der allgemeinen Formel **(VII-1)** können des Weiteren aus halogenierten Verbindungen der allgemeinen Formel **(XVIII)** durch eine Substitutionsreaktion der Halogene mit Cyaniden und anschließender Hydrolyse der Nitrilfunktion mit starken Säure oder Basen dargestellt werden [WO 2005/079801].

Als saure Reaktionshilfsmittel zur Durchführung des erfindungsgemäßen Verfahrens können alle Mineralsäuren (z.B. Halogenwasserstoff-säuren wie Fluorwasserstoffsäure, Chlorwasserstoffsäure, Bromwasserstoffsäure oder Iodwasserstoffsäure sowie Schwefelsäure, Phosphorsäure, Phosphorige Säure, Salpetersäure) eingesetzt werden.

Bevorzugt werden Mineralsäuren wie Chlorwasserstoffsäure eingesetzt.

Die (hetero)aromatischen aktivierten Carbonsäurederivate, wie z.B. (hetero)aromatische Carbonsäurehalogenide, wie sie durch die allgemeine Formel **(VI)** repräsentiert werden, können durch Umsetzung einer (hetero)aromatischen Carbonsäure der allgemeinen Formel **(VI-1)** mit geeigneten Halogenierungsreagenzien wie Thionylchlorid, Thionylbromid, Phosphorylchlorid, Oxalylchlorid, Phosphortrichlorid, etc. hergestellt werden [Organikum, Wiley-VCH, 22. Auflage, 496ff].

Aktivierte (hetero)aromatische Carbonsäurederivate der allgemeinen Formel **(II)** können nach allgemein bekannten Methoden aus (hetero)aromatischen Carbonsäuren der Formel **(II-1)** dargestellt werden [Organikum, Wiley-VCH, 22. Auflage, 496ff; Chem. Ber. 1970, 788; J. Am. Chem. Soc 1967, 5012]. Die Verbindungen der Formel **(II-1)** sind kommerziell erhältlich oder können nach bekannten Literaturverfahren hergestellt werden [Synthesis 2006, (4), 594-596; Tetrahedron Lett. 1995, 36(25), 4369-72; Bioorg..Med. Chem. Lett. 2007, 17(4), 1043-1046; WO 95/02061, J..Org. Chem. 1954, 19, 357-64; WO 2001/083459].

Verbindungen der allgemeinen Formel **(III)** sind kommerziell erhältlich und/oder können nach allgemein in der Fachliteratur bekannten Verfahren hergestellt werden [Houben-Weyl (1992), Vol. E, 16d, 646ff; Tetrahedron Lett., 33(24), 3487-90; 1992].

Verbindungen der allgemeinen Formel **(VI)** sind im Allgemeinen ebenfalls kommerziell erhältlich und/oder können nach bekannten Literaturverfahren dargestellt werden [Journal of Medicinal Chemistry 2008, 51(4), 937-947; Bioorg. Med. Chem. Lett. 2007, 17(22), 6274-6279; Russ. J. .Appl. Chem. 2007, 80(4), 571-575; WO 2005079801; J. Org.Chem. 2008, 73(9), 3523-3529; Bioorg. Med. Chem. Lett. 2005, 15(22), 4898-4906; US-Pat. 2006/069270; J. Fluorine Chem. 1993, 61, 17-21]

### Experimenteller Teil

### Darstellungsverfahren A

### Beispiel Ia-32: N-{4-Chlor-3-r(3-chlorohenyl)carbamoyl]phenyl}-2-(pentafluorethyl)-4-(trifluormethyl)pyrimidin-5-carboxamid

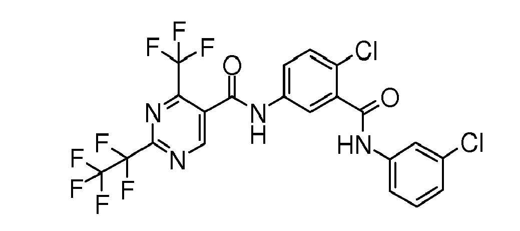

In 50,0 ml Dichlormethan werden 2,10 g (6,77 mM, 1,0 eq) 2-(Pentafluorethyl)-4-(trifluormethyl)pyrimidin-5-carbonsäure mit 2 Tropfen DMF versetzt und auf 0 °C gekühlt. Nach tropfenweiser Zugabe von 0,71 ml (8,13 mM, 1,2 eq) Oxalsaeuredichlorid wird das Reaktionsgemisch 3h bei Raumtemperatur gerührt und anschließend eingeengt. Man erhält das Rohprodukt 2-(Pentafluorethyl)-4-(trifluormethyl)pyrimidin-5-carbonylchlorid.

In 2 mL absolutem Essigsäureethylester werden 84,3 mg (0,3 mM) 5-Amino-2-chlor-N-(3-chlorphenyl)benzamid, 3,05 mg (0,025 mM) *N,N*-Dimethylpyridin-4-amin (DMPA) gelöst. Die Lösung wird mit einem Eisbad auf 0 °C gekühlt und mit 96.9 mg (0.750 mM) *N*-Ethyl-diisopropylamin versetzt. 88,1 mg (0.25 mM) 2-(Pentafluorethyl)-4-(trifluormethyl)pyrimidin-5-carbonylchlorid (wird als Rohprodukt eingesetzt) werden in 2 mL absolutem Essigsäureethylester gelöst bzw. suspendiert und dann zu der gekühlten Reaktionslösung hinzugegeben. Das Reaktionsgemisch wird 6 Stunden in einem Ölbad (Badtemperatur 50 °C) erhitzt und anschließend 16 Stunden bei Raumtemperatur weitergerührt. Die Reaktionslösung wird mit 8 mL Essigsäureethylester verdünnt. Die organische Phase wird zweimal mit 1 N Salzsäure, einmal mit 1 N Natronlauge und einmal mit gesättigter Natriumchloridlösung gewaschen. Die organische Phase wird über Natriumsulfat getrocknet, filtriert und Lösungsmittel am Rotationsverdampfer unter vermindertem Druck entfernt. Nach chromatographischer Reinigung mit einer Mischung von Cyclohexan und Essigsäureethylester (2:1) erhält man 82 mg (0,14 mM, 57 % der Theorie) *N*-{4-Chlor-3-[(3-chlorphenyl)carbamoyl]phenyl}-2-(pentafluorethyl)-4-(trifluormethyl)pyrimidin-5-carboxamid als weißen Feststoff.
1H-NMR (400 MHz, d6-DMSO): δ = 11.02 (bs, 1H), 10.59 (bs, 1H), 9.71 (s, 1H), 7.87 (s, 1H), 7.82 ( d, 1H), 7.74 (dd, 1H), 7.61-7.58 (m, 2H), 7.38 (t, 1H), 7.18-7.15 (m, 1H) ppm.

HPLC-MS^{a)}: logP = 4,32 Masse (m/z) = 573 [M+H]⁺.

### Beispiel Ia-3: N-{4-Chlor-3-[(4-fluorphenyl)carbamoyl]pheny;}-2-methyl-6-(trifluormethxl)nicotinamid

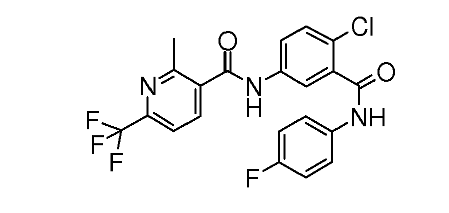

In 4,8 ml Dichlormethan werden 246,2 mg (1,2 mM, 1,0 eq) 2-Methyl-6-(trifluormethyl)nicotinsäure mit 2 Tropfen DMF versetzt und auf 0 °C gekühlt. Nach tropfenweiser Zugabe von 0,13 ml (1,44 mM, 1,2 eq) Oxalsaeuredichlorid wird das Reaktionsgemisch 3h bei Raumtemperatur gerührt und anschließend eingeengt. Man erhält das Rohprodukt 2-Methyl-6-(trifluormethyl)nicotinoylchlorid.

In 2 mL absolutem Essigsäureethylester werden 95,3 mg (0.36 mM) 5-Amino-2-chlor-N-(4-fluorphenyl)benzamid, 18,3 mg (0,15 mM) *N,N*-Dimethylpyridin-4-amin (DMPA) gelöst. Die Lösung wird mit einem Eisbad auf 0 °C gekühlt und mit 0,16ml (0.90 mM) *N*-Ethyl-diisopropylamin versetzt. 67,1 mg (0.30 mM) 2-Methyl-6-(trifluormethyl)nicotinoylchlorid (wird als Rohprodukt eingesetzt) werden in 2 mL absolutem Essigsäureethylester gelöst bzw. suspendiert und dann zu der gekühlten Reaktionslösung hinzugegeben. Das Reaktionsgemisch wird 6 Stunden in einem Ölbad (Badtemperatur 50 °C) erhitzt und anschließend 16 Stunden bei Raumtemperatur weitergerührt. Die Reaktionslösung wird mit 8 mL Essigsäureethylester verdünnt. Die organische Phase wird zweimal mit 1 N Salzsäure, einmal mit 1 N Natronlauge und einmal mit gesättigter Natriumchloridlösung gewaschen. Die organische Phase wird über Natriumsulfat getrocknet, filtriert und Lösungsmittel am Rotationsverdampfer unter vermindertem Druck entfernt.

Man erhält 135 mg (0,29 mM, 96,6% der Theorie) *N*-{4-Chlor-3-[(4-fluorphenyl)carbamoyl]phenyl}-2-methyl-6-(trifluormethyl)nicotinamid als weißen Feststoff.
1H-NMR (400 MHz, d6-DMSO): δ = 10.69 (bs, 1H), 10.41 (bs, 1H), 8.16 (d, 1H), 7.91 (d, 1H), 7.85 (d, 1H), 7.79 (dd, 1H), 7.73-7.70 (m, 2H), 7.54 (d, 1H), 7.19-7.14 (m, 2H), 2.65(s, 3H) ppm HPLC-MS^{a)}: logP = 3,15 Masse (m/z) = 452 [M+H]⁺.

### Darstellungsverfahren B

### Beispiel Ic-1: N-[3-Brom-2-(4-fluor-phenylcarbamoyl)pyrid-6-yl]-1-methyl-3-(pentafluorethyl)-4-(trifluormethyl)-1H-pyrazol-5-carbonsäureamid

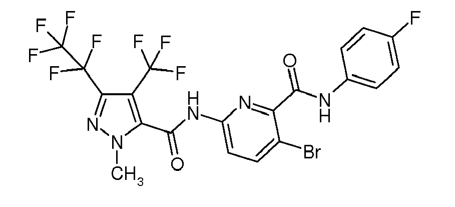

100,0 mg (1.0 eq, 0.196 mM) 3-Brom-6-({[1-methyl-3-(pentafluorethyl)-4-(trifluormethyl)-1*H-*pyrazol-5-yl]carbonyl}-amino)pyridin-2-carbonsäure werden in 9.0 mL Dichlormethan abs. gelöst/suspendiert und anschließend nacheinander mit 21,7 mg (1.0 eq, 0.196 mM) 4-Fluor-anilin, 96,6 mg (1.3 eq, 0.254 mM) 2-(7-Aza-1*H*-benzotriazole-1-yl)-1,1,3,3-tetramethyluronium hexafluorophosphat (HATU) und 97 µL (3.0 eq, 0.587 mM) *N*-Ethyl-diisopropylamin (Hünig's Base) versetzt. Das Reaktionsgemisch wird 30 Stunden bei Raumtemperatur gerührt. Zur Aufarbeitung wird der Reaktionsansatz nacheinander mit 1N Salzsäure und Natriumhydrogencarbonat-Lösung gewaschen. Die organische Phase wird abgetrennt, über Natriumsulfat getrocknet und im Vakuum eingeengt. Der verbleibende Rückstand wird zunächst mittels Säulenchromatographie an Kieselgel (Kieselgel 60 - Merck, Korngröße: 0,04 bis 0,063 mm) mit dem Laufmittelgemisch Cyclohexan:Aceton (Gradient) und anschließend mittels präparativer HPLC mit dem Laufmittelgemisch Aceton:Wasser (neutral) gereinigt.

Man erhält 51,2 mg (43,3% der Theorie) *N*-[3-Brom-2-(4-fluor-phenylcarbamoyl)pyrid-6-yl]-1-methyl-3-(pentafluorethyl)-4-(trifluormethyl)-1*H*-pyrazol-5-carbonsäureamid.
¹H-NMR (400 MHz, d₆-DMSO): δ = 12.0 (s, 1H), 10.5 (s, 1H), 8.29 (d, 1H), 8.15 (br. 1H), 7.68 (dd, 2H), 7,17 (dd, 2H), 3.98 (s, 3H) ppm.
HPLC-MS^{a)}: logP = 4.45, Masse [M]⁺ = 604,0.

### Beispiel Ib-1: N-[2-(4-fluor-phenylcarbamoyl)pyrid-4-yl]-1-methyl-3-(pentafluorethyl)-4-(trifluormethyl)-1H-pyrazol-5-carbonsäureamid

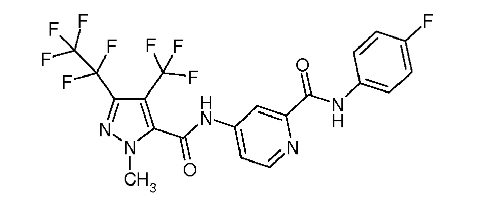

150,0 mg (1.0 eq, 0.347 mM) 4-({[1-methyl-3-(pentafluorethyl)-4-(trifluormethyl)-1*H*-pyrazol-5-yl]carbonyl}-amino)pyridin-2-carbonsäure werden in 13,5 mL Dichlormethan abs. gelöst/suspendiert und anschließend nacheinander mit 38,6 mg (1.0 eq, 0.347 mM) 4-Fluor-anilin, 171,4 mg (1.3 eq, 0.451 mM) 2-(7-Aza-1*H*-benzotriazole-1-yl)-1,1,3,3-tetramethyluronium hexafluorophosphat (HATU) und 172 µL (3.0 eq, 0.587 mM) *N*-Ethyl-diisopropylamin (Hünig's Base) versetzt. Das Reaktionsgemisch wird 30 Stunden bei Raumtemperatur gerührt. Zur Aufarbeitung wird der Reaktionsansatz nacheinander mit 1N Salzsäure und Natriumhydrogencarbonat-Lösung gewaschen. Die organische Phase wird abgetrennt, über Natriumsulfat getrocknet und im Vakuum eingeengt. Der verbleibende Rückstand wird zunächst mittels Säulenchromatographie an Kieselgel (Kieselgel 60 - Merck, Korngröße: 0,04 bis 0,063 mm) mit dem Laufmittelgemisch Cyclohexan:Aceton (Gradient) und anschließend mittels präparativer HPLC mit dem Laufmittelgemisch Aceton:Wasser (neutral) gereinigt.

Man erhält 94,9 mg (52,0 % der Theorie) *N*-[2-(4-fluor-phenylcarbamoyl)pyrid-4-yl]-1-methyl-3-(pentafluorethyl)-4-(trifluormethyl)-1*H*-pyrazol-5-carbonsäureamid.
¹H-NMR (400 MHz, CDCl₃,) δ = 9.88 (s, 1H), 8.52 (s, 1H), 8.19 (d, 1H), 8.11 (s, 1H), 7.58 (s, 1H), 7.08 (d, 2H), 7.05 (d, 2H),1.61 (s, 3H) ppm.
HPLC-MS^{a)}: logP = 4.30, Masse [M+H]⁺ = 526,0.

### Beispiel Ia-18: N-{4-Chlor-3-[(2-fluorohenyl)carbamoyl]phenyl}-1-methyl-3-(pentafluorethyl)-4-(trifluormethyl)-1H-pyrazol-5-carboxamid

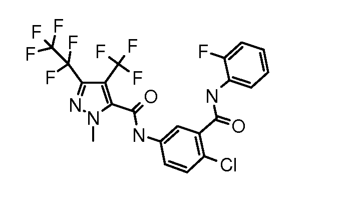

70mg (1eq, 0.15mmol) 2-Chlor-5-({[1-methyl-3-(pentafluorethyl)-4-(trifluormethyl)-1H-pyrazol-5-yl]carbonyl}-amino)benzolcarbonsäure werden in 2mL Dichlormethan gelöst / suspendiert. Dann werden 25mg (1.0 eq, 0.15mmol) 6-Chloro-hydroxybenzotriazol, 37mg (1.3eq, 0.195mmol) *N*-(3-Dimethylaminopropyl)-N'-ethyl-carbodiimid-hydrochloridund 52.3 µL (2eq, 0.3mmol) N-Ethyl-diisopropylamin dazugegeben und die Mischung 20min bei RT gerührt. Anschliessend werden 21mg (1.3eq, 0.195mmol) 2-Fluoroanilin dazugegeben und die Mischung bei Raumtemperatur über Nacht gerührt. Nach dieser Zeit wurde die Reaktionsmischung eingeengt und das erhaltene Rohprodukt über präparative HPLC (C18, Saphir 110, 5 µm, 20x125mm; Gradient: 0-1.5 min 94% Wasser, 5% Acetonitril, 1% Ameisensäure, 1.5-6.0 min linearer Gradient auf 4% Wasser, 95% Acetonitril,1% Ameisensäure, 6.0-14.0 min 4% Wasser, 95% Acetonitril, 1% Ameisensäure) gereinigt. Man erhält 39mg (46%) *N*-{4-Chlor-3-[(2-fluorphenyl)carbamoyl]phenyl}-1-methyl-3-(pentafluorethyl)-4-(trifluormethyl)-1*H*-pyrazol-5-carboxamid als weißen Feststoff.
¹H-NMR (400 MHz, d₆-DMSO): δ = 11.30 (s, 1H), 10.20 (s, 1H), 7.88 (d, 1H), 7.75 (m, 2H), 7.60 (d, 1H), 7.25 (m, 3H), 4.02 (s, 3H) ppm.
HPLC-MS^{b)}: logP = 3.94, Masse [M+H]+ = 558.9

### Beispiel Ia-8: N-[4-Chlor-3-(ptrid-2-yl-carbamoyl)phenyl]-1-methyl-3-(pentafluorethyl)-4-(trifluormethyl)-1H-pyrazol-5-carboxamid

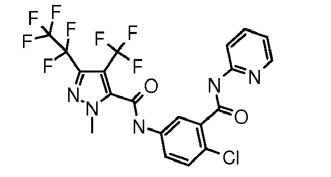

70mg (1eq, 0.15mmol) 2-Chlor-5-({[1-methyl-3-(pentafluorethyl)-4-(trifluormethyl)-1H-pyrazol-5-yl]carbonyl}-amino)benzolcarbonsäure werden in 2mL Dichlormethan gelöst / suspendiert. Dann werden 25mg (1.0 eq, 0.15mmol) 6-Chloro-hydroxybenzotriazol, 37mg (1.3eq, 0.195mmol) *N*-(3-Dimethylaminopropyl)-N'-ethyl-carbodiimid-hydrochloridund 52.3 µL (2eq, 0.3mmol) N-Ethyl-diisopropylamin dazugegeben und die Mischung 20min bei RT gerührt. Anschliessend werden 18mg (1.3eq, 0.195mmol) 2-Aminopyridin dazugegeben und die Mischung bei Raumtemperatur über Nacht gerührt. Nach dieser Zeit wurde die Reaktionsmischung eingeengt und das erhaltene Rohprodukt über präparative HPLC (C18, Saphir 110, 5 µm, 20x125mm; Gradient: 0-1.5 min 94% Wasser, 5% Acetonitril, 1% Ameisensäure, 1.5-6.0 min linearer Gradient auf 4% Wasser, 95% Acetonitril,1% Ameisensäure, 6.0-14.0 min 4% Wasser, 95% Acetonitril, 1% Ameisensäure) gereinigt. Man erhält 46mg (57%) *N*-[4-Chlor-3-(pyrid-2-yl-carbamoyl)phenyl]-1-methyl-3-(pentafluorethyl)-4-(trifluormethyl)-1*H*-pyrazol-5-carboxamid als weißen Feststoff.
¹H-NMR (400 MHz, d6-DMSO): δ = 11.30 (s, 1H), 10.80 (s, 1H), 8.35 (dd, 1H), 8.13 (d, 1H), 7.85 (m, 2H), 7.73 (dd, 1H), 7.55 (d, 1H), 7.15 (dd, 1H), 4.02 (s, 3H) ppm.
HPLC-MS^{b)}: logP = 3.53, Masse [M+H]+ = 541.9

### Beispiel Ia-42: N-{4-Chlor-3-[3,4-dichlorphenyl)carbamoyl]phenyl}-1-meth-3-(pentafluorethyl)-4-(trifluormethyl)-1H-pyrazol-5-carboxamid

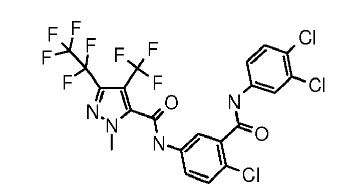

70mg (1eq, 0.15mmol) 2-Chlor-5-({[1-methyl-3-(pentafluorethyl)-4-(trifluormethyl)-1H-pyrazol-5-yl]carbonyl}-amino)benzolcarbonsäure werden in 2mL Dichlormethan gelöst / suspendiert. Dann werden 25mg (1.0 eq, 0.15mmol) 6-Chloro-hydroxybenzotriazol, 37mg (1.3eq, 0.195mmol) *N*-(3-Dimethylaminopropyl)-N'-ethyl-carbodiimid-hydrochlorid und 52.3 µL (2eq, 0.3mmol) N-Ethyl-diisopropylamin dazugegeben und die Mischung 20min bei RT gerührt. Anschliessend werden 32mg (1.3eq, 0.195mmol) 3,4-dichloranilin dazugegeben und die Mischung bei Raumtemperatur über Nacht gerührt. Nach dieser Zeit wurde die Reaktionsmischung eingeengt und das erhaltene Rohprodukt über präparative HPLC (C18, Saphir 110, 5 µm, 20x125mm; Gradient: 0-1.5 min 94% Wasser, 5% Acetonitril, 1% Ameisensäure, 1.5-6.0 min linearer Gradient auf 4% Wasser, 95% Acetonitril, 1% Ameisensäure, 6.0-14.0 min 4% Wasser, 95% Acetonitril, 1% Ameisensäure) gereinigt. Man erhält 20mg (21%) *N*-{4-Chlor-3-[(3,4-dichlorphenyl)carbamoyl]phenyl}-1-methyl-3-(pentafluorethyl)-4-(trifluormethyl)-1*H*-pyrazol-5-carboxamid als weißen Feststoff.
1H-NMR (400 MHz, d6-DMSO): δ = 11.30 (s, 1H), 10.70 (s, 1H), 8.05 (s, 1H), 7.85 (d, 1H), 7.75 (dd, 1H), 7.60 (m, 3H), 4.02 (s, 3H) ppm.
HPLC-MS^{b)}: logP = 4.65, Masse [M+H]+ = 610.8

### Darstellungsverfahren C

### Beispiel Ia-35: 4-Bromo-N-{4-chloro-3-[(4-chloropyridin-2-yl)carbamoyl]phenyl}-1-methyl-3-(pentafluoroethyl)-1H-pyrazole-5-carboxamid

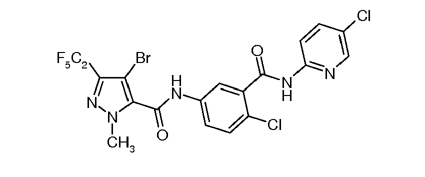

Zu einer Lösung von 0,10 g (0,21mmol) 5-({[4-Bromo-1-methyl-3-(pentafluoroethyl)-1*H*-pyrazol-5-yl]carbonyl}amino)-2-chlorobenzoesäure und 0,0483 g (0,25 mmol) 1-Ethyl-3-[3-dimethylaminopropyl]carbodiimid Hydrochlorid in 20 ml Dioxan werden 0,0270 g (0,210 mmol) 5-chloropyridin-2-amin gelöst in 0,5 ml Dioxan getropft und das Reaktionsgemisch 18 Stunden bei Raumtemperatur gerührt. Das Dioxan wird zum Großteil bei vermindertem Druck am Rotationsverdampfer abdestilliert und der Rückstand mit 10 ml Wasser versetzt. Die wässrige Phase wird dreimal mit Ethylacetat extrahiert und die organische Phase anschließend dreimal mit gesättigter Kochsalzlösung gewaschen. Nach Trocknen über Natriumsulfat wird das Lösungsmittel am Rotationsverdampfer bei vermindertem Druck abdestilliert und der Rückstand mittels Flashchromatographie an Kieselgel gereinigt (Eluent: Cyclohexan/Essigester; Gradient: 2 Stunden, von 0% auf 100 % Essigester). Man erhält 0,011 g (8,1 % der Theorie) 4-Bromo-*N*-{4-chloro-3-[(4-chloropyridin-2-yl)carbamoyl]phenyl}-1-methyl-3-(pentafluoroethyl)-1*H*-pyrazole-5-carboxamide als Öl.
¹H-NMR (400 MHz, d₃-Acetonitril): δ = 4.08 (s, 3H), 7.21 (m, 1H), 7.55 (m, 1H), 7.80 (m, 1H), 7.93 (m, 1H), 8.24 (m, 1H), 8.34 (s, 1H), 9.01 (br. s, 1H), 9.29 (br. s, 1H)ppm.
HPLC-MS^{a)}: logP = 4,38, Masse (m/z) = 587,8 [M+H]⁺.

### Herstellungsverfahren D

### Beispiel Ia-43: 3-{[3.5-Bis(pentafluorethyl)-1H-pyrazol-l-yl]methyl}-N-{4-chlor-3-[(4-fluorphenyl)carbamoyl]phenyl}-1-methyl-4-(trifluormethyl)-1H-pyrazol-5-carboxamid

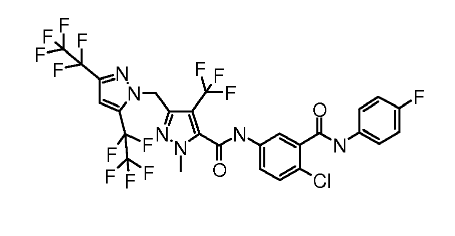

1,83 g (3,59 mM) 3-{[3,5-Bis(pentafluorethyl)-1H-pyrazol-1-yl]methyl}-1-methyl-4-(trifluormethyl)-1H-pyrazol-5-carbonsäure in 10 mL Dichlormethan werden mit 0,90 g Oxalsäuredichlorid (7,17 mM) und 26 mg *N,N*-Dimethylformamid (0,36 mM) versetzt und 3 h bei Raumtemperatur gerührt. Es wird im Vakuum eingeengt und man erhält 1,8 g 3-{[3,5-Bis(pentafluorethyl)-1H-pyrazol-1-yl]methyl}-1-methyl-4-(trifluormethyl)-1H-pyrazol-5-carbonylchlorid (3,40 mM, 90% der Theorie), das ohne weitere Aufreinigung im nächsten Schritt umgesetzt wird.

Zu 0,28 g 5-Amino-2-chlor-N-(4-fluorphenyl)benzamid (1,04 mM) und 0,11 g 4-Methylmorpholin (1,04 mM) in 15 mL Dichlormethan wird bei -10°C 0,50 g 3-{[3,5-Bis(pentafluorethyl)-1H-pyrazol-1-yl]methyl}-1-methyl-4-(trifluormethyl)-1H-pyrazol-5-carbonylchlorid (0,95 mM) in 5 mL Dichlormethan zugetropft. Es wird auf Raumtemperatur erwärmt und 1 h nachgerührt. Nach Zugabe von 10 mL Wasser wird mit gesättigter wässriger Natriumhydrogencarbonat-Lösung gewaschen, über Natriumsulfat getrocknet und im Vakuum eingeengt. Nach säulenchromatographischer Aufreinigung erhält man 0,17 g 3-{[3,5-Bis(pentafluorethyl)-1H-pyrazol-1-yl]methyl}N-{4-chlor-3-[(4-fluorphenyl)carbamoyl]phenyl}-1-methyl-4-(trifluormethyl)-1H-pyrazol-5-carboxamid (24% der Theorie).
¹H-NMR (400 MHz, d₆-DMSO): δ = 11.13 (s, 1H), 10.42 (s, 1H), 7.84 (m, 1H), 7.70-7.76 (m, 3H), 7.56 (d, 1H), 7.52 (s, 1H), 7.16 (m, 2H), 5.72 (s, 2H), 3.85 (s, 3H) ppm.
HPLC-MS^{a)}: logP = 4,99, Masse (m/z) = 757 [M+H]⁺.
^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System.
^{b)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 microm; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters.
Mit Hilfe der oben beschriebenen Darstellungsverfahren A bis D wurden die in den Tabellen **1 - 3** aufgeführten Verbindungen dargestellt.

**Tabelle 1**

| | | | | | | | | | | | | |
|---|---|---|---|---|---|---|---|---|---|---|---|---|
| 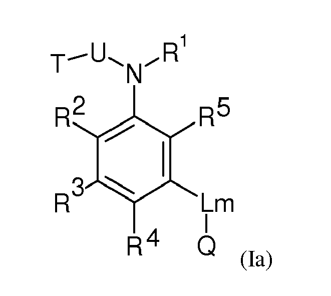 | | | | | | | | | | | | |

| **Bsp.- Nr.** | **T** | **U** | **R¹** | **R²** | **R³** | **R⁴** | **R⁵** | **L** | **m** | **Q** | **log p** | **1H-NMR** |
|---|---|---|---|---|---|---|---|---|---|---|---|---|
| Ia-1 | 1-Methyl-3-(pentafluorethyl)-4-(trifluormethyl)-1H-pyrazol-5-yl | -CO- | H | H | H | Cl | H | -CONH- | 1 | Pyridin-4-yl | 1,99^{b)} | ¹H-NMR (400 MHz, d₆-DMSO): δ = 11.30 (s, 1H), 10.80 (s, 1H), 8.48 (dd, 2H), 7.88 (d, 1H), 7.75(dd,1H), 7.65(dd,2H), 7.60(d, 1H), 4.02 (s, 3H) ppm |
| Ia-2 | 1-Methyl-3-(pentafluorethyl)-4-(trifluormethyl)-1H-pyrazol-5-yl | -CO- | H | H | H | Cl | H | -CONH- | 1 | Pyridin-3-yl | 2,69^{b)} | ¹H-NMR (400 MHz, d₆-DMSO): δ = 11.30 (s, 1H), 10.70 (s, 1H), 8.83 (s, 1H), 8.32 (d, 1H), 8.10 (d, 1H), 7.88 (d, 1H), 7.75 (dd, 1H), 7.63 (d, 1H), 7.40 (dd, 1H), 4.02 (s, 3H) ppm |
| Ia-3 | 2-Methyl-6-(trifluormethyl)pyridin -3-yl | -CO- | H | H | H | Cl | H | -CONH- | 1 | 4-Fluorphenyl | 3,15^{a)} | ¹H-NMR (400 MHz, d₆-DMSO): δ = 10.69 (bs, 1H), 10.41 (bs, 1H), 8.16 (d, 1H), 7.91 (d, 1H), 7.85 (d, 1H), 7.79 (dd, 1H), 7.73-7.70 (m, 2H), 7.54 (d, 1H), 7.19-7.14 (m, 2H), 2.65(s, 3H) ppm |
| Ia-4 | 1-Methyl-3-(pentafluorethyl)-4-(trifluormethyl)-1H-pyrazol-5-yl | -CO- | H | H | H | Cl | H | -CONH- | 1 | 4-Chlorpyridin-3-yl | 3,33^{b)} | ¹H-NMR(400 MHz, d₆-DMSO): δ = 11.30 (s, 1H), 10.75 (s, 1H), 8.90 (d, 1H), 8.68 (d, 1H), 7.95 (d, 1H), 7.88 (d, 1H), 7.75 (dd, 1H), 7.60 (d, 1H), 4.02 (s, 3H) ppm |
| Ia-5 | 2-(Trifluormethyl)-1,3-thiazol-4-yl | -CO- | H | H | H | Cl | H | -CONH- | 1 | 4-Fluorphenyl | 3,35^{a)} | ¹H-NMR (400 MHz, d₆-DMSO): δ = 10.52 (bs, 1H), 10.40 (bs, 1H), 8.84 (s, 1H), 8.05 (d, 1H), 7.97 (dd, 1H), 7.73 (m, 2H), 7.53 (d, 1H), 7.20-7.14 (m, 2H) ppm |
| Ia-6 | 1-Methyl-3-(pentafluorethyl)-4-(trifluormethyl)-1H-pyrazol-5-yl | -CO- | H | H | H | Cl | H | -CONH- | 1 | 2-Chlorpyridin-4-yl | 3,37^{b)} | - |
| Ia-7 | 4-Brom-1-methyl-3-(trifluormethyl)-1H-pyrazol-5-yl | -CO- | H | H | H | Cl | H | -CONH- | 1 | 6-Chlorpyridin-3-yl | 3,42^{a)} | ¹H-NMR (400 MHz, d₆-DMSO): δ = 11.16 (s, 1H), 10.98 (s, 1H), 8.72 (d, 1H), 8.20 (dd, 1H), 7.98 (d, 1H), 7.78 (dd, 1 H), 7.64 (d, 1H), 7.55 (d, 1H), 4.04 (s, 3H) ppm. |
| Ia-8 | 1-Methyl-3-(pentafluorethyl)-4-(trifluormethyl)-1H-pyrazol-5-yl | -CO- | H | H | H | Cl | H | -CONH- | 1 | Pyridin-2-yl | 3,53^{b)} | ¹H-NMR (400 MHz, d₆-DMSO): δ = 11.30 (s, 1H), 10.80 (s, 1H), 8.35 (dd, 1H), 8.13 (d, 1H), 7.85 (m, 2H), 7.73 (dd, 1H), 7.55 (d, 1H), 7.15 (dd, 1H), 4.02 (s, 3H) ppm |
| Ia-9 | 2-Methyl-6-(trifluormethyl)pyridin -3-yl | -CO- | H | H | H | Cl | H | -CONH- | 1 | 3-Chlorphenyl | 3,6^{a)} | ¹H-NMR (400 MHz, d₆-DMSO): δ = 10.70 (bs, 1H), 10.56 (bs, 1H), 8.16 (d, 1H), 7.92 (d, 1H), 7.88 (s, 1H), 7.84 (d, 1H), 7.80 (dd, 1H), 7.59 (d, 1H), 7.56 (d, 1H), 7.37 (t, 1H), 7.18-7.15 (m, 1H), 2.65(s, 3H) ppm |
| Ia-10 | 4-(Difluormethyl)-2-(pentafluorethyl)pyrimi din-5-yl | -CO- | H | H | H | Cl | H | -CONH- | 1 | 4-Fluorphenyl | 3,67^{a)} | ¹H-NMR (400 MHz, d₆-DMSO): δ = 10.99 (bs, 1H), 10.34 (bs, 1H), 9.57 (s, 1H), 7.88 (d, 1H), 7.78-7.70 (m, 3H), 7.58 (d, 1H), 7.34 (t, 1H), 7.18-7.11 (m, 2H) ppm |
| Ia-11 | 1-Methyl-3-(pentafluorethyl)-4-(trifluormethyl)-1H-pyrazol-5-yl | -CO- | H | H | H | Cl | H | -CONH- | 1 | 6-Chlorpyridin-3-yl | 3,68^{b)} | - |
| Ia-12 | 1-Methyl-3-(pentafluorethyl)-4-(trifluormethyl)-1H-pyrazol-5-yl | -CO- | H | H | H | Cl | H | -CONH- | 1 | 4-Methoxyphen yl | 3,68^{b)} | ¹H-NMR (400 MHz, d₆-DMSO): δ = 11.30 (s, 1H), 10.20 (s, 1H), 7.88 (s, 1H), 7.75 (dd, 1H), 7.55 (m, 3H), 6.90 (d, 2H), 4.02 (s, 3H), 3.73 (s, 3H) ppm |
| Ia-13 | 1-Methyl-3-(pentafluorethyl)-4-(trifluormethyl)-1H-pyrazol-5-yl | -CO- | H | H | H | Cl | H | -CONH- | 1 | 2-Chlorpyridin-3-yl | 3,73^{b)} | ¹H-NMR (400 MHz, d₆-DMSO): δ = 11.40 (s, 1H), 10.30 (s, 1H), 8.30 (dd, 1H), 8.15 (d, 1H), 7.88 (d, 1H), 7.75 (dd, 1H), 7.60 (d, 1H), 7.50 (dd, 1H), 4.02 (s, 3H) ppm |
| Ia-14 | 2-(Trifluormethyl)-1,3-thiazol-4-yl | -CO- | H | H | H | Cl | H | -CONH- | 1 | 3-Chlorphenyl | 3,82^{a)} | ¹H-NMR (400 MHz, d₆-DMSO): δ = 10.55 (bs, 1H), 10.53 (bs, 1H), 8.84 (s, 1H), 8.06 (d, 1H), 7.98 (dd, 1H), 7.88 (s, 1H), 7.60 (d, 1H), 7.54 (d, 1H), 7.37 (t, 1H), 7.16 (m, 1H) ppm |
| Ia-15 | 1-Methyl-3-(pentafluorethyl)-4-(trifluormethyl)-1H-pyrazol-5-yl | -CO- | H | H | H | Cl | H | -CONH- | 1 | Phenyl | 3,84^{b)} | ¹H-NMR (400 MHz, d₆-DMSO): δ = 11.40 (s, 1H), 10.30 (s, 1H), 7.82 (d, 1H), 7.75 (dd, 1H), 7.70 (d, 2H), 7.60 (d, 1H), 7.35 (m, 2H), 7.10 (t, 2H), 4.02 (s, 3H) ppm |
| Ia-16 | 1-Methyl-3-(pentafluorethyl)-4-(trifluormethyl)-1H-pyrazol-5-yl | -CO- | H | H | H | Cl | H | -CONH- | 1 | 3-Methoxyphen yl | 3,84^{b)} | ¹H-NMR (400 MHz, d₆-DMSO): δ = 11.30 (s, 1H), 10.40 (s, 1H), 7.82 (d, 1H), 7.75 (dd, 1H), 7.60 (d, 1H), 7.37 (s, 1H), 7.23 (d, 2H), 6.70 (m, 2H), 4.02 (s, 3H), 3.75 (s, 3H) ppm |
| Ia-17 | 1-Methyl-3-(pentafluorethyl)-4-(trifluormethyl)-1H-pyrazol-5-yl | -CO- | H | H | H | Cl | H | -CONH- | 1 | 4-Fluorphenyl | 3,89^{b)} | ¹H-NMR (400 MHz, d₆-DMSO): δ = 11.30 (s, 1H), 10.50 (s, 1H), 7.85 (d, 1H), 7.75 (m, 2H), 7.55 (d, 1H), 7.15 (t, 2H), 4.03 (s, 3H) ppm |
| Ia-18 | 1-Methyl-3-(pentafluorethyl)-4-(trifluormethyl)-1H-pyrazol-5-yl | -CO- | H | H | H | Cl | H | -CONH- | 1 | 2-Fluorphenyl | 3,94^{b)} | ¹H-NMR(400 MHz, d₆-DMSO): δ = 11.30 (s, 1H), 10.20 (s, 1H), 7.88 (d, 1H), 7.75 (m, 2H), 7.60 (d, 1H), 7.25 (m, 3H), 4.02 (s, 3H) ppm |
| Ia-19 | 1-Methyl-3-(pentafluorethyl)-4-(trifluormethyl)-1H-pyrazol-5-yl | -CO- | H | H | H | Cl | H | -CONH- | 1 | 6-Chlorpyridin-2-yl | 3,94^{b)} | - |
| Ia-20 | 2-(Pentafluorethyl)-4-(trifluormethyl)pyrimid in-5-yl | -CO- | H | H | H | Cl | H | -CONH- | 1 | 4-Fluorphenyl | 3,98^{a)} | ¹H-NMR (400 MHz, d₆-DMSO): δ = 11.0 (bs, 1H), 10.44 (bs, 1H), 9.71 (s, 1H), 7.82 (d, 1H), 7.74-7.70 (m, 3H), 7.59 (d, 1H), 7.19 - 7.14 (m, 2H) ppm |
| Ia-21 | 1-Methyl-3-(pentafluorethyl)-4-(trifluormethyl)-1H-pyrazol-5-yl | -CO- | H | H | H | Cl | H | -CONH- | 1 | 3-Fluorphenyl | 4,00^{b)} | - |
| Ia-22 | 1-Methyl-3-(pentafluorethyl)-4-(trifluormethyl)-1H-pyrazol-5-yl | -CO- | H | H | H | Cl | H | -CONH- | 1 | 2,4-Difluorphenyl | 4,05^{b)} | ¹H-NMR (400 MHz, d₆-DMSO): δ = 10.20 (s, 1H), 7.85 (d, 1H), 7.75 (m, 2H), 7.60 (d, 1H), 7.28 (ddd, 1H), 7.10 (ddd, 1H), 4.02 (s, 3H) ppm |
| Ia-23 | 1-Methyl-3-(pentafluorethyl)-4-(trifluormethyl)-1H-pyrazol-5-yl | -CO- | H | H | H | Cl | H | -CONH- | 1 | 4-Fluor-2-methylphenyl | 4,05^{b)} | ¹H-NMR (400 MHz, d₆-DMSO): δ = 11.30 (s, 1H), 10.00 (s, 1H), 7.88 (s, 1H), 7.75 (d, 1H), 7.60 (d, 1H), 7.40 (s, 1H), 7.10 (dd, 1H), 7.02 (m, 1H), 4.02 (s, 3H), 2.30 (s, 3H) ppm |
| Ia-24 | 4-Cyclopropyl-2-(pentafluorethyl)pyrimi din-5-yl | -CO- | H | H | H | Cl | H | -CONH- | 1 | 4-Fluorphenyl | 4,08^{a)} | ¹H-NMR (400 MHz, d₆-DMSO): δ = 10.93 (bs, 1H), 10.41 (bs, 1H), 9.06 (s, 1H), 7.92 (d, 1H), 7.79 (dd, 1H), 7.73-7.69 (m, 2H), 7.56 (d, 1H), 7.17 (t, 2H), 2.5 (m, 1H), 1.29-1.25 (m, 2H), 1.20-1.17 (m, 2H) ppm |
| Ia-25 | 4-(Difluormethyl)-2-(pentafluorethyl)pyrimi din-5-yl | -CO- | H | H | H | Cl | H | -CONH- | 1 | 3-Chlorphenyl | 4,08^{a)} | ¹H-NMR (400 MHz, d₆-DMSO): δ = 11.0 (bs, 1H), 10.59 (bs, 1H), 9.58 (s, 1H), 7.88 (d, 2H), 7.74 (dd, 1H), 7.59 (d, 2H), 7.38 (t, 1H), 7.34 (t, 1H), 7.18 (dd, 1H) ppm |
| Ia-26 | 1-Methyl-3-(pentafluorethyl)-4-(trifluormethyl)-1H-pyrazol-5-yl | -CO- | H | H | H | Cl | H | -CONH- | 1 | 4-Chlorpyridin-2-yl | 4,17^{b)} | - |
| Ia-27 | 1-Methyl-3-(pentafluorethyl)-4-(trifluormethyl)-1H-pyrazol-5-yl | -CO- | H | H | H | Cl | H | -CONH- | 1 | 5-Chlorpyridin-2-yl | 4,17^{b}) | - |
| Ia-28 | 1-Methyl-3-(pentafluorethyl)-4-(trifluormethyl)-1H-pyrazol-5-yl | -CO- | H | H | H | Cl | H | -CONH- | 1 | 4-Chlorphenyl | 4,23^{b)} | - |
| Ia-29 | 1-Methyl-3-(pentafluorethyl)-4-(trifluormethyl)-1H-pyrazol-5-yl | -CO- | H | H | H | Cl | H | -CONH- | 1 | 4-Fluor-3-methylphenyl | 4,23^{b)} | ¹H-NMR (400 MHz, d₆-DMSO): δ = 11.40 (s, 1H), 10.30 (s, 1H), 7.88 (d, 1H), 7.75 (dd, 1H), 7.63 (s, 1H), 7.60 (d, 1H), 7.50 (s, 1H), 7.10 (t, 1H), 4.02 (s, 3H), 2.22 (s, 3H) ppm |
| Ia-30 | 1-Methyl-3-(pentafluorethyl)-4-(trifluormethyl)-1H-pyrazol-5-yl | -CO- | H | H | H | Cl | H | -CONH- | 1 | 3-Chlorphenyl | 4,28^{b)} | ¹H-NMR (400 MHz, d₆-DMSO): δ = 11.30 (s, 1H), 10.60 (s, 1H), 7.90 (s, 1H), 7.85 (s, 1H), 7.75 (dd, 1H), 7.60 (m, 2H), 7.35 (dd, 1H), 7.15 (d, 1H), 4.02 (s, 3H) ppm |
| Ia-31 | 1-Methyl-3-(pentafluorethyl)-4-(trifluormethyl)-1H-pyrazol-5-yl | -CO- | H | H | H | Cl | H | -CONH- | 1 | 2-Chlorphenyl | 4,28^{b)} | ¹H-NMR (400 MHz, d₆-DMSO): δ = 11.40 (s, 1H), 10.00 (s, 1H), 7.90 (d, 1H), 7.75 (dd, 1H), 7.68 (d, 1H), 7.60 (d, 1H), 7.53 (d, 1H), 7.40 (t, 1H), 7.25 (dd, 1H), 4.02 (s, 3H) ppm |
| Ia-32 | 2-(Pentafluorethyl)-4-(trifluormethyl)pyrimid in-5-yl | -CO- | H | H | H | Cl | H | -CONH- | 1 | 3-Chlorphenyl | 4,32^{a)} | ¹H-NMR (400 MHz, d₆-DMSO): δ = 11.02 (bs, 1H), 10.59 (bs, 1H), 9.71 (s, 1H), 7.87 (s, 1H), 7.82 ( d, 1H), 7.74 (dd, 1H), 7.61-7.58 (m, 2H), 7.38 (t, 1H), 7.18-7.15 (m, 1H) ppm |
| Ia-33 | 1-Methyl-3-(pentafluorethyl)-4-(trifluormethyl)-1H-pyrazol-5-yl | -CO- | H | H | H | Cl | H | -CONH- | 1 | 3-Chlor-4-fluor-2-methylphenyl | 4,34^{b)} | ¹H-NMR (400 MHz, d₆-DMSO): δ = 11.30 (s, 1H), 10.10 (s, 1H), 7.88 (s, 1H), 7.75 (dd, 1H), 7.60 (d, 1H), 7.40 (m, 1H), 7.25 (t, 1H), 4.02 (s, 3H), 2.35 (s, 3H) ppm |
| Ia-34 | 2,4-Bis(pentafluorethyl)pyr imidin-5-yl | -CO- | H | H | H | Cl | H | -CONH- | 1 | 4-Fluorphenyl | 4,36^{a)} | ¹H-NMR (400 MHz, d₃-Acetonitril): δ = 9.39 (s, 1H), 9.07 (bs, 1H), 8.75 (bs, 1H), 7.83 (d, 1H), 7.73 (dd, 1H), 7.67 (m, 2H), 7.53 (d, 1H), 7.15- 7.11 (m, 2H) ppm |
| Ia-35 | 4-Brom-1-methyl-3-(pentafluorethyl)-1 H-pyrazol-5-yl | -CO- | H | H | H | Cl | H | -CONH- | 1 | 5-Chlorpyridin-2-yl | 4,38^{a)} | ¹H-NMR (400 MHz, d₃-Acetonitril): δ =4.07 (s, 3H), 7.20 (dd, 1H), 7.50 (d, 1H), 7.78(m, 1H), 7.93(m, 1H), 8.25(dd, 1H), 8.34(m, 1H), 9.01 (bs, 1H), 9.29 (bs, 1H) |
| Ia-36 | 1-Methyl-3-(pentafluorethyl)-4-(trifluormethyl)-1H-pyrazol-5-yl | -CO- | H | H | H | Cl | H | -CONH- | 1 | 4-(Trifluormeth yl)phenyl | 4,40^{b)} | ¹H-NMR (400 MHz, d₆-DMSO): δ = 11.30 (s, 1H), 10.80 (s, 1H), 7.90 (d, 2H), 7.88 (d, 1H), 7.75 (dd, 1H), 7.70 (d, 2H), 7.60 (d, 1H), 4.02 (s, 3H) ppm |
| Ia-37 | 1-Methyl-3-(pentafluorethyl)-4-(trifluormethyl)-1H-pyrazol-5-yl | -CO- | H | H | H | Cl | H | -CONH- | 1 | 3-(Trifluormeth yl)phenyl | 4,40^{b)} | - |
| Ia-38 | 4-Brom-1-methyl-3-(pentafluorethyl)-1H-pyrazol-5-yl | -CO- | H | H | H | Cl | H | -CONH- | 1 | 4-Chlorphenyl | 4,43^{a)} | ¹H-NMR (400 MHz, d₃-Acetonitril): δ =4.04 (s, 3H), 7.34 (m, 1H), 7.50 (d, 1H), 7.78(m, 2H), 7.93(m, 2H), 8.34(m, 1H), 9.06 (bs, 1H), 9.15 (bs, 1H) |
| Ia-39 | 1-Methyl-3-(pentafluorethyl)-4-(trifluormethyl)-1H-pyrazol-5-yl | -CO- | H | H | H | Cl | H | -CONH- | 1 | 4-Fluor-3-(trifluormethy 1)phenyl | 4,51^{b)} | ¹H-NMR (400 MHz, d₆-DMSO): δ = 11.40 (s, 1H), 10.80 (s, 1H), 8.15 (m, 1H), 7.95 (m, 1H), 7.88 (s, 1H), 7.75 (dd, 1H), 7.60 (d, 1H), 7.50 (m, 1H), 4.02 (s, 3H) ppm |
| Ia-40 | 4-Cyclopropyl-2-(pentafluorethyl)pyrimi din-5-yl | -CO- | H | H | H | Cl | H | -CONH- | 1 | 3-Chlorphenyl | 4,54^{a)} | ¹H-NMR (400 MHz, d₆-DMSO): δ = 10.95 (bs, 1H), 10.56 (bs, 1H), 9.06 (s, 1H), 7.93 (d, 1H), 7.87 (s, 1H), 7.80 (dd, 1H), 7.59 (t, 2H), 7.37 (t, 1H), 7.17 (m, 1H), 2.5 (m, 1H), 1.29-1.25 (m, 2H), 1.20-1.16 (m, 2H) ppm |
| Ia-41 | 1-Methyl-3-(pentafluorethyl)-4-(trifluormethyl)-1H-pyrazol-5-yl | -CO- | H | H | H | Cl | H | -CONH- | 1 | 2-Chlor-4-fluor-5-methylphenyl | 4,64^{b)} | ¹H-NMR (400 MHz, d₆-DMSO): δ = 11.30 (s, 1H), 10.10 (s, 1H), 7.90 (s, 1H), 7.75 (d, 1H), 7.60 (d, 1H), 7.55 (d, 1H), 7.40 (d, 1H), 4.02 (s, 3H), 2.23 (s, 3H) ppm |
| Ia-42 | 1-Methyl-3-(pentafluorethyl)-4-(trifluormethyl)-1H-pyrazol-5-yl | -CO- | H | H | H | Cl | H | -CONH- | 1 | 3,4-Dichlorphenyl | 4,65^{b)} | ¹H-NMR (400 MHz, d₆-DMSO): δ = 11.30 (s, 1H, small), 10.70 (s, 1H), 8.05 (s, 1H), 7.85 (d, 1H), 7.75 (dd, 1H), 7.60 (m, 3H), 4.02 (s, 3H) ppm |
| Ia-43 | 3-{[3,5-Bis(pentafluorethyl)-1H-pyrazol-1-yl]methyl} -1-methyl-4-(trifluormethyl)-1H-pyrazol-5-yl | -CO- | H | H | H | Cl | H | -CONH- | 1 | 4-Fluorphenyl | 4,99^{a)} | ¹H-NMR (400 MHz, d₃-Acetonitril): δ = 11.13 (bs, 1H), 10.42 (bs, 1H), 7.84 (m, 1H), 7.69 - 7.75 (m, 3H), 7.56 (d, 1H), 7.52 (s, 1H), 7.14 - 7.19 (m, 2H), 5.72 (s, 2H), 3.85 (s, 3H) ppm |
| Ia-44 | 1-Methyl-3-(pentafluorethyl)-4-(trifluormethyl)-1H-pyrazol-5-yl | -CO- | H | H | H | Cl | H | -CONH- | 1 | 3,4,5-Trichlorpheny 1 | 5,15^{b)} | ¹H-NMR (400 MHz, d₆-DMSO): δ = 11.40 (s, 1H), 10.80 (s, 1H), 7.95 (s, 2H), 7.88 (d, 1H), 7.75 (dd, 1H), 7.60 (d, 1H), 4.02 (s, 3H) ppm |

**Tabelle 2**

| | | | | | | | | | | | |
|---|---|---|---|---|---|---|---|---|---|---|---|
| 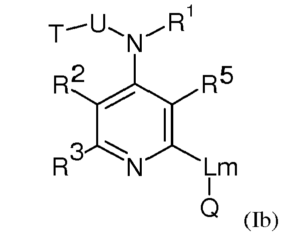 | | | | | | | | | | | |

| **Bsp.-Nr.** | **T** | **U** | | **R²** | **R³** | **R⁵** | **L** | **m** | | **log p** | **1H-NMR** |
|---|---|---|---|---|---|---|---|---|---|---|---|
| Ib-1 | 1-Methyl-3-(pentafluorethyl)-4-(trifluormethyl)-1H-pyrazol-5-yl | -CO- | H | H | H | H | -CONH- | 1 | 4-Fluorphenyl | 4.30^{a)} | ¹H-NMR (400 MHz, CDCl₃,) δ = 9.88 (s, 1H), 8.52 (s, 1H), 8.19 (d, 1H), 8.11 (s, 1H), 7.58 (s, 1H), 7.08 (d, 2H), 7.05 (d, 2H),1.61 (s, 3H) ppm. |

**Tabelle 3**

| | | | | | | | | | | | |
|---|---|---|---|---|---|---|---|---|---|---|---|
| 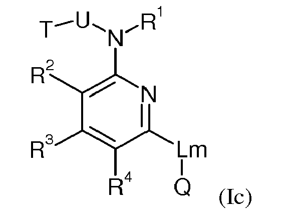 | | | | | | | | | | | |

| **Bsp.-Nr.** | **T** | **U** | | **R² R³** | | **R⁴** | **L** | **m** | **Q** | **log p** | **1H-NMR** |
|---|---|---|---|---|---|---|---|---|---|---|---|
| Ic-1 | 1-Methyl-3-(pentafluorethyl)-4-(trifluormethyl)-1H-pyrazol-5-yl | -CO- | H | H | H | Br | -CONH- | 1 | 4-Fluorphenyl | 4,45^{a)} | ¹H-NMR (400 MHz, d₆-DMSO): δ = 12.0 (s, 1H), 10.5 (s, 1H), 8.29 (d, 1H), 8.15(br.1H), 7.68(dd,2H), 7,17 (dd, 2H), 3.98 (s, 3H) ppm |

| | | | | | | | | | | | |
|---|---|---|---|---|---|---|---|---|---|---|---|
| ^{a)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18).Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min. Die Massendetektion erfolgt über ein Agilend MSD-System. ^{b)} Anmerkung zur Bestimmung der logP-Werte und Massendetektion: Die Bestimmung der angegebenen logP-Werte erfolgte gemäß EEC-Direktive 79/831 Annex V.A8 durch HPLC (High Performance Liquid Chromatography) an einer Phasenumkehrsäule (C18). HP1100; 50*4,6 Zorbax Eclipse Plus C18 1,8 microm; Eluent A: Acetonitril (0,1 % Ameisensäure); Eluent B: Wasser (0,08 % Ameisensäure); linearer Gradient von 5 % Acetonitril bis 95 % Acetonitril in 1,70 min, dann 95 % Acetonitril für weitere 1,00 min; Ofentemperatur 55°C; Fluß:2,0 mL/min. Die Massendetektion erfolgt über den Massendetektor Micromass ZQ2000 der Firma Waters. | | | | | | | | | | | |

### Herstellung der Ausgangsverbindungen

### 1-Methyl-3-pentafluorethyl-1H-pyrazol

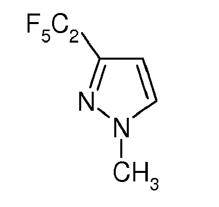

Zu einer Lösung von 30,90 g (141,67 mmol) (E)-5-Ethoxy-1,1,1,2,2-pentafluoropent-4-en-3-one (Herstellung : Synthesis 2000, 5, 738-42) in 56 ml Methanol werden 7.18 g (155.83 mmol) Methylhydrazin getropft und das Reaktionsgemisch 18 Stunden lang unter Rückfluß erhitzt. Das Methanol wird zum Großteil bei Normaldruck abdestillert abdestilliert und der Rückstand auf Eis gegeben. Die wässrige Phase wird dreimal mit Dichlormethan extrahiert und die organische Phase anschließend dreimal mit gesättigter Kochsalzlösung gewaschen. Nach Trocknen über Natriumsulfat wird das Lösungsmittel am Rotationsverdampfer bei vermindertem Druck abdestilliert. Man erhält 15,81 g (51,82 % der Theorie) 1-Methyl-3-pentafluorethyl-1*H*-pyrazol als Öl.
¹H-NMR (400 MHz, d₃-Acetonitril) δ = 3.89 (s, 3H, CH₃), 6.57 (m, 1H, CH), 7.61(m, 1H, CH) ppm;
HPLC-MS: logP = 2,29; Masse (m/z): 201 (M+H)⁺.

### 4-Brom-1-Methyl-3-pentafluorethyl-1H-pyrazol

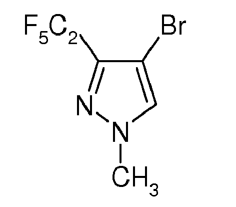

Zu einer Lösung von 4,20 g (20,90 mmol) 1-Methyl-3-pentafluorethyl-1H-pyrazol in 18 ml Wasser werden bei 40°C 3,27 g (20,45 mmol) Brom getropft und das Reaktionsgemisch zunächst 1Stunde bei 60°C und dann 18 Stunden bei Raumtemperatur nachgerührt. Die wässrige Phase wird dreimal mit Dichlormethan extrahiert und die organische Phase über Natriumsulfat getrocknet. Das Dichlormethan wird am Rotationsverdampfer bei vermindertem Druck abdestilliert. Man erhält 5,42 g (70,90 % der Theorie) 4-Brom-1-Methyl-3-pentafluorethyl-1H-pyrazol als Öl.
¹H-NMR (400 MHz, d₃-Acetonitril) δ = 3.90 (s, 3H, CH₃), 7.77(m, 1H, CH) ppm;
HPLC-MS^{a)}: logP = 2,99; Masse (m/z): 280 (M+H)⁺.

### 4-Brom-1-Methyl-3-pentafluoroethyl-1H-pyrazol-5-carbonsäure

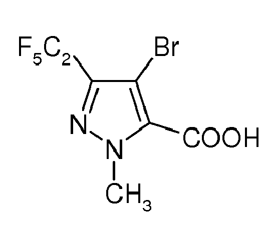

Unter Argonatmosphäre werden 2,70 g (9,67 mmol) 4-Brom-1-Methyl-3-pentafluorethyl-1H-pyrazolin Diethylether vorgelegt und die Lösung auf -78°C gekühlt. Man tropft 5,32 ml (10,60 mmol) 2M Lithiumdiisopropylamidlösung in THF/Heptan zu und gibt bei -30°C unter starkem Rühren 250 g zerstoßenes Trockeneis zu. Nach Beendigung der Gasentwicklung versetzt man das Reaktionsgemisch mit 235 ml Wasser und stellt mit 1N Natronlauge pH 11 ein. Die alkalische Lösung wird dreimal mit Ethylacetat extrahiert und daraufhin mit 1N Salzsäure auf pH 2 gestellt. Die wässrige Phase wird dreimal mit Ethylacetat extrahiert und die organische Phase über Natriumsulfat getrocknet. Nach Abdestillieren des Lösungsmittels am Rotationsverdampfer unter vermindertem Druck erhält man 1,00 g (32,15 % der Theorie) 14-Brom-1-Methyl-3-pentafluoroethyl-1H-pyrazol-5-carbonsäure als Feststoff.

### Ethyl-5-({[4-brom-1-methyl-3-(pentafluorethyl)-1H-pyrazol-5-yl]carbonyl}amino)-2-chlorbenzoat

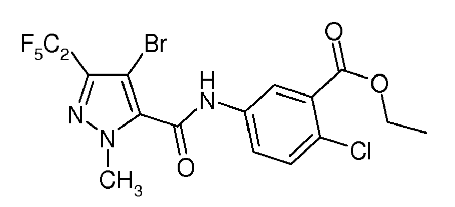

2.088 g (6,46 mmol) 4-Brom-1-Methyl-3-pentafluoroethyl-1H-pyrazol-5-carbonsäure und 1,49 g (7,75 mmol) 1-Ethyl-3-[3-dimethylaminopropyl]carbodiimid Hydrochlorid werden in 30 ml Dioxan vorgelegt und nach 30 Minuten Rühren mit einer Lösung von 1,29 g (6,46 mmol) Ethyl-5-amino-2-chlorbenzolcarboxylat in 20 ml Dioxan versetzt. Nach 18 Stunden Rühren bei Raumtemperatur wird das Dioxan am Rotationsverdampfer bei vermindertem Druck abdestilliert und das Reaktionsgemisch mit 50 ml Wasser versetzt. Die wässrige Phase wird dreimal mit Ethylacetat extrahiert und die organische Phase über Natriumsulfat getrocknet. Nach Abdestillieren des Lösungsmittels am Rotationsverdampfer unter vermindertem Druck wird der Rückstand mittels Flashchromatographie an Kieselgel gereinigt (Eluent: Cyclohexan/Essigester; Gradient: 2 Stunden, von 0% auf 100 % Essigester). Man erhält 0,629 g (16 % der Theorie) Ethyl-5-({[4-brom-1-methyl-3-(pentafluorethyl)-1H-pyrazol-5-yl]carbonyl} amino)-2-chlorbenzoat.
¹H-NMR (400 MHz, d₃-Acetonitril) δ = 1.36 (t, 3H), 4.10 (s, 3H), 4.40 (q, 2H), 7.52 (m, 1H), 7.60 (m, 1H), 8.10 (s, 1H), 8.87 (br. s, 1H) ppm;
HPEC-MS^{a)}: logP = 4,48; Masse (m/z): 505,8 (M+H)⁺.

### 5-({[4-Brom-1-methyl-3-(pentafluorethyl)-1H-pyrazol-5-yl]carbonyl}amino)-2-chlorbenzoesäure

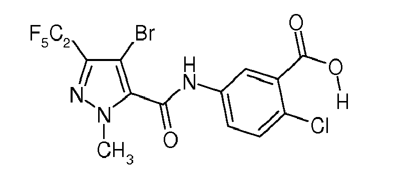

Zu einer Lösung von 0,551 g (1,09 mmol) Ethyl-5-({[4-brom-1-methyl-3-(pentafluorethyl)-1H-pyrazol-5-yl]carbonyl}amino)-2-chlorbenzoat in 6 ml Ethanol wird eine Lösung von 0,105 g (4,36 mmol) Lithiumhydroxid in 1,50 ml Wasser getropft und die Lösung 18 Stunden bei Raumtemperatur gerührt. Das Ethanol wird am Rotationsverdampfer bei vermindertem Druck abdestilliert und die Lösung mit halbkonzentrierter Salsäure auf pH1 gestellt. Die wässrige Phase wird dreimal mit Ethylacetat extrahiert und die organische Phase über Natriumsulfat getrocknet. Nach Abdestillieren des Lösungsmittels am Rotationsverdampfer unter vermindertem Druck erhält man 0,540 g (85,5 % der Theorie) 5-({[4-Brom-1-methyl-3-(pentafluorethyl)-1H-pyrazol-5-yl]carbonyl}amino)-2-chlorbenzoesäure.
¹H-NMR (400 MHz, d₃-Acetonitril) δ = 4.06 (s, 3H), 7.53 (m. 1H), 7.80 (m, 1H), 8.17 (s, 1H), 9.05 (br. s, 1H) ppm;
HPLC-MS^{a)}: logP = 3,13; Masse (m/z): 475,8 (M+H)⁺.

### Ethyl-2-(pentafluorethyl)-4-(trifluormethyl)pyrimidin-5-carboxylat

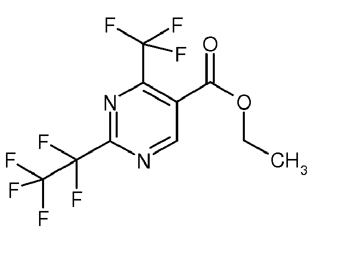

Eine Mischung von 8,1 g (50 mmol) 2,2,3,3,3-Pentafluorpropanimidamid (käuflich) und 12,0 g (50 mmol) Ethyl-2-(ethoxymethyliden)-4,4,4-trifluor-3-oxobutanoat (kommerziell verfügbar) in 70 ml absolutem Ethanol wird nach Zugabe von 3,4 g (50 mmol) Natriumethylat 3 Tage unter Rückfluss gerührt. Anschließend wird im Vakuum eingeengt und der Rückstand in 50 ml Wasser aufgenommen und zweimal mit 50 ml Essigsäureethylester extrahiert. Die organischen Phasen werden successiv mit 20 ml Wasser und 20 ml gesättigter Natriumchloridlösung gewaschen, über Natriumsulfat getrocknet, filtriert und im Vakuum eingeengt. Nach chromatographischer Reinigung mit einer Mischung von Cyclohexan und Essigsäureethylester (5:1) erhält man 9,546 g (28,23 mM, 56,5 % der Theorie) Ethyl-2-(pentafluorethyl)-4-(trifluormethyl)pyrimidin-5-carboxylat als weißen Feststoff..
¹H-NMR (400 MHz, d₆-DMSO): δ = 9.66 (s, 1H), 4.45 (q, 2H), 1.36 (t, 3H) ppm.
HPLC-MS^{a)}: logP = 3,86 Masse (m/z) = 339 [M+H]⁺.

### 2-(Pentafluorethyl)-4-(trifluormethyl)pyrimidin-5-carbonsäure

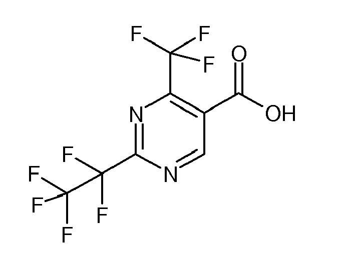

In 5 ml absolutem Ethanol werden 4,470 g (13,2 mM) Ethyl-2-(pentafluorethyl)-4-(trifluormethyl)pyrimidin-5-carboxylat gelöst. Es werden 19,8 ml (39,66 mM) 2N Natronlauge zugegeben und das Reaktionsgemisch wird 4 h bei Raumtemperatur gerührt. Es wird durch Zugabe von 2N Salzsäure auf pH 2-3 gestellt. Der entstandene Feststoff wird abgesaugt, mit wenig Wasser gewaschen und mit Cyclohexan verrieben. Man erhält 3,741 g (12,07 mM, 91,1% der Theorie) 2-(Pentafluorethyl)-4-(trifluormethyl)pyrimidin-5-carbonsäure als weißen Feststoff.
¹H-NMR (400 MHz, d₆-DMSO): δ = 9.40 (s, 1H) ppm.
HPLC-MS^{a)}: logP = 1,80 Masse (m/z) = 311 [M+H]⁺.

### 3-Brom-6-({[1-methyl-3-(pentafluorethyl)-4-(trifluormethyl)-1H-pyrazol-5-yl]carbonyl}-amino)pyridin-2-carbonsäuremethylester

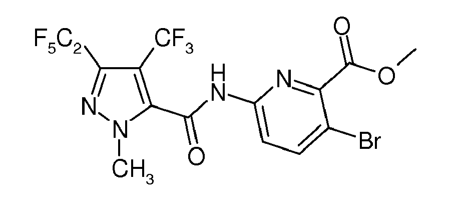

Zu einer Lösung aus 369,0 mg (2,0 eq, 1,59 mM) 6-Amino-3-brom-pyridin-2-carbonsäuremethylester (vgl. WO 2008/084717) in 13,4 ml Dichlormethan werden 528,0 mg (2,0 eq., 1.59 mM) 1-Methyl-3-(pentafluorethyl)-4-(trifluormethyl)-1*H*-pyrazol-5-carbonsäurechlorid und eine Suspension aus 213,8 mg Silber(I)-cyanid in 13,4 ml Acetonitril gegeben. Anschließend wird das Reaktionsgemisch 24 Stunden bei Raumtemperatur gerührt. Danach wird die Reaktionsmischung filtriert und das Lösungsmittelgemisch im Vakuum abgezogen. Der verbleibende Rückstand wird mittels Säulenchromatographie an Kieselgel (Kieselgel 60 - Merck, Korngröße: 0,04 bis 0,063 mm) mit dem Laufmittelgemisch Cyclohexan:Aceton (Gradient) gereinigt.

Man erhält 301,7 mg (34,1 % der Theorie) 3-Brom-6-({[1-methyl-3-(pentafluorethyl)-4-(trifluormethyl)-1H-pyrazol-5-yl]carbonyl}-amino)pyridin-2-carbonsäuremethylester.
LC-MS^{a)}: logP = 4,06, Masse (m/z) = 526.9 [M+H]⁺

### 3-Brom-6-({[1-methyl-3-(pentafluorethyl)-4-(trifluormethyl)-1H-pyrazol-5-yl]carbonyl}-amino)pyridin-2-carbonsäure

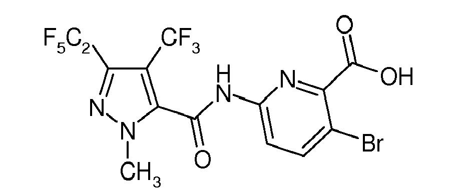

425,1 mg (1,0 eq, 0,80 mM) 3-Brom-6-({[1-methyl-3-(pentafluorethyl)-4-(trifluormethyl)-1*H-*pyrazol-5-yl]carbonyl}-amino)pyridin-2-carbonsäuremethylester werden in 13,6 mL Methanol p.A. suspendiert und dann mit 0,60 mL 2N Natronlauge versetzt. Das Reaktionsgemisch wird 4 Stunden bei 50 °C gerührt. Danach wird der Reaktionsansatz in Essigsäureethylester aufgenommen und einmal gegen 1N Salzsäure geschüttelt. Die organische Phase wird abgetrennt, getrocknet und im Vakuum eingeengt.

Man erhält 360,2 mg (65,3 % der Theorie; Reinheit: 75 %ig) 3-Brom-6-({[1-methyl-3-(pentafluorethyl)-4-(trifluormethyl)-1H-pyrazol-5-yl]carbonyl}-amino)pyridin-2-carbonsäure, die ohne weitere Reinigung für Folgereaktionen verwendet werden kann..
LC-MS^{a)}: logP = 2,78, Masse (m/z) = 512.9 [M+H]⁺.

### 4-({[1-methyl-3-(pentafluorethyl)-4-(trifluormethyl)-1H-pyrazol-5-yl]carbonyl}-amino)pyridin-2-carbonsäuremethylester

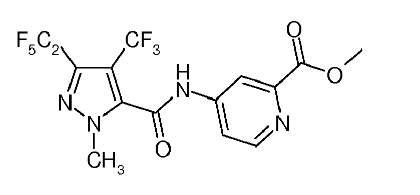

Zu einer Lösung aus 486,0 mg (2,0 eq, 3,19 mM) 4-Amino-pyridin-2-carbonsäuremethylester (vgl. WO 2001/074788) in 26,8 ml Dichlormethan werden 1056,0 mg (2,0 eq., 3,19 mM) 1-Methyl-3-(pentafluorethyl)-4-(trifluormethyl)-1*H*-pyrazol-5-carbonsäurechlorid und eine Suspension aus 427,7 mg Silber(I)-cyanid in 26,8 ml Acetonitril gegeben. Anschließend wird das Reaktionsgemisch 24 Stunden bei Raumtemperatur gerührt. Danach wird die Reaktionsmischung filtriert und das Lösungsmittelgemisch im Vakuum abgezogen. Der verbleibende Rückstand wird mittels Säulenchromatographie an Kieselgel (Kieselgel 60 - Merck, Korngröße: 0,04 bis 0,063 mm) mit dem Laufmittelgemisch Cyclohexan:Aceton (Gradient) gereinigt.

Man erhält 984,3 mg (69,0 % der Theorie) 4-({[1-methyl-3-(pentafluorethyl)-4-(trifluormethyl)-1H-pyrazol-5-yl]carbonyl}-amino)pyridin-2-carbonsäuremethylester.
¹H-NMR (400 MHz, d₆-DMSO): δ = 8,67 (d, 1H), 8,31 (s, 1H), 7,80 (d, 1H), 4,03 (s, 3H), 3.90 (s, 3H) ppm.
LC-MS^{a)}: logP = 3,01, Masse (m/z) = 447.1 [M+H]⁺

### 4-({[1-methyl-3-(pentafluorethyl)-4-(trifluormethyl)-1H-pyrazol-5-yl]carbonyl}-amino)pyridin-2-carbonsäure

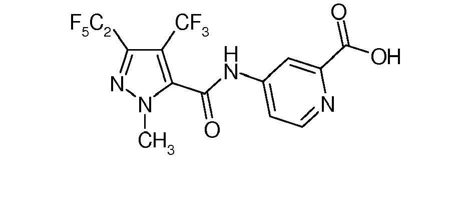

900,0 mg (1,0 eq, 2,01 mM) 4-({[1-methyl-3-(pentafluorethyl)-4-(trifluormethyl)-1*H*-pyrazol-5-yl]carbonyl}-amino)pyridin-2-carbonsäuremethylester werden in 25,0 mL Methanol p.A. suspendiert und dann mit 1,51 mL 2N Natronlauge versetzt. Das Reaktionsgemisch wird 10 Stunden bei 50 °C gerührt. Danach wird der Reaktionsansatz in Essigsäureethylester aufgenommen und einmal gegen 1N Salzsäure geschüttelt. Die organische Phase wird abgetrennt, getrocknet und im Vakuum eingeengt. Der verbleibende Rückstand wird mittels Säulenchromatographie an Kieselgel (Kieselgel 60 - Merck, Korngröße: 0,04 bis 0,063 mm) mit dem Laufmittelgemisch Cyclohexan:Aceton (Gradient; von 100% Aceton auf 100 % Methanol gewechselt) gereinigt.

Man erhält 806,5 mg (92,5 % der Theorie) 4-({[1-methyl-3-(pentafluorethyl)-4-(trifluormethyl)-1*H*-pyrazol-5-yl]carbonyl}-amino)pyridin-2-carbonsäure, die für Folgereaktionen verwendet werden kann.
LC-MS^{a)}: logP = 2,06, Masse (m/z) = 433.1 [M+H]⁺.

### Biologische Beispiele

### Beispiel A

### Ctenocephalides felis oral (CTECFE)

### Lösungsmittel: 1 Gewichtsteil Dimethylsulfoxid

Zwecks Herstellung einer zweckmäßigen Wirkstoffzubereitung vermischt man 10 mg Wirkstoff mit 0,5 ml Dimethylsulfoxid. Ein Teil des Konzentrats wird mit citriertem Rinderblut verdünnt und die gewünschte Konzentration hergestellt.

20 nüchterne adulte Flöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Nach 2 Tagen wird die Abtötung in % bestimmt. Dabei bedeutet 100 %, dass alle Flöhe abgetötet wurden; 0 % bedeutet, dass kein Floh abgetötet wurde.

Bei diesem Test zeigen z.B. die folgenden Verbindungen der Herstellungsbeispiele eine Wirkung von 80 % bei einer Aufwandmenge von 100 ppm :
Ia-11, Ia-26, Ia-28
   Bei diesem Test zeigen z.B. die folgenden Verbindungen der Herstellungsbeispiele eine Wirkung von 90 % bei einer Aufwandmenge von 100 ppm :
Ia-17, Ia-29, Ia-30
   Bei diesem Test zeigen z.B. die folgenden Verbindungen der Herstellungsbeispiele eine Wirkung von 95 % bei einer Aufwandmenge von 100 ppm :
Ia-16, Ia-21, Ia-31
   Bei diesem Test zeigen z.B. die folgenden Verbindungen der Herstellungsbeispiele eine Wirkung von 100 % bei einer Aufwandmenge von 100 ppm :
Ia-2, Ia-8, Ia-15, Ia-18, Ia-22

### Beispiel B

### Lucilia cuprina-Test (LUCICU)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.Gefäße, die Pferdefleisch enthalten, das mit der Wirkstoffzubereitung der gewünschten Konzentration behandelt wurde, werden mit *Lucilia cuprina* Larven besetzt.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 90 % bei einer Aufwandmenge von 100 ppm :
Ia-16, Ia-36
   Bei diesem Test zeigen z.B. die folgenden Verbindungen der Herstellungsbeispiele eine Wirkung von 100 % bei einer Aufwandmenge von 100 ppm :
Ia-8, Ia-11, Ia-15, Ia-17, Ia-18, Ia-21, Ia-22, Ia-26, Ia-28, Ia-29, Ia-30

### Beispiel C

### Musca domestica-Test (MUSCDO)

### Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration. Gefäße, die einen Schwamm enthalten, der mit der Wirkstoffzubereitung der gewünschten Konzentration behandelt wurde, werden mit *Musca domestica Adulten* besetzt.

Nach 2 Tagen wird die Abtötung in % bestimmt. Dabei bedeutet 100 %, dass alle Fliegen abgetötet wurden; 0 % bedeutet, dass keine Fliegen abgetötet wurden.

Bei diesem Test zeigen z.B. die folgenden Verbindungen der Herstellungsbeispiele eine Wirkung von 80 % bei einer Aufwandmenge von 100 ppm :
Ia-26
   Bei diesem Test zeigen z.B. die folgenden Verbindungen der Herstellungsbeispiele eine Wirkung von 90 % bei einer Aufwandmenge von 100 ppm :
Ia-22
   Bei diesem Test zeigen z.B. die folgenden Verbindungen der Herstellungsbeispiele eine Wirkung von 100 % bei einer Aufwandmenge von 100 ppm :
Ia-17

### Beispiel D

### Boophilus microplus -Test (BOOPMI Injektion)

### Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Wasser auf die gewünschte Konzentration. Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier. Nach 7 Tagen wird die Wirkung in % bestimmt. Dabei bedeutet 100%, dass keine Zecke fertile Eier gelegt hat.

Bei diesem Test zeigen z.B. die folgenden Verbindungen der Herstellungsbeispiele eine Wirkung von 100 % bei einer Aufwandmenge von 20 µg / Tier :
Ia-2, Ia-8, Ia-11, Ia-15, Ia-16, Ia-17, Ia-18, Ia-21, Ia-22, Ia-26, Ia-28, Ia-29, Ia-30, Ia-36

### Beispiel E

### Phaedon-Test (PHAECO Spritzbehandlung)

Lösungsmittel: 78,0 GewichtsteileAceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z.B. die folgenden Verbindungen der Herstellungsbeispiele eine Wirkung von 80 % bei einer Aufwandmenge von 500 g/ha:
Ia-38,Ia-9
   Bei diesem Test zeigen z.B. die folgenden Verbindungen der Herstellungsbeispiele eine Wirkung von 100 % bei einer Aufwandmenge von 500 g/ha :
Ia-1, Ia-2, Ia-3, Ia-4, Ia-6, Ia-8, Ia-10, Ia-11, Ia-13, Ia-15, Ia-16, Ia-17, Ia-18, Ia-19, Ia-20,
Ia-21,Ia-22, Ia-23, Ia-24, Ia-25, Ia-26, Ia-27, Ia-28, Ia-29, Ia-30, Ia-31, Ia-32, Ia-33, Ia-34,
Ia-35, Ia-36, Ia-37, Ia-39, Ia-41, Ia-42, Ia-43, Ia-44, Ic-1

Bei diesem Test zeigen z.B. die folgenden Verbindungen der Herstellungsbeispiele eine Wirkung von 100 % bei einer Aufwandmenge von 100 g/ha :
Ia-7

### Beispiel F

### Spodoptera frugiperda-Test (SPODFR Spritzbehandlung)

Lösungsmittel: 78,0 GewichtsteileAceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays)* werden mit einer Wirkstoffzubereitung der gewünschten Konzentration gespritzt und nach dem Abtrocknen mit Raupen des Heerwurms (*Spodoptera frugiperda*) besetzt.Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z.B. die folgenden Verbindungen der Herstellungsbeispiele eine Wirkung von 80 % bei einer Aufwandmenge von 500 g/ha :
Ia-4, Ia-35
   Bei diesem Test zeigen z.B. die folgenden Verbindungen der Herstellungsbeispiele eine Wirkung von 100 % bei einer Aufwandmenge von 500 g/ha :
Ia-2, Ia-3, Ia-6, Ia-8, Ia-11, Ia-12, Ia-13, Ia-15, Ia-16, Ia-17, Ia-18, Ia-19, Ia-20, Ia-21, Ia-22, Ia-23, Ia-24, Ia-25, Ia-26, Ia-27, Ia-28, Ia-29, Ia-30, Ia-31, Ia-32, Ia-33, Ia-34, Ia-36, Ia-37, Ia-39, Ia-40, Ia-42, Ia-43, Ia-44, Ic-1

### Beispiel G

### Tetranychus-Test, OP-resistent (TETRUR Spritzbehandlung)

Lösungsmittel: 78,0 GewichtsteileAceton
   1,5 Gewichtsteile Dimethylformamid
Emulgator: 0,5 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z.B. die folgenden Verbindungen der Herstellungsbeispiele eine Wirkung von 80 % bei einer Aufwandmenge von 500 g/ha :
Ia-6, Ia-8, Ia-16, Ia-17, Ia-21, Ia-28, Ia-34
   Bei diesem Test zeigen z.B. die folgenden Verbindungen der Herstellungsbeispiele eine Wirkung von 90 % bei einer Aufwandmenge von 500 g/ha :
Ia-2, Ia-11, Ia-13, Ia-15, Ia-23, Ia-26, Ia-30, Ia-33
   Bei diesem Test zeigen z.B. die folgenden Verbindungen der Herstellungsbeispiele eine Wirkung von 100 % bei einer Aufwandmenge von 500 g/ha:
Ia-18, Ia-19, Ia-22
   Bei diesem Test zeigen z.B. die folgenden Verbindungen der Herstellungsbeispiele eine Wirkung von 80 % bei einer Aufwandmenge von 100 g/ha :
Ia-7

## Patentansprüche

1. Verbindungen der allgemeinen Formel (I), 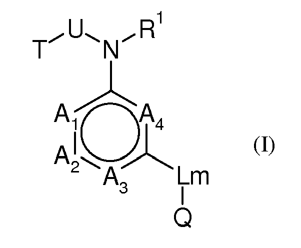 in denen
R¹ für Wasserstoff, gegebenenfalls substituiertes C₁-C₆-Alkyl, C₁-C₆-Halogenalkyl, C₂-C₆-Alkylen, C₂-C₆-Alkinyl, C₃-C₇-Cycloalkyl, (C₁-C₆-Alkyl)carbonyl, (C₁-C₆- Alkoxy)carbonyl, (C₁-C₂-Alkoxy)-(C₁-C₂-alkyl), Cyan-(C₁-C₂-alkyl), Aryl-(C₁-C₃- alkyl), Heteroaryl-(C₁-C₃-alkyl) steht,
die chemischen Gruppierungen
A₁ für CR², einen gegebenenfalls mit G substituierten Stickstoff, Sauerstoff, Schwefel oder eine Bindung,
A₂ für CR³ oder einen gegebenenfalls mit G substituierten Stickstoff, Sauerstoff oder Schwefel,
A₃ für CR⁴ oder einen gegebenenfalls mit G substituierten Stickstoff, Sauerstoff oder Schwefel und
A₄ für CR⁵ oder einen gegebenenfalls mit G substituierten Stickstoff, Sauerstoff oder Schwefel stehen,
unter der Maßgabe, dass nicht mehr als drei der chemischen Gruppierungen A₁ bis A₄ gleichzeitig für Stickstoff stehen;
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆-Halogenalkylsulfonyl, *N*-(C₁-C₆-Alkyl)amino, *N*,*N*-Di-(C₁-C₆-alkyl)amino, *N*-(C₁-C₆-Alkyl)aminocarbonyl, *N*-(C₃-C₇- Cycloalkyl)amino-carbonyl oder (C₁-C₄-Alkoxy)carbonyl, stehen;
wenn keine der Gruppierungen A₂ und A₃ für Stickstoff steht, können R³ und R⁴ gemeinsam mit dem Kohlenstoff an den sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält und gegebenenfalls substituiert ist, oder
wenn keine der Gruppierungen A₁ und A₂ für Stickstoff steht, können R² und R³ gemeinsam mit dem Kohlenstoff an den sie gebunden sind einen 6-gliedrigen Ring bilden, der 0, 1 oder 2 Stickstoffatome enthält und gegebenenfalls substituiert ist;
G für Wasserstoff, gegebenenfalls substituiertes C₁-C₆-Alkyl, C₁-C₄-Alkenyl, C₁-C₄- Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
U für eine Gruppierung C(=W), SO oder SO₂ steht,
wobei
W für Sauerstoff oder Schwefel steht;
L für eine bivalente chemische Gruppierung steht, die ausgewählt ist aus den Gruppierungen, -CH₂NHC(=W)-, -CH₂NR⁶C(=W)-, -C(=W)NH-, -C(=W)NR⁶, -C(=W)NHCH₂-, -C(=W)NR⁶CH₂-, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=NH)-, -SO(=N-CN)- und -S(=N-CN)-, -C(=W)NHSO₂-, bei der die rechte Seite
der Gruppierung direkt an den Rest Q anknüpft und wobei,
p die Werte 0, 1 oder 2 annehmen kann;
R⁶ für Wasserstoff, gegebenenfalls substituiertes C₁-C₆-Alkyl, C₁-C₆-Halogenalkyl, Aryl-(C₁-C₃-alkyl), Heteroaryl-(C₁-C₃-alkyl), C₂-C₆-Alkenyl, C₂-C₆-Alkinyl, C₃- C₆-Cycloalkyl, (C₁-C₂-Alkyl)-(C₃-C₅-cyloalkyl), (C₃-C₅-Cycloalkyl)-(C₁-C₂- alkyl), (C₁-C₆-Alkyl)carbonyl und (C₁-C₆-Alkoxy)carbonyl steht;
m die Werte 0 oder 1 annehmen kann;
Q für einen gegebenenfalls mehrfach mit V substituierten gesättigten oder ungesättigten 5- oder 6-gliedrigen Ring steht, oder für einen gegebenenfalls mehrfach substituierten 5- oder 6-gliedrigen, heterozyklischen Ring steht, wobei V für Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino steht
die chemische Gruppierung T für einen gegebenenfalls. mehrfach mit Z substituierten gesättigten oder ungesättigten 5- oder 6-gliedrigen Ring steht, oder für einen gegebenenfalls mehrfach mit Z substituierten 5- oder 6-gliedrigen, heterozyklischen Ring steht;
Z für Wasserstoff, Halogen, Cyano, Nitro, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino, -C(=W)NR⁷R⁶, -C(=W)OR⁸, -S(O)₂NR⁹R¹⁰, -S(O)ₚR¹¹, -S(O)(=NR¹²)R¹³, (C₃-C₆- Cycloalkyl)carbonyl, (C₃-C₆-Cycloalkyl)-(C₁-C₃-alkyl), Aryl-(C₁-C₃-alkyl), Heteroaryl-(C₁-C₃-alkyl) und gegebenenfalls mit R¹⁴ substituiertes Phenyl und Pyridinyl steht;
R⁷ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
R⁸ ausgewählt ist aus Wasserstoff oder den gegebenenfalls mit R⁶ substituierten Gruppierung C₁-C₆-Alkyl, C₁-C₆-Halogenalkyl, C₂-C₆-Alkenyl, C₂-C₆- Halogenalkenyl, C₂-C₆-Alkinyl, C₃-C₆-Cycloalkyl, C₃-C₆-Halogencycloalkyl, (C₁- C₂-Alkyl)-(C₃-C₆-cycloalkyl) und (C₃C₆-Cycloalkyl)-(C₁-C₂Alkyl);
R⁹ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆-Cycloalkyl)-(C₁-C₂- Alkyl), (C₁-C₆-Alkyl)carbonyl und (C₁-C₆-Alkoxy)carbonyl;
R¹⁰ ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂Alkyl)-(C₃-C₆-cycloalkyl) und (C₃-C₆-Cycloalkyl)-(C₁-C₂Alkyl);
R¹¹ ausgewählt ist aus den gegebenenfalls mit R¹⁶ substituierten Gruppierungen C₁-C₆- Alkyl, C₂-C₆-Alkenyl, C₂-C₆-Alkinyl, C₃-C₆-Cycloalkyl, (C₁-C₂-Alkyl)-(C₃-C₆- cycloalkyl) und (C₃-C₆-Cycloalkyl)-(C₁-C₂-alkyl), C₁-C₄-Haloalkyl;
R¹² ausgewählt ist aus Wasserstoff, C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆- Cycloalkyl)-(C₁-C₂-alkyl), (C₁-C₆-Alkyl)carbonyl und (C₁-C₆-Alkoxy)carbonyl;
R¹³ ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂Alkyl)-(C₃C₆-cycloalkyl) und (C₃C₆-Cycloalkyl)-(C₁-C₂alkyl);
R¹⁴ ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-C₁-C₆-Alkylamino, *N*,*N*-Di-(C₁- C₆)alkylamino, (C₁-C₆-Alkyl)carbonyl, (C₁-C₆-Alkoxy)carbonyl, *N*-(C₁-C₆- Alkyl)aminocarbonyl und *N*,*N*-Di-(C₁-C₆-alkyl)aminocarbonyl;
R¹⁵ ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂ sowie gegebenenfalls mit R¹⁶ substituiertem Phenyl oder Pyridyl;
R¹⁶ ausgewählt ist aus Halogen, -CN, -NO₂, C₁-C₆-Alkyl, C₁-C₆-Alkoxy, C₁-C₆- Alkylthio, C₁-C₆-Alkylsulfinyl, C₁-C₆-Alkylsulfonyl, (C₁-C₆-Alkyl)carbonyl, (C₁- C₆-Alkoxy)carbonyl, *N*-(C₁-C₆-Alkyl)aminocarbonyl, oder gegebenenfalls mit R¹⁸ substituiertem Phenyl oder Pyridyl;
R¹⁷ ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆- Halogenalkylsulfonyl, *N*-C₁-C₆-Alkylamino, *N*,*N*-Di-(C₁-C₆)alkylamino, (C₁-C₃- Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
R¹⁸ ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-C₁-C₆-Alkylamino, *N*,*N*-Di-(C₁- C₆)alkylamino, (C₁-C₃-Alkyl)carbonyl, (C₁-C₃-Alkoxy)carbonyl, *N*-(C₁-C₆- Alkyl)aminocarbonyl, *N*,*N*-Di-(C₁-C₆-alkyl)aminocarbonyl; oder
L, Q und R⁴ und zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

2. Verbindungen der allgemeinen Formel (I) gemäß Anspruch 1, in welcher
R¹ für Wasserstoff, gegebenenfalls substituiertes C₁-C₆-Alkyl, C₂-C₆-Alkylen, C₂-C₆-Alkinyl, C₃-C₇-Cycloalkyl, (C₁-C₆-Alkyl)carbonyl, (C₁-C₆-Alkoxy)carbonyl, (C₁-C₂-Alkoxy)-(C₁- C₂-alkyl), Cyan-(C₁-C₂-alkyl), Aryl(C₁-C₃-alkyl), Heteroaryl(C₁-C₃-alkyl) steht,
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆- Cycloalkyl, C₃-C₆-Halogencycloalkyl, C₁-C₆-Alkoxy, C₁-C₆-Halogenalkoxy, C₁- C₆-Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N*-(C₁- C₆-Alkyl)amino, *N*,*N*-Di-(C₁-C₆-alkyl)amino, *N*-(C₁-C₆-Alkyl)aminocarbonyl, *N*-(C₃-C₆-Cycloalkyl)aminocarbonyl oder (C₁-C₄-Alkoxy)carbonyl, stehen; wenn keine der Gruppierungen A₂ und A₃ für Stickstoff steht, können R³ und R⁴ gemeinsam mit dem Kohlenstoff an das sie gebunden sind einen 5- oder 6- gliedrigen Ring bilden, der 0, 1 oder 2 Stickstoffatome und/oder 0 oder 1 Sauerstoffatom und/oder 0 oder 1 Schwefelatom enthält, oder wenn keine der Gruppierungen A₁ und A₂ für Stickstoff steht, können R² und R³ gemeinsam mit dem Kohlenstoff an das sie gebunden sind einen 6- gliedrigen Ring bilden, der 0, 1 oder 2 Stickstoffatome enthält;
G für Wasserstoff, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆- Halogencycloalkyl und gegebenenfalls mit R¹⁴ substituiertes Phenyl und Pyridinyl steht;
U für eine Gruppierung C(=W), SO oder SO₂ steht,
wobei
W für Sauerstoff oder Schwefel steht;
L für eine bivalente chemische Gruppierung steht, die ausgewählt ist aus den Gruppierungen -CH₂NHC(=W)-, -CH₂NR⁶C(=W)-, -C(=W)NH-, -C(=W)NR⁶, -C(=W)NHCH₂-, -C(=W)NR⁶CH2₋, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=N-CN)- und -S(=N-CN)-, -C(=W)NHSO₂-, bei der die rechte Seite der Gruppierung direkt an den Rest Q anknüpft und wobei,
p die Werte 0, 1 oder 2 annehmen kann;
R⁶ für Wasserstoff, gegebenenfalls substituiertes C₁-C₆-Alkyl, C₁-C₆-Halogenalkyl, Aryl-(C₁-C₃-alkyl), Heteroaryl-(C₁-C₃-alkyl), C₂-C₆-Alkenyl, C₂-C₆-Alkinyl, C₃- C₆-Cycloalkyl, (C₁-C₂-Alkyl)-(C₃-C₆-cycloalkyl), (C₃-C₆-Cycloalkyl)-(C₁-C₂- alkyl), (C₁-C₆-Alkyl)carbonyl und (C₁-C₆-Alkoxy)carbonyl steht;
m den Wert 1 annimmt;
Q für ein gegebenenfalls mehrfach mit V substituiertes Phenyl, Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxadiazol, Thiadiazol steht, wobei
V für Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁C₆-Halogenalkylthio, C₁-C₆-Alkylsulfinyl, C₁-C₆-Halogenalkylsulfinyl, C₁- C₆-Alkylsulfonyl, C₁-C₆-Halogenalkylsulfonyl, *N*,*N*-Di-(C₁-C₆-alkyl)amino steht, die Gruppierung T für ein gegebenenfalls mehrfach mit Z substituiertes Pyridazin, Pyrazin, Pyrimidin, Triazin, Pyridin, Pyrazol, Thiazol, Isothiazol, Oxazol, Isoxazol, Triazol, Imidazol, Furan, Thiophen, Pyrrol, Oxdiazol oder Thiadiazol stehen kann,
Z für Wasserstoff, Halogen, Cyano, Nitro, gegebenenfalls substituiertes C₁-C₆- Alkyl, C₁-C₄-Alkenyl, C₁-C₄-Alkinyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆- Alkylthio, C₁-C₆-Halogenalkylthio, C₁-C₆-Alkylsulfinyl, C₁-C₆- Halogenalkylsulfinyl, C₁-C₆-Alkylsulfonyl, C₁-C₆-Halogenalkylsulfonyl, *N,N-* Di-(C₁-C₆-alkyl)amino, -C(=W)NR⁷R⁶, -C(=W)OR⁸, -S(O)₂NR⁹R¹⁰, -S(O)ₚR¹¹, - S(O)(=NR¹²)R¹³, (C₃-C₆-Cycloalkyl)carbonyl, (C₃C₆-Cycloalkyl)-(C₁-C₃alkyl), Aryl-(C₁-C₃-alkyl), Heteroaryl-(C₁-C₃-alkyl) und gegebenenfalls mit R¹⁴ substituiertes Phenyl und Pyridinyl steht;
R⁷ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
R⁸ ausgewählt ist aus Wasserstoff oder den gegebenenfalls mit R⁶ substituierten Gruppierung C₁-C₆-Alkyl, C₁-C₆-Halogenalkyl, C₂-C₆-Alkenyl, C₂-C₆- Halogenalkenyl, C₂-C₆-Alkinyl, C₃-C₆-Cycloalkyl, C₃-C₆-Halogencycloalkyl, (C₁-C₂Alkyl)-(C₃C₆-cycloalkyl) und (C₃C₆-Cycloalkyl)-(C₁-C₂Alkyl);
R⁹ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆- Alkinyl, C₃-C₆-Cycloalkyl, (C₁-C₂-Alkyl)-(C₃-C₆-cycloalkyl), (C₃-C₆- Cycloalkyl)-(C₁-C₂-Alkyl), (C₁-C₆-Alkyl)carbonyl und (C₁-C₆-Alkoxy)carbonyl;
R¹⁰ ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂Alkyl)-(C₃C₆-cycloalkyl) und (C₃C₆-Cycloalkyl)-(C₁-C₂Alkyl);
R¹¹ ausgewählt ist aus den gegebenenfalls mit R¹⁶ substituierten Gruppierungen C₁- C₆-Alkyl, C₂-C₆-Alkenyl, C₂-C₆-Alkinyl, C₃-C₆-Cycloalkyl, (C₁-C₂-Alkyl)-(C₃- C₆-cycloalkyl) und (C₃C₆-Cycloalkyl)-(C₁-C₂alkyl), C₁-C₄-Haloalkyl;
R¹² ausgewählt ist aus Wasserstoff, C₁-C₆-Alkyl, C₁-C₆-Halogenalkyl, C₂-C₆- Alkenyl, C₂-C₆-Alkinyl, C₃-C₆-Cycloalkyl, (C₁-C₂-Alkyl)-(C₃-C₆-cycloalkyl), (C₃-C₆-Cycloalkyl)-(C₁-C₂-alkyl), (C₁-C₆-Alkyl)carbonyl und (C₁-C₆- Alkoxy)carbonyl;
R¹³ ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂Alkyl)-(C₃C₆-cycloalkyl) und (C₃C₆-Cycloalkyl)-(C₁-C₂alkyl);
R¹⁴ ausgewählt ist aus Halogen, -OH, -NH₂, -COOH, -CN, -NO₂, C₁-C₆-Alkyl, C₁- C₆-Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-C₁-C₆-Alkylamino, *N*,*N*-Di-(C₁- C₆-alkyl)amino, (C₁-C₆-Alkyl)carbonyl, (C₁-C₆-Alkoxy)carbonyl, *N*-(C₁-C₆- Alkyl)aminocarbonyl und *N,N*-Di-(C₁-C₆-alkyl)aminocarbonyl;
R¹⁵ ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆- Alkylsulfinyl, C₁-C₆-Alkylsulfonyl, -CN, -NO₂ sowie gegebenenfalls mit R¹⁶ substituiertem Phenyl oder Pyridyl;
R¹⁶ ausgewählt ist aus Halogen, -CN, -NO₂, C₁-C₆-Alkyl, C₁-C₆-Alkoxy, C₁-C₆- Alkylthio, C₁-C₆-Alkylsulfinyl, C₁-C₆-Alkylsulfonyl, (C₁-C₆-Alkyl)carbonyl, (C₁-C₆-Alkoxy)carbonyl, *N*-(C₁-C₆-Alkyl)aminocarbonyl, oder gegebenenfalls mit R¹⁸ substituiertem Phenyl oder Pyridyl;
R¹⁷ ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆- Halogenalkylsulfonyl, *N*-C₁-C₆-Alkylamino, *N*,*N*-Di-(C₁-C₆-alkyl)amino, (C₁- C₃-Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei, R¹⁸ ausgewählt ist aus Halogen, -OH, -NH₂, -COOH, -CN -No₂, C₁-C₆-Alkyl, C₁- C₆-Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-(C₁-C₆-Alkyl)amino, *N,N*-Di- (C₁-C₆-alkyl)amino, (C₁-C₃-Alkyl)carbonyl, (C₁-C₃-Alkoxy)carbonyl, *N*-(C₁-C₆- Alkyl)aminocarbonyl, *N*,*N*-Di-(C₁-C₆-alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

3. Verbindungen der allgemeinen Formel (I) gemäß einem der Ansprüche 1 oder 2, in welcher
R¹ für Wasserstoff, gegebenenfalls substituiertes C₁-C₆-Alkyl, C₂-C₆-Alkylen, C₂-C₆- Alkinyl, C₃-C₇-Cycloalkyl, (C₁-C₆-Alkyl)carbonyl, (C₁-C₆-Alkoxy)carbonyl, (C₁- C₂-Alkoxy)-(C₁-C₂-alkyl), Cyan-(C₁-C₂-alkyl), Aryl(C₁-C₃-alkyl), Heteroaryl(C₁- C₃-alkyl) steht,
R² R³ R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-(C₁-C₆-Alkyl)amino, *N*,*N*-Di-(C₁- C₆-alkyl)amino, *N*-(C₁-C₆-Alkyl)aminocarbonyl, *N*-(C₃-C₆- Cycloalkyl)amino- carbonyl oder (C₁-C₄-Alkoxy)carbonyl, stehen;
wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ gemeinsam mit dem Kohlenstoff an das sie gebunden sind einen 6-gliedrigen Ring bilden, der 0, 1 oder 2 Stickstoffatome enthält;
G für Wasserstoff, gegebenenfalls substituiertes C₁-C₆-Alkyl, C₁-C₄-Alkenyl, C₁-C₄- Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
U für eine Gruppierung C(=W), SO oder SO₂ steht,
wobei
W für Sauerstoff oder Schwefel steht;
L für eine bivalente chemische Gruppierung steht, die ausgewählt ist aus den Gruppierungen -CH₂NHC(=W)-, -CH₂NR⁶C(=W)-, -C(=W)NH-, -C(=W)NR⁶, -C(=W)NHCH₂-, -C(=W)NR6^{C}H2₋, -CH=N-OCH₂C(=W)NH-, -CH=N- OCH₂C(=W)NR⁶-, -CH₂NHC(=W)NH-, -CH₂NHC(=W)NR⁶-, -NHC(=W)NH-, -NHC(=W)NR⁶-, -NR⁶C(=W)NH-, -NR⁶C(=W)NR⁶-, -C(=W)-, -C(=W)O-, -C(=W)OCH₂C(=W)-, -C(=W)OCH₂C(=W)NR⁶-, -C(=W)OCH₂C(=W)NHC(=W)NH-, -C(=W)OCH₂C(=W)NH-, -CH₂-, -(CH₂)₂-, -(CH₂)₃-, -Si-, -O-, -S(O)ₚ-, und -CH₂-S(O)ₚ-, -SO(=N-CN)- und -S(=N-CN)-, -C(=W)NHSO₂-, bei der die rechte Seite der Gruppierung direkt an den Rest Q anknüpft und wobei, p die Werte 0, 1 oder 2 annehmen kann;
R⁶ für Wasserstoff, gegebenenfalls substituiertes C₁-C₆-Alkyl, Aryl-(C₁-C₃-alkyl), Heteroaryl-(C₁-C₃-alkyl), C₂-C₆-Alkenyl, C₂-C₆-Alkinyl, C₃-C₆-Cycloalkyl, (C₁- C₂-Alkyl)-(C₃-C₆-cycloalkyl), (C₃-C₆-Cycloalkyl)-(C₁-C₂-alkyl), (C₁-C₆- Alkyl)carbonyl und (C₁-C₆-Alkoxy)carbonyl steht;
m den Werte 1 annimmt;
Q für ein gegebenenfalls mehrfach mit V substituiertes Phenyl, Pyridin, Oxadiazol, Oxazol, Isoxazol, Pyrazol, Thiazol, Thiadiazol, Triazol steht, wobei
V für Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆-
T Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino steht; für einen der unten dargestellten Reste (T-1) - (T-56), 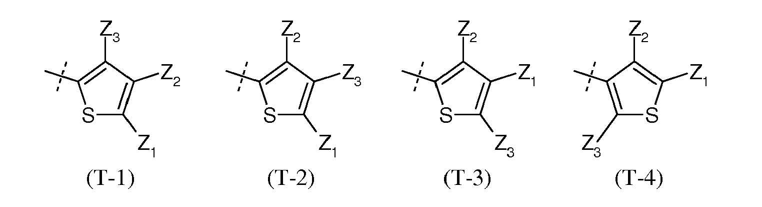 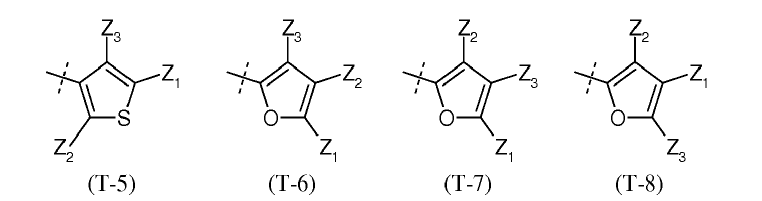 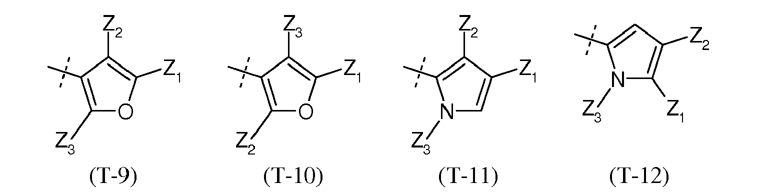 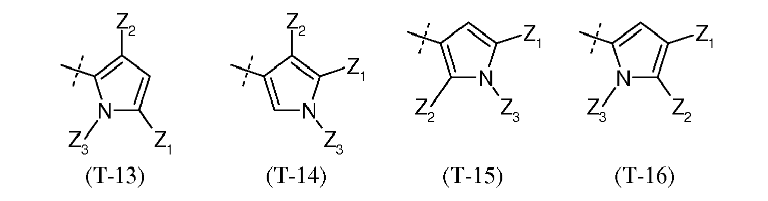 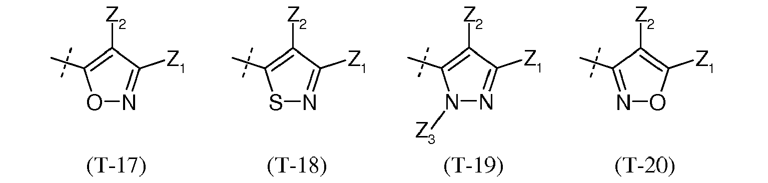 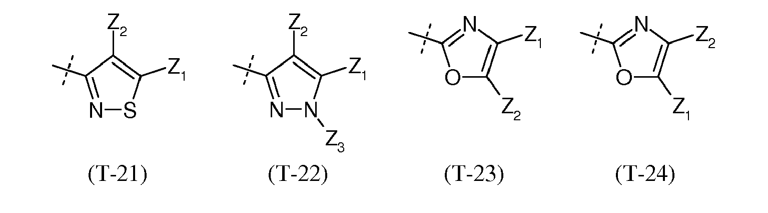 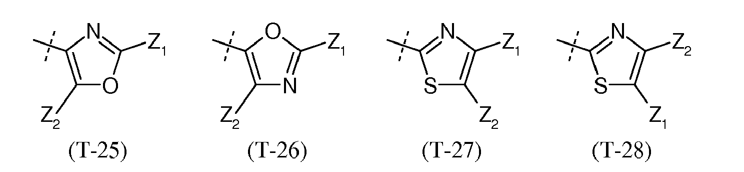 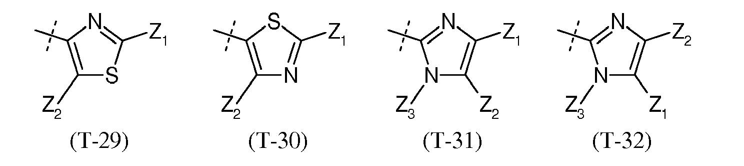 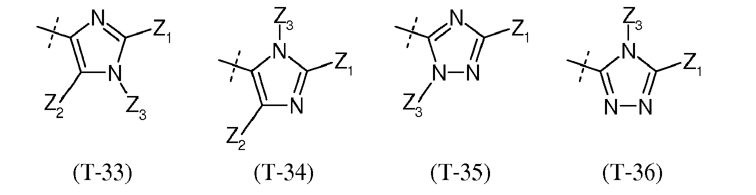 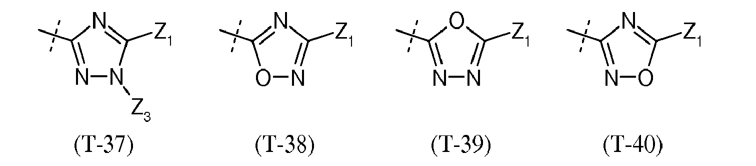 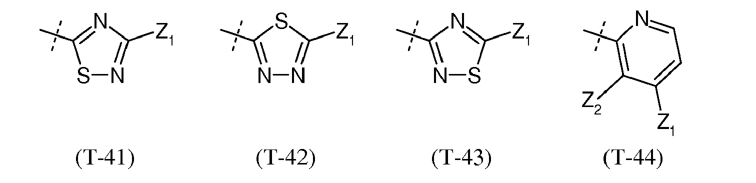 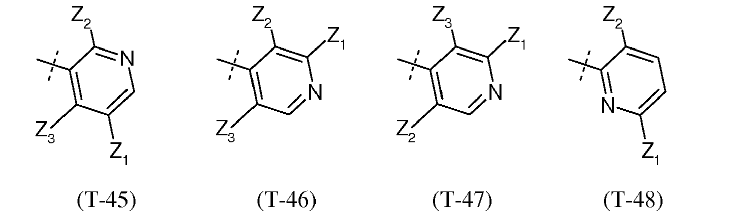 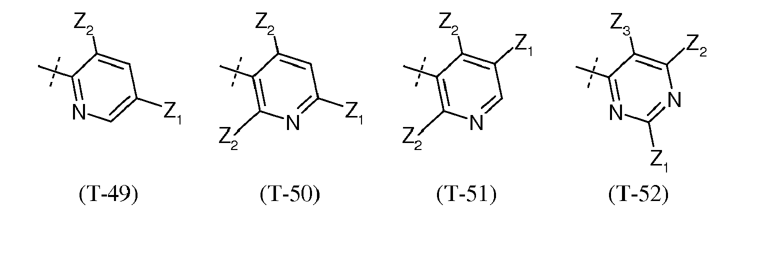 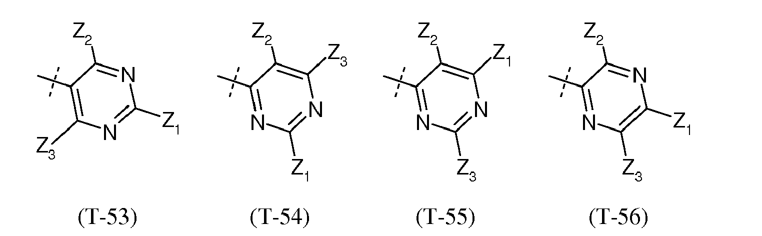 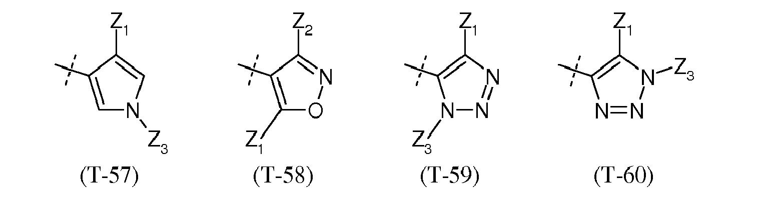
wobei
Z₁ für ein gegebenenfalls substituiertes C₁-C₆-Halogenalkyl, C₃-C₆-Halogencyclo alkyl, C₁-C₆-Halogenalkoxy, C₁-C₆-Halogenalkylthio, C₁-C₆-Halogenalkylsulfinyl, C₁-C₆-Halogenalkylsulfonyl, (C₃-C₆-Cycloalkyl)carbonyl, (C₃-C₆-Cycloalkyl)-(C₁- C₃-alkyl), Aryl-(C₁-C₃-alkyl), Heteroaryl-(C₁-C₃-alkyl) oder gegebenenfalls mit R¹⁴ substituiertes Phenyl und Pyridinyl steht,
Z₂ für Wasserstoff, Halogen, Cyano, Nitro, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino, -C(=W)NR⁷R⁶, -C(=W)OR⁸, -S(O)₂NR⁹R¹⁰, -S(O)ₚR¹¹, -S(O)(=NR¹²)R¹³ und gegebenenfalls mit R¹⁴ substituiertes Phenyl und Pyridinyl steht;
Z₃ für Wasserstoff, gegebenenfalls substituiertes C₁-C₆-Alkyl, C₁-C₄-Alkenyl, C₁-C₄- Alkinyl, C₁-C₆-Halogenalkyl, C₃-C₆-Cycloalkyl, C₃-C₆-Halogencycloalkyl, C₁-C₆- Alkoxy, C₁-C₆-Halogenalkoxy, C₁-C₆-Alkylthio, C₁-C₆-Halogenalkylthio, und gegebenenfalls mit R¹⁴ substituiertes Phenyl und Pyridinyl steht;
R⁷ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
R⁸ ausgewählt ist aus Wasserstoff oder den gegebenenfalls mit R⁶ substituierten Gruppierung C₁-C₆-Alkyl, C₁-C₆-Halogenlkyl, C₂-C₆-Alkenyl, C₂-C₆- Halogenalkenyl, C₂-C₆-Alkinyl, C₃-C₆-Cycloalkyl, C₃-C₆-Halogencycloalkyl, (C₁- C₂-Alkyl)-(C₃-C₆-cycloalkyl) und (C₃-C₆-Cycloalkyl)-(C₁-C₂-Alkyl);
R⁹ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆-Cycloalkyl)-(C₁-C₂- Alkyl), (C1-C₆-Alkyl)carbonyl und (C₁-C₆-Alkoxy)carbonyl;
R¹⁰ ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃-C₆-Cycloalkyl)-(C₁-C₂-Alkyl);
R¹¹ ausgewählt ist aus den gegebenenfalls mit R¹⁶ substituierten Gruppierungen C₁-C₆- Alkyl, C₂-C₆-Alkenyl, C₂-C₆-Alkinyl, C₃-C₆-Cycloalkyl, (C₁-C₂-Alkyl)-(C₃-C₆- cycloalkyl), (C₃-C₆-Cycloalkyl)-(C₁-C₂-alkyl) und C₁-C₄-Haloalkyl;
R¹² ausgewählt ist aus Wasserstoff, C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆- Cycloalkyl)-(C₁-C₂-alkyl), (C₁-C₆-Alkyl)carbonyl und (C₁-C₆-Alkoxy)carbonyl;
R¹³ ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃-C₆-Cycloalkyl)-(C₁-C₂-alkyl);
R¹⁴ ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-C₁-C₆-Alkylamino, *N,N*-Di-(C₁-C₆- alkyl)amino, (C₁-C₆-Alkyl)carbonyl, (C₁-C₆-Alkoxy)carbonyl, *N*-(C₁-C₆- Alkyl)aminocarbonyl und *N*,*N*-Di-(C₁-C₆-alkyl)aminocarbonyl;
R¹⁵ ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂ sowie gegebenenfalls mit R¹⁶ substituiertem Phenyl oder Pyridyl;
R¹⁶ ausgewählt ist aus Halogen, -CN, -NO₂, C₁-C₆-Alkyl, C₁-C₆-Alkoxy, C₁-C₆- Alkylthio, C₁-C₆-Alkylsulfinyl, C₁-C₆-Alkylsulfonyl, (C₁-C₆-Alkyl)carbonyl, (C₁-
R¹⁷ C₆-Alkoxy)carbonyl, *N*-(C₁-C₆-Alkyl)aminocarbonyl, oder gegebenenfalls mit R¹⁸ substituiertem Phenyl oder Pyridyl; ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆- Halogenalkylsulfonyl, *N*-C₁-C₆-Alkylamino, *N*,*N*-Di-(C₁-C₆-alkyl)amino, (C₁-C₃- Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
R¹⁸ ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-(C₁-C₆-Alkyl)amino, *N*,*N*-Di-(C₁- C₆-alkyl)amino, (C₁-C₃-Alkyl)carbonyl, (C₁-C₃-Alkoxy)carbonyl, *N*-(C₁-C₆- Alkyl)aminocarbonyl, *N*,*N*-Di-(C₁-C₆-alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

4. Verbindungen der allgemeinen Formel (I) gemäß einem der Ansprüche 1bis 3, in welchen
R¹ für Wasserstoff, gegebenenfalls substituiertes C₁-C₆-Alkyl, C₂-C₆-Alkylen, C₂-C₆- Alkinyl, C₃-C₇-Cycloalkyl, (C₁-C₆-Alkyl)carbonyl, (C₁-C₆-Alkoxy)carbonyl, Cyan-(C₁-C₂-alkyl), (C₁-C₂-Alkoxy)-(C₁-C₂-alkyl), Aryl(C₁-C₃-alkyl), Heteroaryl(C₁-C₃-alkyl) steht,
R², R³, R⁴ und R⁵ unabhängig voneinander für Wasserstoff, Halogen, CN, NO₂, gegebenenfalls substituiertes C₁-C₆-Alkyl, C₁-C₆-Halogenalkyl, C₃-C₆-Cycloalkyl, C₃-C₆-Halogencycloalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-(C₁-C₆-Alkyl)amino, *N*,*N*-Di-(C₁- C₆-alkyl)amino, *N*-(C₁-C₆-Alkyl)aminocarbonyl, *N*-(C₃-C₆-Cycloalkyl)amino- carbonyl oder (C₁-C₄-Alkoxy)carbonyl, stehen;
wenn keine der Gruppierungen A₂ und A₃ für Stickstoff steht, können R³ und R⁴ gemeinsam mit dem Kohlenstoff an das sie gebunden sind einen 5- oder 6-gliedrigen Ring bilden, der 0, 1 oder 2 Stickstoffatome und/oder 0 oder 1 Sauerstoffatom und/oder 0 oder 1 Schwefelatom enthält, oder
wenn keine der Gruppierungen A₁ und A₂ für Stickstoff steht, können R² und R³ emeinsam mit dem Kohlenstoff an das sie gebunden sind einen 6-gliedrigen Ring bilden, der 0, 1 oder 2 Stickstoffatome enthält;
G für Wasserstoff, gegebenenfalls substituiertes C₁-C₆-Alkyl, C₁-C₄-Alkenyl, C₁-C₄- Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
U für eine Gruppierung C(=W), SO oder SO₂ steht,
wobei
W für Sauerstoff oder Schwefel steht;
L für eine bivalente chemische Gruppierung steht, die ausgewählt ist aus den Gruppierungen -C(=O)NH-, -C(=O)NR⁶, -C(=O)NHSO₂-, bei der die rechte Seite der Gruppierung direkt an den Rest Q anknüpft und wobei,
R⁶ für Wasserstoff, gegebenenfalls substituiertes C₁-C₆-Alkyl, Aryl-(C₁-C₃-alkyl), Heteroaryl-(C₁-C₃-alkyl), C₂-C₆-Alkenyl, C₂-C₆-Alkinyl, C₃-C₆-Cycloalkyl, (C₁- C₂-Alkyl)-(C₃-C₆-cycloalkyl), (C₃-C₆-Cycloalkyl)-(C₁-C₂-alkyl), (C₁-C₆- Alkyl)carbonyl und (C₁-C₆-Alkoxy)carbonyl steht;
m den Wert 1 annimmt;
Q für einen der unten dargestellten Reste (Q-1) - (Q-8) steht, 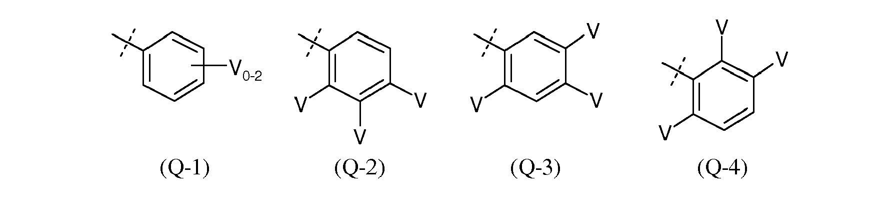 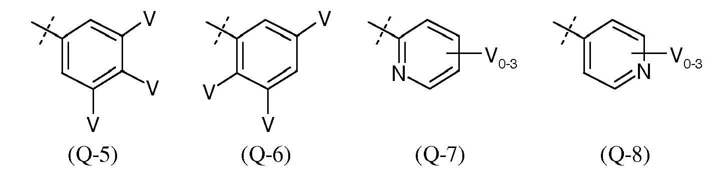
V für Halogen, Cyano, Nitro, gegebenenfalls substituiertes C₁-C₆-Alkyl, C₁-C₄- Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino steht;
T für einen der Reste (T-1), (T-2), (T-3), (T-4), (T-5), (T-8), (T-9), (T-11), (T-12), (T-13), (T-14), (T-17), (T-18), (T-19), (T-20), (T-21), (T-22), (T-23), (T-24), (T- 25), (T-26), (T-27), (T-28), (T-29), (T-30), (T-31), (T-33), (T-34), (T-35), (T-44) (T-45), (T-46), (T-47), (T-48), (T-49), (T-50), (T-51), (T-53), (T-55), (T-57),(T- 58),(T-59),(T-60) steht,
wobei
Z₁ für ein gegebenenfalls substituiertes C₁-C₆-Halogenalkyl, C₃-C₆-Halogencyclo alkyl" C₁-C₆-Halogenalkoxy, C₁-C₆-Halogenalkylthio, C₁-C₆- Halogenalkylsulfinyl, C₁-C₆-Halogenalkylsulfonyl, (C₃-C₆-Cycloalkyl)carbonyl, (C₃-C₆-Cycloalkyl)-(C₁-C₃-alkyl), Aryl-(C₁-C₃-alkyl), Heteroaryl-(C₁-C₃-alkyl) oder gegebenenfalls mit R¹⁴ substituiertes Phenyl und Pyridinyl steht,
Z₂ für Wasserstoff, Halogen, Cyano, Nitro, gegebenenfalls substituiertes C₁-C₆-Alkyl, C₁-C₄-Alkenyl, C₁-C₄-Alkinyl, C₁-C₆-Halogenalkyl, C₃-C₆-Cycloalkyl, C₃-C₆- Halogencycloalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*,*N*-Di-(C₁-C₆-alkyl)amino, - C(=W)NR⁷R⁶, -C(=W)OR⁸, -S(O)₂NR⁹R¹⁰, -S(O)ₚR¹¹, -S(O)(=NR¹²)R¹³ und gegebenenfalls mit R¹⁴ substituiertes Phenyl und Pyridinyl steht;
Z₃ für Wasserstoff, gegebenenfalls substituiertes C₁-C₆-Alkyl, C₁-C₄-Alkenyl, C₁-C₄- Alkinyl, C₁-C₆-Halogenalkyl, C₃-C₆-Cycloalkyl, C₃-C₆-Halogencycloalkyl und gegebenenfalls mit R¹⁴ substituiertes Phenyl und Pyridinyl steht;
R⁷ ausgewählt ist aus Wasserstoff oder den gegebenenfalls substituierten Gruppierungen C₁-C₆-Alkyl, C₂-C₆-Alkenyl, C₂-C₆-Alkinyl, C₃-C₆-Cycloalkyl, (C₁-C₆-Alkyl)carbonyl und (C₁-C₆-Alkoxy)carbonyl;
R⁸ ausgewählt ist aus Wasserstoff oder den gegebenenfalls mit R⁶ substituierten Gruppierung C₁-C₆-Alkyl, C₁-C₆-Halogenlkyl, C₂-C₆-Alkenyl, C₂-C₆- Halogenalkenyl, C₂-C₆-Alkinyl, C₃-C₆-Cycloalkyl, C₃-C₆-Halogencycloalkyl, (C₁- C₂-Alkyl)-(C₃-C₆-cycloalkyl) und (C₃-C₆-Cycloalkyl)-(C₁-C₂-Alkyl);
R⁹ ausgewählt ist aus Wasserstoff oder den gegebenenfalls substituierten Gruppierungen C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆-Cycloalkyl)-(C₁-C₂- Alkyl), (C₁-C₆-Alkyl)carbonyl und (C₁-C₆-Alkoxy)carbonyl;
R¹⁰ ausgewählt ist aus Wasserstoff oder den gegebenenfalls mit R¹⁵ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃-C₆-Cycloalkyl)-(C₁-C₂-Alkyl);
R¹¹ ausgewählt ist aus den gegebenenfalls mit R¹⁶ substituierten Gruppierungen C₁-C₆- Alkyl, C₂-C₆-Alkenyl, C₂-C₆-Alkinyl, C₃-C₆-Cycloalkyl, (C₁-C₂-Alkyl)-(C₃-C₆- cycloalkyl), (C₃-C₆-Cycloalkyl)-(C₁-C₂-alkyl) und C₁-C₄-Haloalkyl;
R¹² ausgewählt ist aus Wasserstoff, C₁-C₆-Alkyl, C₁-C₆-Halogenalkyl, C₂-C₆-Alkenyl, C₂-C₆-Alkinyl, C₃-C₆-Cycloalkyl, (C₁-C₂-Alkyl)-(C₃-C₆-cycloalkyl), (C₃-C₆- Cycloalkyl)-(C₁-C₂-alkyl), (C₁-C₆-Alkyl)carbonyl und (C₁-C₆-Alkoxy)carbonyl;
R¹³ ausgewählt ist aus Wasserstoff oder den gegebenenfalls mit R¹⁶ substituierten Gruppierungen C₁-C₆-Alkyl, C₂-C₆-Alkenyl, C₂-C₆-Alkinyl, C₃-C₆-Cycloalkyl, (C₁-C₂-Alkyl)-(C₃-C₆-cycloalkyl) und (C₃-C₆-Cycloalkyl)-(C₁-C₂alkyl);
R¹⁴ ausgewählt ist aus Halogen, -OH, -NH₂, -COOH, -CN, -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-C₁-C₆-Alkylamino, *N*,*N*-Di-(C₁-C₆- alkyl)amino, (C₁-C₆-Alkyl)carbonyl, (C₁-C₆-Alkoxy)carbonyl, *N*-(C₁-C₆- Alkyl)aminocarbonyl und *N*,*N*-Di-(C₁-C₆-alkyl)aminocarbonyl;
R¹⁵ ausgewählt ist aus Wasserstoff oder den gegebenenfalls mit R¹⁷ substituierten Gruppierungen C₁-C₆-Alkyl, C₁-C₆-Alkoxy, C₁-C₆-Alkylthio, C₁-C₆-Alkylsulfinyl, C₁-C₆-Alkylsulfonyl, -CN, -NO₂ sowie gegebenenfalls mit R¹⁶ substituiertem Phenyl oder Pyridyl;
R¹⁶ ausgewählt ist aus Halogen, -CN, -NO₂, C₁-C₆-Alkyl, C₁-C₆-Alkoxy, C₁-C₆- Alkylthio, C₁-C₆-Alkylsulfinyl, C₁-C₆-Alkylsulfonyl, (C₁-C₆-Alkyl)carbonyl, (C₁- C₆-Alkoxy)carbonyl, *N*-(C₁-C₆-Alkyl)aminocarbonyl, oder gegebenenfalls mit R¹⁸ substituiertem Phenyl oder Pyridyl;
R¹⁷ ausgewählt ist aus Halogen, -OH, -NH₂, -COOH, -CN, -NO₂ oder den gegebenenfalls subsitutierten Gruppierungen C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl, C₁-C₆- Halogenalkylsulfonyl, *N*-C₁-C₆-Alkylamino, *N*,*N*-Di-(C₁-C₆-alkyl)amino, (C₁-C₃- Alkyl)carbonyl, (C₁-C₃-Alkoxy)carbonyl, *N*-(C₁-C₆-Alkyl)aminocarbonyl und *N*,*N*-Di-(C₁-C₆-alkyl)aminocarbonyl, wobei,
R¹⁸ ausgewählt ist aus Halogen, -OH, -NH₂, -COOH, -CN -NO₂, C₁-C₆-Alkyl, C₁-C₆- Halogenalkyl, C₁-C₆-Alkoxy, C₁-C₆-Halogenalkoxy, C₁-C₆-Alkylthio, C₁-C₆- Halogenalkylthio, C₁-C₆-Alkylsulfinyl, C₁-C₆-Halogenalkylsulfinyl, C₁-C₆- Alkylsulfonyl, C₁-C₆-Halogenalkylsulfonyl, *N*-(C₁-C₆-Alkyl)amino, *N*,*N*-Di-(C₁- C₆-alkyl)amino, (C₁-C₃-Alkyl)carbonyl, (C₁-C₃-Alkoxy)carbonyl, *N*-(C₁-C₆- Alkyl)aminocarbonyl, *N*,*N*-Di-(C₁-C₆-alkyl)aminocarbonyl; oder
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

5. Verbindungen der allgemeinen Formel (I) gemäß einem der Ansprüche 1 bis 4, in welchen
R¹ für Wasserstoff, Methyl, Ethyl, n-Propyl, Isopropyl, n-Butyl, Isobutyl, sec-Butyl, tert-Butyl, Methoxymethyl, Ethoxymethyl, Propoxymethyl, Methylcarbonyl, Ethylcarbonyl, n-Propylcarbonyl, Isopropylcarbonyl, sec-Butylcarbonyl, tert- Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2- Cyanoethyl steht;
R² und R⁵ unabhängig voneinander für Wasserstoff, Methyl, Fluor und Chlor stehen und
R³ und R⁴ unabhängig voneinander für Wasserstoff, Fluor, Chlor, Brom, Cyano, Nitro, Methyl, Ethyl, Fluormethyl, Difluormethyl, Trifluormethyl, 2,2,2-Trilfluorethyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2- Trifluorethoxy, 2-Chlor-2,2-difluorethoxy, Pentafluorethoxy, Methylsulfonyl, Methylsulfinyl, Trifluormethylsulfonyl, Trifluormethylsulfinyl und *N*- Cyclopropylaminocarbonyl stehen;
G für Wasserstoff, Methyl, Ethyl, n-Propyl, Isopropyl, n-Butyl, Isobutyl, sec-Butyl, tert-Butyl" Ethenyl, 1-Propenyl, 2-Propenyl, Fluormethyl, Difluormethyl, Trifluormethyl, Chlormethyl, Pentafluorethyl, 2,2,2-Trifluorethyl, Cyclopropyl, Cyclobutyl, Methoxymethyl, Ethoxymethyl, Propoxymethyl, 2- Methoxyethoxymethyl, Methylcarbonyl, Methoxycarbonyl, Ethoxycarbonyl, n- Propoxycarbonyl, Isopropoxycarbonyl, sec-Butoxycarbonyl, tert-Butoxycarbonyl, Cyanomethyl, 2-Cyanoethyl, Phenyl, 4-Fluorphenyl, 4-Chlorphenyl, 4- Trifluormethylphenyl, 2,4-Dichlorphenyl, 2-Chlor-4-trifluormethyl-phenyl steht;
wobei
U für C(=W) und
W für Sauerstoff steht;
L für eine bivalente chemische Gruppierung steht, die ausgewählt ist aus den Gruppierungen -C(=O)NH-, -C(=O)NR⁶, -C(=O)NHSO₂-, bei der die rechte Seite der Gruppierung direkt an den Rest Q anknüpft und wobei,
R⁶ für Wasserstoff, Methyl, Ethyl, n-Propyl, Isopropyl, n-Butyl, Isobutyl, sec-Butyl, tert-Butyl, Methoxymethyl, Ethoxymethyl, Propoxymethyl, Methylcarbonyl, Ethylcarbonyl, n-Propylcarbonyl, Isopropylcarbonyl, sec-Butylcarbonyl, tert- Butylcarbonyl, Methoxycarbonyl, Ethoxycarbonyl, n-Propoxycarbonyl, Isopropoxycarbonyl, sec-Butoxycarbonyl, tert-Butoxycarbonyl, Cyanomethyl, 2- Cyanoethyl, Pyrid-2-yl-methyl, Pyrid-3-yl-methyl und Pyrid-4-yl-methyl steht;
m den Wert 1 annimmt;
Q für einen der unten dargestellten Reste (Q-1) - (Q-8) steht; 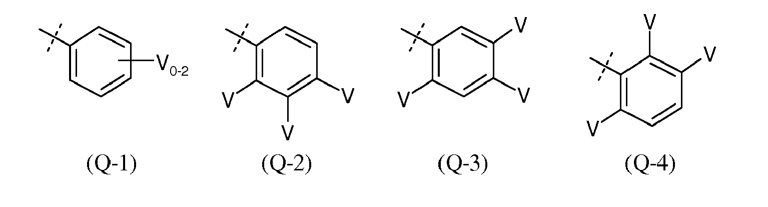 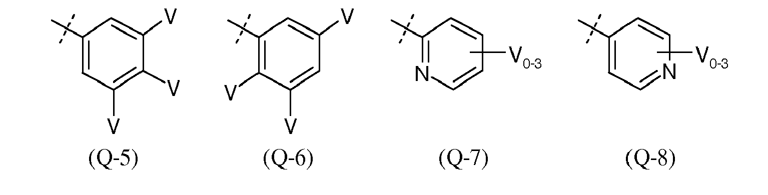
V für Fluor, Chlor, Brom, Iod, Cyano, Nitro, Methyl, Ethyl, Difluormethyl, Trichlormethyl, Chlordifluormethyl, Dichlorfluormethyl, Trifluormethyl, Chlormethyl, Brommethyl, 1-Fluorethyl, 2-Fluorethyl, 2,2-Difluorethyl, 2,2,2- Trifluorethyl, 1,2,2,2-Tetrafluorethyl, 1-Chlor-1,2,2,2-tetrafluorethyl, 2,2,2- Trichlorethyl, 2-Chlor-2,2-difluorethyl, 1,1-Dilfluorethyl, Pentafluorethyl Pentafluor-tert-butyl, Heptafluor-n-propyl, Heptafluor-isopropyl, Nonafluor-n- butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2-Trifluorethoxy, 2-Chlor-2,2-difluorethoxy, Pentafluorethoxy, Methylthio, Methylsulfonyl, Methylsulfinyl, Trifluormethylsulfonyl, Trifluormethylsulfinyl steht;
T für einen der Reste (T-8), (T-9), (T-11, (T-17), (T-18), (T-19), (T-21, (T-29), (T- 35), (T-44), (T-49), (T-50), (T-53), (T-55), (T-57) steht,
wobei
Z₁ für Difluormethyl, Trichlormethyl, Chlordifluormethyl, Dichlorfluormethyl, Trifluormethyl, Chlormethyl, Brommethyl, 1-Fluorethyl, 1-Fluor-1-methylethyl, 2- Fluorethyl, 2,2-Difluorethyl, 2,2,2-Trifluorethyl, 1,2,2,2-Tetrafluorethyl, 1-Chlor- 1,2,2,2-tetrafluorethyl, 2,2,2-Trichlorethyl, 2-Chlor-2,2-difluorethyl, 1,1- Dilfluorethyl, Pentafluorethyl Pentafluor-tert-butyl, Heptafluor-n-propyl, Heptafluor-isopropyl, Nonafluor-n-butyl, Trifluormethoxy-1,1,2,2- tetrafluorethoxy-difluormethyl, Trifluormethylthio, Trifluormethylsulfinyl, Trifluormethylsulfonyl, Trifluormethoxy, Difluormethoxy, Cyclopropyl, Cyclobutyl, Cyclopropyl-hydroxymethyl, Cyclopropyl-fluormethyl, Cyclopropylcarbonyl, 2,2,2-Trifluorethoxy, 1-Trifluormethylethoxy, 3,3,3,2,2- Pentafluorpropoxy, Phenyl, 2-Chlorphenyl, 3-Chlorphenyl, 4-Chlorphenyl, 4- Fluorphenyl, 2-Methylphenyl, 3-Methylphenyl, 4-Methylphenyl, 3- Trifluormethylphenyl, 4-Trifluormethylphenyl, 4- Nitrophenyl, *4-*(*tert-* Butyl)phenyl, 4-Methoxyphenyl, 4-Phenoxyphenyl, 2,4-Dichlorphenyl, 3,4- Dichlorphenyl, 3,5-Dichlorphenyl, 2,5-Dichlorphenyl, 2,6-Difluorphenyl, 4- Trifluormethoxyphenyl, 4-Cyanophenyl, 2-Trifluormethyl-pyrid-5-yl, 3-Chlor-5- trifluormethyl-pyrid-2-yl, (3,5-Bis(trifluormethyl)-1H-pyrazol-1-yl)-methyl, (3,5- Bis(pentafluorethyl)-1H-pyrazol-1-yl)-methyl, (3,5-Bis(trifluormethyl)-1H-triazol- 1-yl)-methyl, (2-Methyl-2-H-tetrazol-5-yl)-methyl, (5-Trifluormethyl-2H-tetrazol- 2-yl)-methyl, (3,5-Dichlorphenyl)-methyl, (3,5-Bis(trifluormethyl)phenyl)-methyl, (2,4-Dichlorphenyl)-methyl, (2,6-Dichlorphenyl)-methyl, (4-Chlorphenyl)-methyl, (4-Trifluormethylphenyl)-methyl steht,
Z₂ für Wasserstoff, Halogen, Cyano, Nitro, Methyl, Ethyl, n-Propyl, Isopropyl, n- Butyl, Isobutyl, sec-Butyl, tert-Butyl, Ethenyl, 1-Propenyl, 2-Propenyl, Ethinyl, 1- Propinyl, 1-Butinyl, Difluormethyl, Trichlormethyl, Chlordifluormethyl, Dichlorfluormethyl, Trifluormethyl, Chlormethyl, Brommethyl, 1-Fluorethyl, 1- Fluor-1-methylethyl, 2-Fluorethyl, 2,2-Difluorethyl, 2,2,2-Trifluorethyl, 1,2,2,2- Tetrafluorethyl, 1-Chlor-1,2,2,2-tetrafluorethyl, 2,2,2-Trichlorethyl, 2-Chlor-2,2- difluorethyl, 1,1-Dilfluorethyl, Pentafluorethyl Pentafluor-tert-butyl, Heptafluor-n- propyl, Heptafluor-isopropyl, Nonafluor-n-butyl, Cyclopropyl, Cyclobutyl, Methoxy, Ethoxy, n-Propoxy, 1-Methylethoxy, Fluormethoxy, Difluormethoxy, Chlor-difluormethoxy, Dichlor-fluormethoxy, Trifluormethoxy, 2,2,2- Trifluorethoxy, 2-Chlor-2,2-difluorethoxy, Pentafluorethoxy, Methylthio, Methylsulfonyl, Methylsulfinyl, Trifluormethylsulfonyl, Trifluormethylsulfinyl, *N,N*-Dimethylamino, 4-Fluorphenyl, 4-Chlorphenyl, 4-Trifluormethylphenyl steht;
Z₃ für Wasserstoff, Methyl, Ethyl, n-Propyl, Isopropyl, n-Butyl, Isobutyl, sec-Butyl, tert-Butyl" Ethenyl, 1-Propenyl, 2-Propenyl, Fluormethyl, Difluormethyl, Trifluormethyl, Chlormethyl, Pentafluorethyl, 2,2,2-Trifluorethyl, Cyclopropyl, Cyclobutyl, Methoxymethyl, Ethoxymethyl, Propoxymethyl, 2- Methoxyethoxymethyl, Methylcarbonyl, Methoxycarbonyl, Ethoxycarbonyl, n- Propoxycarbonyl, Isopropoxycarbonyl, sec-Butoxycarbonyl, tert-Butoxycarbonyl, Cyanomethyl, 2-Cyanoethyl, Phenyl, 4-Fluorphenyl, 4-Chlorphenyl, 4- Trifluormethylphenyl, 2,4-Dichlorphenyl, 2-Chlor-4-trifluormethyl-phenyl steht;
ferner können
L, Q und R⁴ zusammen mit den Kohlenstoffatomen, an die sie gebunden sind, einen gegebenenfalls substituierten 5- oder 6-gliedrigen Ring bilden, der gegebenenfalls 0, 1 oder 2 Stickstoffatome und/oder 0 oder 1 Sauerstoffatom und/oder 0 oder 1 Schwefelatom enthält.

6. Verbindungen der allgemeinen Formeln (Ia) bis (Ij), 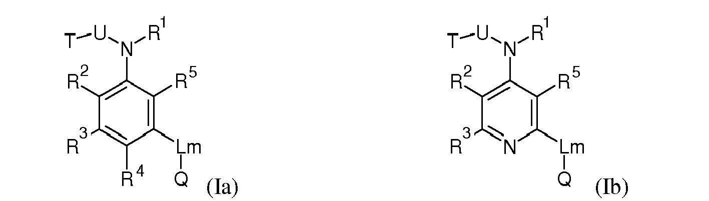 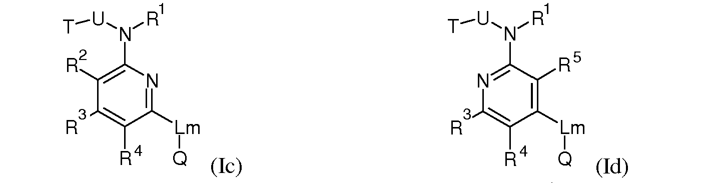 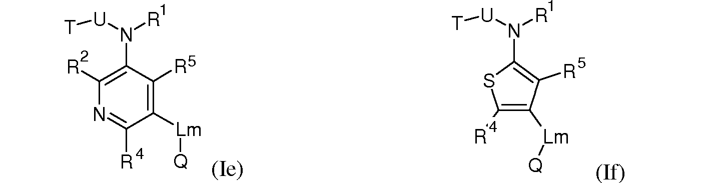 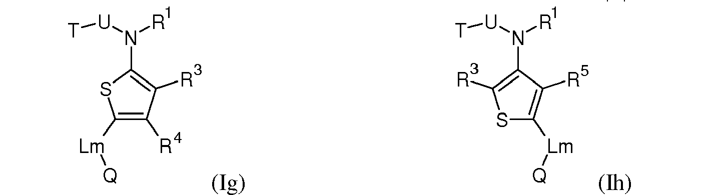 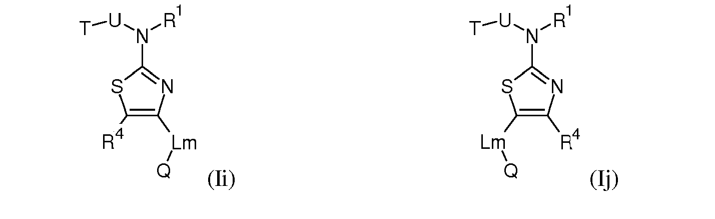 wobei die Reste T, U, R¹, R², R³,R⁴, R⁵, Lm und Q die in den Ansprüchen 1 bis 5 angegebenen Bedeutungen haben.

7. Verbindungen der allgemeinen Formel (Iaa), 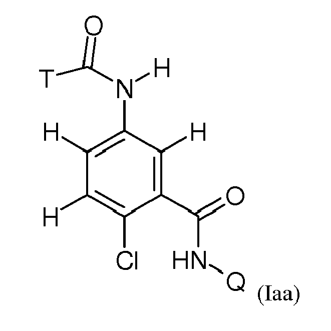 wobei der Rest T ausgewählt sein kann aus Methyl-3-(pentafluorethyl)-4-(trifluormethyl)-1H-pyrazol-5-yl, 2-Methyl-6-(trifluormethyl)pyridin-3-yl, 2-(Trifluormethyl)-1,3-thiazol-4-yl, 4-Brom-1-methyl-3-(trifluormethyl)-1H-pyrazol-5-yl, 4-(Difluormethyl)-2-(pentafluorethyl)pyrimidin-5-yl, 4-Cyclopropyl-2-(pentafluorethyl)pyrimidin-5-yl, 2,4-Bis(pentafluorethyl)pyrimidin-5-yl, 3-{[3,5-Bis-(pentafluorethyl)-1H-pyrazol-1-yl]methyl}-1-methyl-4-(trifluormethyl)-1H-pyrazol-5-yl, und
der Rest Q ausgewählt sein kann aus
Pyridin-4-yl, Pyridin-3-yl, 4-Fluorphenyl, 4-Chlorpyridin-3-yl, 2-Chlorpyridin-4yl, 6-Chlorpyridin-3-yl, Pyridin-2-yl, 3-Chlorphenyl, 4-Methoxyphenyl, 2-Chlorpyridin-3-yl, Phenyl, 3-Methoxyphenyl, 6-Chlorpyridin-2-yl, 2,4-Difluorphenyl, 4-Fluor-2-methylphenyl, 4-Chlorpyridin-2-yl, 4-Fluor-3-methylphenyl, 3-Chlor-4-fluor-2-methylphenyl, 5-Chlorpyridin-2-yl, 4-Fluor-3-(trifluormethyl)phenyl, 3,4-Dichlorphenyl, 3,4,5-Trichlorphenyl.

8. Verwendung von Verbindungen der allgemeinen Formel (I) gemäß einem der Ansprüche 1 bis 5 oder Verbindungen der allgemeinen Formeln (Ia) bis (Ij) oder Verbindungen der allgemeinen Formel (Iaa) gemäß Anspruch 6 zur Bekämpfung von Insekten, Spinnentieren und Nematoden.

9. Pharmazeutische Zusammensetzungen, enthaltend wenigstens eine Verbindung gemäß einem der Ansprüche 1 bis 8.

10. Verwendung von Verbindungen der allgemeinen Formel (I) gemäß einem der Ansprüche 1 bis 5 oder Verbindungen der allgemeinen Formeln (Ia) bis (Ij) gemäß Anspruch 6 oder der Verbindungen allgemeinen Formel (Iaa) zur Herstellung pharmazeutischer Zusammensetzungen zur Bekämpfung von Parasiten auf Tieren.

11. Verfahren zur Herstellung von Pflanzenschutzmitteln enthaltend Verbindungen der allgemeinen Formel (I) gemäß einem der Ansprüche 1 bis 5 oder der allgemeinen Formeln (Ia) bis (Ij) gemäß Anspruch 6 oder Verbindungen der allgemeinen Formel (Iaa) sowie übliche Streckmittel und/oder oberflächenaktive Substanzen.

12. Verfahren zur Bekämpfung von Schädlingen, **dadurch gekennzeichnet, dass** man eine Verbindung der allgemeinen Formel (I) gemäß einem der Ansprüche 1 bis 5 oder der allgemeinen Formeln (Ia) bis (Ij) gemäß Anspruch 6 oder Verbindungen der allgemeinen Formel (Iaa) auf die Schädlinge und/oder ihren Lebensraum einwirken lässt.

13. Verwendung von Verbindungen der allgemeinen Formel (I) gemäß einem der Ansprüche 1 bis 5 sowie der Verbindungen der allgemeinen Formeln (Ia) bis (Ij) gemäß Anspruch 6 oder Verbindungen der allgemeinen Formel (Iaa) zum Schutz des Vermehrungsmaterials von Pflanzen, insbesondere von Saatgut.
